# EUROPEAN PATENT SPECIFICATION

(11) **EP 2 525 809 B1**
(45) Date of publication and mention of the grant of the patent: **03.08.2016**
(21) Application number: 11704174.9
(22) Date of filing: 20.01.2011
(51) Int. Cl.: A61K 38/26, A61P 9/04

(54) **GLUCAGON-GLP1 DUAL AGONISTS FOR USE IN THE TREATMENT OF CARDIAC CONDITIONS**
GLUKAGON-GLP1 DOPPELAGONISTEN ZUR BEHANDLUNG VON HERZKRANKHEITEN
GLUCAGON-GLP1 AGONISTES DOUBLES POUR LE TRAITEMENT DES MALADIES CARDIAQUES

(30) Priority: 20.01.2010 US 296657 P
(43) Date of publication of application: 28.11.2012
(73) Proprietor: Zealand Pharma A/S, 2600 Glostrup (DK)
(72) Inventor: PETERSEN, Jørgen Søberg, CH-1278 La Rippe (CH); KJOELBYE, Anne Louise, CH-1278 La Rippe (CH); SKOVGAARD, Marie, DK-2100 Copenhagen Ø (DK); PEDERSEN, Henrik Duelund, DK-2800 Lyngby (DK); AXELSEN, Lene, DK-2605 Brøndby (DK); RIBER, Ditte, DK-2700 Brønshøj (DK); MEIER, Eddi, DK-3500 Værløse (DK); HANSEN, Rie Schultz, DK-2720 Vanløse (DK); FOSGERAU, Keld, DK-2720 Vanløse (DK); LARSEN, Bjarne Due, DK-4000 Roskilde (DK)
(74) Representative: Forrest, Graham Robert
(86) International application number: PCT/DK2011/050018
(87) International publication number: WO 2011/088837

(56) References cited:
- EP-A1- 1 421 950
- WO-A1-2008/152403
- WO-A2-02/34285
- US-B1- 6 703 359
- MAYER S E ET AL: "EFFECT OF GLUCAGON ON CYCLIC 3 5 AMP PHOSPHORYLASE ACTIVITY AND CONTRACTILITY OF HEART MUSCLE OF THE RAT", CIRCULATION RESEARCH, vol. 26, no. 2, 1970, pages 225-233, XP002637631, ISSN: 0009-7330
- SARACENI CHRISTINE ET AL: "Effects of glucagon-like peptide-1 and long-acting analogues on cardiovascular and metabolic function", DRUGS IN R & D, ADIS INTERNATIONAL, AUCKLAND, NZ, vol. 8, no. 3, 1 January 2007 (2007-01-01) , pages 145-153, XP009095298, ISSN: 1174-5886, DOI: DOI:10.2165/00126839-200708030-00002
- GRIEVE D J ET AL: "Emerging cardiovascular actions of the incretin hormone glucagon-like peptide-1: Potential therapeutic benefits beyond glycaemic control?", BRITISH JOURNAL OF PHARMACOLOGY 2009 NATURE PUBLISHING GROUP GBR LNKD- DOI:10.1111/J.1476-5381.2009.00376.X, vol. 157, no. 8, 2009, pages 1340-1351, XP002637632, ISSN: 0007-1188
- UESAKA TOSHIHIRO ET AL: "Glucagon-like peptide isolated from the eel intestine: Effects on atrial beating", JOURNAL OF EXPERIMENTAL BIOLOGY, vol. 204, no. 17, September 2001 (2001-09) , pages 3019-3026, XP002637633, ISSN: 0022-0949
- SOWDEN GILLIAN L ET AL: "Oxyntomodulin increases intrinsic heart rate in mice independent of the glucagon-like peptide-1 receptor", AMERICAN JOURNAL OF PHYSIOLOGY - REGULATORY INTEGRATIVE AND COMPARATIVE PHYSIOLOGY, vol. 292, no. 2, February 2007 (2007-02), pages R962-R970, XP002637634, ISSN: 0363-6119
- ROBBERECHT P ET AL: "Comparative efficacy of seven synthetic glucagon analogs, modified in position 1, 2 and/or 12, on liver and heart adenylate cyclase from rat", PEPTIDES, ELSEVIER, AMSTERDAM, vol. 7, 1 January 1986 (1986-01-01), pages 109-112, XP023486854, ISSN: 0196-9781, DOI: DOI:10.1016/0196-9781(86)90172-5 [retrieved on 1986-01-01]

## Description

### FIELD OF THE INVENTION

The invention relates to a glucagon-GLP-1 dual agonist for use in a method of treatment of myocardial infarction, cardiogenic shock or heart failure, wherein said glucagon-GLP-1 dual agonist is administered for use as a positive inotropic agent, and wherein said glucagon-GLP-1 dual agonist is a glucagon or oxyntomodulin analogue..

### BACKGROUND OF THE INVENTION

Positive inotropic agents are used to improve hemodynamic parameters and thereby relieve symptoms and protect end-organs in patients with myocardial infarction, heart failure or cardiogenic shock. The heart requires large amounts of chemical energy to support systolic and diastolic work. Therefore, by increasing cardiac work, inotropic agents also increase cardiac energy demand. However, the failing or diseased heart is usually energy starved (Ingwall, JS and Weiss, RG. Circ Res. 2004; 95: 135-145), and the use of inotropic agents may therefore result in energy depletion and ultimately increased mortality (Hamad, E et al. American Journal of cardiovascular Drugs. 2007; 7: 235-248; White, CM. J Clin Pharmacol. 1999; 39: 442-447).

Preproglucagon is a 158 amino acid precursor polypeptide that is differentially processed in the tissues to form a number of structurally related proglucagon-derived peptides, including glucagon (Glu), glucagon-like peptide-1 (GLP-1), glucagon-like peptide-2 (GLP-2), and oxyntomodulin (OXM). These molecules are involved in a wide variety of physiological functions, including glucose homeostasis, insulin secretion, gastric emptying and intestinal growth, as well as regulation of food intake.

A major biologically active fragment of GLP-1 is produced as a 30-amino acid, C-terminally amidated peptide that corresponds to amino acids 98 to 127 of preproglucagon. GLP-1 is produced in the intestinal epithelial endocrine L-cells by differential processing of proglucagon, a hormone normally secreted by neuroendocrine cells of the gut in response to food. It increases insulin release by the beta cells even in subjects with long-standing type 2 diabetes. GLP-1 treatment has an advantage over insulin therapy because GLP-1 stimulates endogenous insulin secretion, which turns off when blood glucose levels drop. GLP-1 promotes euglycemia by increasing insulin release and synthesis, inhibiting glucagon release, and decreasing gastric emptying ). GLP-1 (Holst, JJ. Physiol Rev. 2007; 87: 1409-1439), has been found to increase myocardial glucose uptake in an insulin-independent manner in normal and post-ischemic rat hearts (Zhao, T et al. J Pharmacol Exp Ther. 2006; 317: 1106-1113), isolated mouse hearts (Ban, K et al. Circulation. 2008; 117: 2340-2350), as well as in conscious dogs with dilated cardiomyopathy (Nikolaidis, LA et al. Am J Physiol Heart Circ Physiol. 2005; 289: H2401-H2408; Nikolaidis, LA et al. Circulation. 2004; 110: 955-961).

Glucagon is a 29-amino acid peptide that corresponds to amino acids 53 to 81 of pre-proglucagon and has the sequence His-Ser-Gln-Gly-Thr-Phe-Thr-Ser-Asp-Tyr-Ser-Lys-Tyr-Leu-Asp-Ser-Arg-Arg-Ala-Gln-Asp-Phe-Val-Gln-Trp-Leu-Met-Asn-Thr(Compound 1) Glucagon helps maintain the level of glucose in the blood by binding to glucagon receptors on hepatocytes, causing the liver to release glucose - stored in the form of glycogen - through glycogenolysis. As these stores become depleted, glucagon stimulates the liver to synthesize additional glucose by gluconeogenesis. This glucose is released into the bloodstream, preventing the development of hypoglycemia.

Glucagon has a well documented inotropic effect on the heart (Buse, MG et al. J Biol Chem. 1973; 248: 697-706; Farah, A and Tuttle, R. J Pharmacol Exp Ther. 1960; 129: 49-55; Levey, GS and Epstein, SE. Circ Res. 1969; 24: 151-156; Mayer, SE et al. Circ Res. 1970; 26: 225-233).

Oxyntomodulin (OXM) is a 37 amino acid peptide which includes the complete 29 amino acid sequence of glucagon with an octapeptide carboxyterminal extension (amino acids 82 to 89 of pre-proglucagon, having the sequence Lys-Arg-Asn-Arg-Asn-Asn-Ile-Ala (Compound 2) and termed "intervening peptide 1" or IP-1; the full sequence of human oxyntomodulin is thus His-Ser-Gln-Gly-Thr-Phe-Thr-Ser-Asp-Tyr-Ser-Lys-Tyr-Leu-Asp-Ser-Arg-Arg-Ala-Gln-Asp-Phe-Val-Gln-Trp-Leu-Met-Asn-Thr-Lys-Arg-Asn-Arg-Asn-Asn-Ile-Ala) (Compound 3). OXM is released into the blood in response to food ingestion and in proportion to meal calorie content. OXM has been shown to suppress appetite and inhibit food intake in humans (Cohen et al, Journal of Endocrinology and Metabolism, 88, 4696-4701. 2003; WO 2003/022304). In addition to these anorectic effects, which are similar to those of GLP-1, OXM must also affect body weight by another mechanism, since rats treated with oxyntomodulin show less body weight gain than pair-fed rats (Bloom, Endocrinology 2004, 145, 2687).

OXM activates both the glucagon receptor and the GLP-1 receptor with a two-fold higher potency for the glucagon receptor over the GLP-1 receptor, but is less potent than native glucagon and GLP-1 on their respective receptors. Glucagon is also capable of activating both receptors, though with a strong preference for the glucagon receptor over the GLP-1 receptor. GLP-1 on the other hand is not capable of activating the glucagon receptor. The mechanism of action of oxyntomodulin is not well understood. In particular, it is not known whether the effects of the hormone are mediated exclusively through the glucagon receptor and the GLP-1 receptor, or through one or more as-yet unidentified receptors.

An eel analogue of oxyntomodulin appears to have an inotropic effect on eel heart (Uesaka et al, J Experimental Biol. 2001; 204, 3019-3026) and inotropic effects have also been documented for oxyntomodulin in mouse (Sowden et al. Am J Phys Regul Integr Comp Physiol. 2007; 292: R962-R970).

### SUMMARY OF THE INVENTION

The present inventors have found that certain compounds can act as inotropic agents, more particularly positive inotropic agents, while having considerably less effect on the heart's energy status than known inotropic agents such as dobutamine, norepinephrine and glucagon. Consequently these compounds are more suitable for use as therapeutic agents than known inotropic agents.

Without wishing to be bound by any particular theory, the useful properties of these compounds may be due to their ability to activate both the glucagon receptor and the GLP-1 receptor. Thus, the compounds which can be used in the methods of the invention will be referred to as glucagon-GLP-1 dual agonists, or simply as "dual agonists".

Thus, the present disclosure provides the use of a glucagon-GLP-1 dual agonist as a positive inotropic agent, in the treatment of heart disease or heart dysfunction.

The invention further provides a glucagon-GLP-1 dual agonist for use as a positive inotropic agent in the treatment of heart disease or heart dysfunction.

The invention further provides a glucagon-GLP-1 dual agonist for use in the preparation of a medicament for the treatment of heart disease or heart dysfunction, wherein the glucagon-GLP-1 dual agonist is to be administered for use as a positive inotropic agent.

The invention further provides the use of a glucagon-GLP-1 dual agonist in the preparation of a medicament for the treatment of heart disease or heart dysfunction, wherein the glucagon-GLP-1 dual agonist is to be administered for use as a positive inotropic agent.

The present disclosure still further provides the use of a glucagon-GLP-1 agonists in the preparation of a medicament cabable of improving cardiac contractility without causing concomitant increase in heart rate.

The disclosure further provides a method of treatment of heart disease or heart dysfunction in a subject, comprising administering a glucagon-GLP-1 dual agonist to the subject as a positive inotropic agent.

Glucagon-GLP-1 dual agonists are well known in the art.

Oxyntomodulin is one example of a naturally-occurring dual agonist. Analogues of oxyntomodulin are described in WO2008/071972 and WO2007/100535.

Other dual agonists are described in WO2008/101017. The majority of those compounds are more similar in length to glucagon than OXM, being around 29 amino acids long, and so can be regarded as analogues of glucagon. However others are longer. Any of the dual agonists described in that document may be suitable for use as described herein. Further dual agonists are described in WO2009/155257 and WO2009/155258 and may also be suitable for use in the methods of the invention.

Still further dual agonists are described in WO2008/152403. PCT/GB2008/004132, PCT/GB2008/004121, PCT/GB2008/004157. PCT/GB20081004130 and European patent application no. 09251780.4, and may also be suitable for use in the methods of the invention.

The dual agonist may be a compound having the formula:

R¹-X-Z¹-Z²-R²

wherein:
R¹ is hydrogen, C₁₋₄ alkyl (e.g. methyl), acetyl, formyl, benzoyl or trifluoroacetyl;
X has the Formula I: 
X1-X2-X3-Gly-Thr-Phe-Thr-Ser-Asp-X10-Ser-X12-Tyr-Leu-X15-X16-X17-X18-Ala-X20-X21-Phe-
X23-X24-Trp-Leu-X27-X28-X29
 wherein
   X1 is His, D-His, (Des-amino)His, hydroxyl-His, acetyl-His, homo-His, alpha,alpha-dimethyl imidiazole acetic acid (DMIA), N-methyl His, alpha-methyl His or imidazole acetic acid;
   X2 is Ser, Aib or D-Ser;
   X3 is Gln, Glu, Orn or Nle;
   X10 is Tyr or Trp;
   X12 is Lys, Arg, His, Ala, Leu, Dpu, Dpr, Orn, Citrulline or Ornithine;
   X15 is Asp, Glu, cysteic acid, homoglutamic acid or homocysteic acid;
   X16 is Ser, Thr, Lys, Arg, His, Glu, Asp, Ala, Gly, Gln, homoglutamic acid or homocysteic acid; X17 is Arg, Lys, His, Glu, Gln, Ala, Leu, Dpu, Dpr, Orn, Cys, homocysteine or acetyl phenylalanine;
   X18 is Arg, Lys, His, Tyr, Ala, Ser, Leu, Cys, Orn, homocysteine or acetyl phenylalanine;
   X20 is Gln, Lys, Arg, His, Glu, Asp, Ala, Cys, Orn or Citrulline;
   X21 is Asp, Glu, Gln, Lys, Cys, Orn, homocysteine or acetyl phenyalanine;
   X23 is Val, Ile or Leu;
   X24 is Gln, Lys, Arg, Glu, Asp, Ser, Ala, Leu, Cys, Orn, homocysteine or acetyl phenyalanine;
   X27 is Met, Lys, Arg, Glu, Leu, Nle, Cys or absent;
   X28 is Asn, Lys, Arg, Glu, Asp, Ser, Ala, Leu, Cys, Citrulline, Orn, or absent;
   X29 is Thr, Lys, Arg, Glu, Ser, Ala, Gly, Cys, Orn, homocysteine, acetyl phenyalanine or absent;
R² is NH₂ or OH;
Z¹ is absent or has the sequence:
   GlyProSerSerGlyAlaProProProSer;
   GlyProSerSerGlyAlaProProProSerCys;
   LysArgAsnArgAsnAsnIleAla; or
   LysArgAsnArg;
Z² is absent or a peptide sequence of 1-20 amino acid units selected from the group consisting of Ala, Leu, Ser, Thr, Tyr, Cys, Glu, Lys, Arg, Dbu, Dpr and Orn;
wherein, if Z¹ is present, X27, X28 and X29 are also present; and
if Z¹ is absent, the compound has a substitution or deletion relative to human glucagon at one or more of positions X1, X2, X3, X10, X12, X15, X16, X17, X18, X20, X21, X23, X24, X27, X28 and X29;
or a pharmaceutically acceptable salt or derivative thereof;
wherein said compound has higher GLP-1 receptor selectivity than human glucagon.

Independently, where present, Z² may be or comprise one or more amino acid residues. For example, Z² may be a γ-Glu (also denoted isoGlu), Glu, β-Ala or ε-Lys residue, or a 4-aminobutanoyl, 8-aminooctanoyl or 8-amino-3,6-dioxaoctanoyl moiety.

The compound may have the formula R¹-X-Z²-R²
wherein
R¹ is hydrogen, alkyl, acetyl, formyl, benzoyl or trifluoroacetyl;
R² is OH or NH₂;
X is a peptide which has the Formula II

His-Ser-Gln-Gly-Thr-Phe-Thr-Ser-Asp-Tyr-Ser-Lys-Tyr-Leu-Asp-Arg-Ala-Arg-Ala-Asp-Asp-Phe-
Val-Ala-Trp-Leu-Lys-Glu-Ala (Compound 4)

or differs from Formula II at up to 4 of the following positions whereby, if different from Formula I: the residue at position 2 is selected from: Aib, D-Ser;
the residue at position 16 is: Lys, Asp, Glu;
the residue at position 18 is selected from: Lys, His, Ala, Ser, Tyr;
the residue at position 20 is selected from: Gln, His, Lys, Arg, Glu;
the residue at position 21 is: Glu;
the residue at position 24 is selected from: Gln, Leu, Glu, Lys, Arg, Asp;
the residue at position 27 is selected from: Met, Cys, Arg, Glu, Leu or is absent;
the residue at position 28 is selected from: Asn, Ser, Arg, Lys, Ala, Leu, Glu, Asp or is absent; and
the residue at position 29 is selected from: Thr, Glu, Lys or is absent;
and Z² is absent or is a sequence of 1-20 amino acid units selected from the group consisting of Ala, Leu, Ser, Thr, Tyr, Cys, Glu, Lys, Arg, Dbu, Dpr and Orn;
or a pharmaceutically acceptable salt thereof,

In some embodiments, X may differ from Formula II at up to 4 of the following positions whereby, if different from Formula II:
the residue at position 2 is selected from: Alb, D-Ser;
the residue at position 18 is selected from: Lys, His, Ala, Ser, Tyr;
the residue at position 20 is selected from: Gln, His, Lys, Arg, Glu;
the residue at position 24 is selected from: Gln, Leu, Glu, Lys, Arg;
the residue at position 27 is selected from: Met, Cys, Arg, Glu, Leu;
the residue at position 28 is selected from: Asn, Ser, Arg, Lys, Ala, Leu; and
the residue at position 29 is selected from: Thr, Glu, Lys.

In other embodiments, X comprises the residues 27-Lys and 28-Ser. In such cases, X may additionally differ from Formula II at one or two of the following positions whereby, if different from Formula II:
the residue at position 2 is selected from: Alb, D-Ser;
the residue at position 18 is selected from: Lys, His, Ala. Ser, Tyr;
the residue at position 20 is selected from: Gln, His, Lys, Arg, Glu;
the residue at position 24 is selected from: Gln, Leu, Glu, Lys, Arg; and
the residue at position 29 is selected from: Thr, Glu, Lys.
in any of the embodiments described above, the residues at positions 16 and 20 may be capable of forming a salt bridge. Examples of suitable pairs of residues include:
   16-Asp, 20-Lys;
   16-Glu, 20-Lys;
   16-Asp, 20-Arg;
   16-Glu, 20-Arg;
   16-Lys, 20-Asp;
   16-Arg, 20-Asp;
   16-Lys, 20-Glu; and
   16-Arg, 20-Glu.

While maintaining consistency with the definitions above, it may be desirable that X comprises one or more of the following sets of residues:
16-Arg;
16-Arg, 20-Asp;
16-Arg, 20-Asp, 24-Ala;
16-Arg, 20-Asp, 27-Lys, 28-Ser;
16-Arg, 20-Asp, 29-Ala;
16-Arg, 27-Lys, 28-Ser;
16-Arg, 27-Lys, 28-Ser, 29-Ala;
24-Ala, 27-Lys, 28-Ser;
24-Ala, 27-Lys, 28-Ser, 29-Ala;
24-Ala;
27-Lys;
28-Ser;
20-Glu, 28-Ser, 29-Thr;
24-Glu, 28-Ser, 29-Thr;
27-Glu, 28-Arg;
2-D-Ser, 28-Ser, 29-Thr; or
20-His, 28-Ser, 29-Thr.

For example, X may have the sequence:
HSQGTFTSDYSKYLDRARADDFVAWLKSA; (Compound 5)
HSQGTFTSDYSKYLDRARADDFVAWLKEA; (Compound 6)
HSQGTFTSDYSKYLDRARAEDFVAWLKST; (Compound 7)
HSQGTFTSDYSKYLDRARADDFVEWLKST; (Compopund 8)
HSQGTFTSDYSKYLDRARADDFVAWLERA; (Compound 9)
H-DSer-QGTFTSDYSKYLDkARADDFVAWLKST; (Compound 10)
HSQGTFTSDYSKYLDRARAHDFVAWLKST; or (Compound 11)
HSQGTFTSDYSKYLDRARADDFVAWLKST. (Compound 12)

The peptides defined by Formula II may carry one or more intramolecular bridge within the peptide sequence X. Each such bridge may suitably be formed between the side chains of two amino acid residues of X which are typically separated by three amino acids in the linear sequence of X (i.e. between amino acid A and amino acid A+4).

More particularly, the bridges may be formed between the side chains of residue pairs 12 and 16, 16 and 20, 17 and 21, 20 and 24, or 24 and 28. The two side chains can be linked to one another through ionic interactions or by covalent bonds. Thus these pairs of residues may comprise oppositely charged side chains in order to form a salt bridge by ionic interactions. For example, one of the residues may be Glu or Asp, while the other may be Lys or Arg. The pairings of Lys and Glu and Lys and Asp, may also be capable of reacting to form a lactam ring. Likewise, a Tyr and a Glu or a Tyr and an Asp are capable of forming a lactone ring.

In particular, residues at positions 16 and 20 may be capable of forming an intramolecular bridge. Examples of suitable pairs of residues at these positions include:
16-Asp, 20-Lys;
16-Glu, 20-Lys;
16-Asp, 20-Arg;
16-Glu, 20-Arg;
16-Lys, 20-Asp;
16-Arg, 20-Asp;
16-Lys, 20-Glu; and
16-Arg, 20-Glu.

The compound may have the formula R¹-X-Z²-R²
wherein
R¹ is H, C₁₋₄ alkyl, acetyl, formyl, benzoyl or trifluoroacetyl;
R² is OH or NH₂;
X is a peptide which has the Formula III: 
His-Ser-Gln-Gly-Thr-Phe-Thr-Ser-Asp-Tyr-Ser-Leu-Tyr-Leu-Asp-Ser-Arg-Arg-Ala-Lys-Asp-Phe-
Ile-Glu-Trp-Leu-Glu-Ser-Ala (Compound 13)
or differs from Formula III at up to 4 of the following positions whereby, if different from Formula III:
the residue at position 2 is selected from: Aib, D-Ser;
the residue at position 16 is selected from: Arg, His, Lys, Glu, Gly, Asp;
the residue at position 17 is selected from: Lys, Leu;
the residue at position 18 is selected from: Lys, His, Ala, Ser, Tyr;
the residue at position 20 is selected from: Gln, His, Arg, Glu, Asp;
the residue at position 21 is: Glu;
the residue at position 23 is selected from: Val, Leu;
the residue at position 24 is selected from: Gln, Leu, Ala, Lys, Arg, Asp;
the residue at position 27 is selected from: Met, Cys, Lys, Arg, Leu or is absent;
the residue at position 28 is selected from: Asn, Arg, Lys, Glu, Ala, Leu, Asp or is absent; and
the residue at position 29 is selected from: Thr, Glu, Lys or is absent;
and Z² is absent or a peptide sequence of 1-20 amino acid units selected from the group consisting of Ala, Leu, Ser, Thr, Tyr, Cys, Glu, Lys, Arg, Dbu, Dpr and Orn;
or a pharmaceutically acceptable salt thereof.

In some embodiments, X differs from Formula III at up to 4 of the following positions whereby, if different from Formula III:
the residue at position 2 is selected from: Aib, D-Ser;
the residue at position 16 is selected from: Arg, His, Lys, Glu, Gly;
the residue at position 17 is selected from: Lys, Leu;
the residue at position 18 is selected from: Lys, His, Ala, Ser, Tyr;
the residue at position 23 is selected from: Val, Leu;
the residue at position 27 is selected from: Met, Cys, Lys, Arg, Leu;
the residue at position 28 is selected from: Asn, Arg, Lys, Glu, Ala, Leu; and
the residue at position 29 is selected from: Thr, Glu, Lys;

In some embodiments, X differs from Formula III at up to 4 of the following positions whereby, if different from Formula III:
the residue at position 2 is selected from: Alb, D-Ser;
the residue at position 16 is selected from: Arg, His, Lys, Glu, Gly;
the residue at position 17 is selected from: Lys, Leu;
the residue at position 18 is selected from: Lys, His, Ala, Ser, Tyr; and
the residue at position 23 is selected from: Val, Leu.

In some embodiments, X differs from Formula III at up to 4 of the following positions whereby, if different from Formula III:
the residue at position 2 is selected from: Aib, D-Ser;
the residue at position 23 is selected from: Val, Leu;
the residue at position 27 is selected from: Met, Cys, Lys, Arg, Leu;
the residue at position 28 is selected from: Asn, Arg, Lys, Glu, Ala, Leu; and
the residue at position 29 is selected from: Thr, Glu, Lys.

While maintaining consistency with the definitions above, it may be desirable that X comprises one or more of the following sets of residues:
20-Lys, 24-Glu;
20-Lys, 24-Glu, 29-Ala;
20-Lys, 23-Ile, 24-Glu;
27-Glu, 28-Ser, 29-Ala;
29-Ala;
20-Gln;
23-Val;
24-Gln;
29-Thr;
27-Met, 28-Asn, 29-Thr;
20-Gln, 23-Val, 24-Gln;
20-Glu, 24-Lys; or
28-Arg.

For example, X may have the sequence:
HSQGTFTSDYSLYLDSRRAQDFIEWLESA; (Compound 14)
HSQGTFTSDYSLYLDSRRAKDFVEWLESA; (Compound 15)
HSQGTFTSDYSLYLDSRRAKDFIQWLESA; (Compound 16)
HSQGTFTSDYSLYLDSRRAKDFIEWLEST; (Compound 17)
HSQGTFTSDYSLYLDSRRAKDFIEWLMNT; (Compound 18)
HSQGTFTSDYSLYLDSRRAQDFVQWLESA; (Compound 19)
HSQGTFTSDYSLYLDSRRAEDFIKWLESA; or (Compound 20)
HSQGTFTSDYSLYLDSRRAKDFIEWLERA. (Compound 21)

The peptides defined by Formula III may carry one or more intramolecular bridges within the peptide sequence X. Each such bridge may suitably be formed between the side chains of two amino acid residues of X which are typically separated by three amino acids in the linear sequence of X (i.e. between amino acid A and amino acid A+4).

More particularly, the bridge may be formed between the side chains of residue pairs 16 and 20, 17 and 21, 20 and 24, or 24 and 28. The two side chains can be linked to one another through ionic interactions, or by covalent bonds. Thus these pairs of residues may comprise oppositely charged side chains in order to form a salt bridge by ionic interactions. For example, one of the residues may be Glu or Asp, while the other may be Lys or Arg. The pairings of Lys and Glu and Lys and Asp, may also be capable of reacting to form a lactam ring. Likewise, a Tyr and a Glu or a Tyr and a Asp are capable of forming a lactone ring.

In particular, the residues at positions 20 and 24 may be capable of forming an intramolecular bridge. Examples of suitable pairs of residues at these positions include:
20-Asp, 24-Lys;
20-Glu, 24-Lys;
20-Asp, 24-Arg;
20-Glu, 24-Arg;
20-Lys, 24-Asp;
20-Arg, 24-Asp;
20-Lys, 24-Glu; and
20-Arg, 24-Glu.

Without wishing to be bound by any particular theory, it is believed that such intramolecular bridges stabilise the alpha helical structure of the molecule and so increase potency and/or selectivity at the GLP-1 receptor and possibly also at the glucagon receptor.

The compound may have the formula R¹-X-Z²-R²wherein
R¹ is H, C₁₋₄ alkyl, acetyl, formyl, benzoyl or trifluoroacetyl;
R² is OH or NH₂;
X is a peptide which has the Formula IV: 
His-Ser-Gln-Gly-Thr-Phe-Thr-Ser-Asp-Tyr-Ser-Lys-Tyr-Leu-Asp-Glu-Arg-Arg-Ala-Lys-Asp-Phe-
Ile-Glu-Trp-Leu-Leu-Ser-Ala (Compound 22)
 or differs from formula IV at up to 4 of the following positions whereby, if different from Formula IV:
the residue at position 2 is selected from: D-Ser, Aib;
the residue at position 16 is selected from: Ser, Asp; Lys, Arg;
the residue at position 18 is: Ala;
the residue at position 20 is selected from: Gln, Arg, Glu, Asp;
the residue at position 21 is: Glu;
the residue at position 23 is: Val;
the residue at position 24 is selected from: Gln, Asp, Lys, Arg, Ala;
the residue at position 27 is selected from: Met, Cys, Lys or is absent;
the residue at position 28 is selected from: Asn, Arg, Lys, Ala, Glu, Asp or is absent; and the
residue at position 29 is selected from: Thr, Arg or is absent;
and Z² is absent or a sequence of 1-20 amino acid units selected from the group consisting of Ala, Leu, Ser, Thr, Tyr, Cys, Glu, Lys, Arg, Dbu, Dpr and Orn;
or a pharmaceutically acceptable salt thereof.

In some embodiments, X differs from Formula IV at up to 4 of the following positions whereby, if different from Formula IV:
the residue at position 2 is selected from: D-Ser, Alb;
the residue at position 16 is selected from: Ser, Asp, Lys;
the residue at position 20 is selected from: Gln, Arg, Glu;
the residue at position 27 is selected from: Met, Cys, Lys; and
the residue at position 28 is selected from: Asn, Arg, Ala.

In some of those embodiments, X may differ from Formula IV at up to 3 of the following positions whereby, if different from Formula IV:
the residue at position 2 is selected from: D-Ser, Aib;
the residue at position 16 is selected from: Ser, Asp, Lys; and
the residue at position 20 is selected from: Gln, Arg, Glu.

In alternative embodiments, X may differ from Formula IV at up to 4 of the following positions whereby, if different from Formula IV:
the residue at position 2 is selected from: D-Ser, Aib;
the residue at position 16 is selected from: Ser, Asp, Lys;
the residue at position 18 is: Ala; and
the residue at position 20 is selected from: Gln, Arg, Glu.

In still further alternative embodiments, X may differ from Formula IV at up to 4 of the following positions whereby, if different from Formula IV:
the residue at position 23 is: Val;
the residue at position 24 is selected from: Gln, Asp, Lys, Arg, Ala;
the residue at position 27 is selected from: Met, Cys, Lys; and
the residue at position 28 is selected from: Asn, Arg, Ala.

In any of the embodiments described above, the residues at positions 16 and 20 may be capable of forming a salt bridge. Examples of suitable pairs of residues include:
16-Asp, 20-Lys;
16-Glu, 20-Lys;
16-Asp, 20-Arg;
16-Glu, 20-Arg;
16-Lys, 20-Asp;
16-Arg, 20-Asp;
16-Lys, 20-Glu;
16-Arg, 20-Glu.

Additionally or alternatively, the residues at positions 20 and 24 may be capable of forming a salt bridge. Examples of suitable pairs of residues include:
20-Asp, 24-Lys;
20-Glu, 24-Lys;
20-Asp, 24-Arg;
20-Glu, 24-Arg;
20-Lys, 24-Asp;
20-Arg, 24-Asp;
20-Lys, 24-Glu;
20-Arg, 24-Glu.

While maintaining consistency with the definitions above, it may be desirable that X comprises one or more of the following sets of residues:
20-Lys, 24-Glu;
20-Lys, 23-Ile, 24-Glu;
16-Glu, 20-Lys, 24-Glu;
16-Glu, 20-Lys;
16-Glu, 20-Lys, 29-Ala;
16-Glu, 20-Lys, 23-Ile, 24-Glu;
16-Glu, 20-Lys, 23-Ile, 24-Glu, 29-Ala;
16-Glu, 20-Lys, 24-Glu, 29-Ala;
20-Lys, 23-Ile, 24-Glu, 29-Ala;
27-Leu, 28-Ser, 29-Ala;
29-Ala;
16-Ser;
20-Gln;
23-Val;
24-Gln;
16-Ser, 20-Gln;
16-Asp, 20-Arg, 24-Asp;
16-Lys, 20-Glu;
24-Arg; or
28-Arg.

For example, X may have the sequence:
HSQGTFTSDYSKYLDERRAGIDFIEWLLSA; (Compound 23)
HSQGTFTSDYSKYLDERRAKDFVEWLLSA; (Compound 24)
HSQGTFTSDYSKYLDERRAKDFIQWLLSA; (Compound 25)
HSQGTFTSDYSKYLDSRRAQDFIEWLLSA; (Compound 26)
HSQGTFTSDYSKYLDDRRARDFIDWLLSA; (Compound 27)
HSQGTFTSDYSKYLDKRRAEDFIKWLLSA; (Compound 28)
HSQGTFTSDYSKYLDERRAKDFIRWLLSA; (Compound 29)
HSQGTFTSDYSKYLDERRAKDFIEWLLRA; (Compound 30)
HSQGTFTSDYSKYLDSRRAKDFIEWLLSA; (Compound 31)
HSQGTFTSDYSKYLDERAAKDFIEWLLSA; (Compound 32)
HSQGTFTSDYSKYLDERRAKDFIDWLLSA; (Compound 33)
HSQGTFTSDYSKYLDERRAKDFIEWLLAA; or (Compound 34)
HSQGTFTSDYSKYLDERRAKDFIEWLLSA. (Compound 35)

The peptides defined by Formula IV may carry one or more intramolecular bridge within the peptide sequence X. Each such bridge may suitably be formed between the side chains of two amino acid residues of X which are typically separated by three amino acids in the linear sequence of X (i.e. between amino acid A and amino acid A+4).

More particularly, the bridge may be formed between the side chains of residue pairs 12 and 16, 16 and 20, 17 and 21, 20 and 24, or 24 and 28. The two side chains can be linked to one another through ionic interactions, or by covalent bonds. Thus these pairs of residues may comprise oppositely charged side chains in order to form a salt bridge by ionic interactions. For example, one of the residues may be Glu or Asp, while the other may be Lys or Arg. The pairings of Lys and Glu and Lys and Asp, may also be capable of reacting to form a lactam ring. Likewise, a Tyr and a Glu or a Tyr and a Asp are capable of forming a lactone ring.

In particular, the residues at positions 16 and 20, and/or 20 and 24 may be capable of forming an intramolecular bridge. Examples of suitable pairs of residues at these positions include:
16-Asp, 20-Lys;
16-Glu, 20-Lys;
16-Asp, 20-Arg;
16-Glu, 20-Arg;
16-Lys, 20-Asp;
16-Arg, 20-Asp;
16-Lys, 20-Glu;
16-Arg, 20-Glu; and/or

20-Asp, 24-Lys;
20-Glu, 24-Lys;
20-Asp, 24-Arg;
20-Glu, 24-Arg;
20-Lys, 24-Asp;
20-Arg, 24-Asp;
20-Lys, 24-Glu;
20-Arg, 24-Glu.

Without wishing to be bound by any particular theory, it is believed that such intramolecular bridges stabilise the alpha helical structure of the molecule and so increase potency and/or selectivity at the GLP-1 receptor and possibly also the glucagon receptor.

The compound may have the formula R¹-X-Z²-R²
wherein
R¹ is H, C₁₋₄ alkyl, acetyl, formyl, benzoyl or trifluoroacetyl;
R² is OH or NH₂;
X is a peptide which has the Formula V: 
His-Ser-Gln-Gly-Thr-Phe-Thr-Ser-Asp-Tyr-Ser-Lys-Tyr-Leu-Asp-Ser-Lys-Ala-Ala-His-Asp-Phe-
Val-Glu-Trp-Leu-Leu-Arg-Ala (Compound 36)
   or differs from Formula V at up to 4 of the following positions whereby, if different from Formula V: the residue at position 2 is selected from: Aib, D-Ser;
   the residue at position 12 is selected from: Leu, Arg, Dpu, Dpr, Orn;
   the residue at position 16 is selected from: Arg, His, Lys, Glu, Asp;
   the residue at position 17 is selected from: Arg, Leu, Dpu, Dpr, Orn;
   the residue at position 18 is selected from: Arg, Lys, His, Ser, Tyr;
   the residue at position 20 is selected from: Gln, Lys, Arg, Glu, Asp;
   the residue at position 21 is Glu;
   the residue at position 24 is selected from: Gln, Leu, Ala, Lys, Arg, Asp;
   the residue at position 27 is selected from: Met, Cys, Lys, Arg, Glu or is absent;
   the residue at position 28 is selected from: Asn, Ser, Lys, Glu, Ala, Leu, Asp or is absent; and
   the residue at position 29 is selected from: Thr, Glu, Lys or is absent;
and Z² is absent or a peptide sequence of 1-20 amino acid units selected from the group consisting of Ala, Leu, Ser, Thr, Tyr, Cys, Glu, Lys, Arg, Dbu, Dpr and Orn;
or a pharmaceutically acceptable salt thereof.

In certain embodiments of this aspect, X may differ from Formula V at up to 4 of the following positions whereby, if different from Formula V:
the residue at position 2 is selected from: Aib, D-Ser;
the residue at position 16 is selected from: Arg, His, Lys, Glu;
the residue at position 17 is selected from: Arg, Leu;
the residue at position 18 is selected from: Arg, Lys, His, Ser, Tyr;
the residue at position 20 is selected from: Gln, Lys, Arg, Glu;
the residue at position 24 is selected from: Gln, Leu, Ala, Lys, Arg;
the residue at position 27 is selected from: Met, Cys, Lys, Arg, Glu;
the residue at position 28 is selected from: Asn, Ser, Lys, Glu, Ala, Leu; and
the residue at position 29 is selected from: Thr, Glu, Lys.

In certain embodiments of this aspect, X may differ from Formula V at up to 4 of the following positions wnereby, it different from Formula V:
the residue at position 2 is selected from: Alb, D-Ser;
the residue at position 16 is selected from: Arg, His, Lys, Glu, Gly;
the residue at position 24 is selected from: Gln, Leu, Ala, Lys, Arg;
the residue at position 27 is selected from: Met, Cys, Lys, Arg, Glu;
the residue at position 28 is selected from: Asn, Ser, Lys, Glu, Ala, Leu; and
the residue at position 29 is selected from: Thr, Glu, Lys.

While maintaining consistency with the definitions relating to Formula V above, it may be desirable that X comprises one or more of the following sets of residues:
17-Lys, 18-Ala;
17-Leu, 18-Ala;
17-Lys, 18-Ala, 20-His;
17-Leu, 18-Ala, 20-His;
17-Lys, 18-Ala. 24-Glu;
17-Leu, 18-Ala, 24-Glu;
17-Lys, 18-Ala, 27-Leu;
17-Leu, 18-Ala, 27-Leu;
17-Lys, 18-Ala, 29-Ala;
17-Leu, 18-Ala, 29-Ala;
17-Lys, 18-Ala, 27-Leu, 29-Ala;
17-Leu, 18-Ala, 27-Leu, 29-Ala;
17-Lys, 18-Ala, 27-Leu, 28-Arg, 29-Ala;
17-Leu, 18-Ala, 27-Leu, 28-Arg, 29-Ala;
24-Glu, 28-Arg;
24-Glu, 28-Arg, 27-Leu;
24-Glu, 28-Arg, 27-Leu, 29-Ala;
27-Leu, 28-Arg, 29-Ala;
29-Ala;
20-Arg, 24-Arg, 27-Lys, 28-Leu;
17-Arg;
18-Arg;
20-Gln;
24-Gln;
27-Met, 28-Asn, 29-Thr; or
24-Lys
and combinations thereof.

For example, X may have the sequence:
HSQGTFTSDYSKYLDSKAARDFVRWLKLA; (Compound 37)
HSQGTFTSDYSKYLDSRAAHDFVEWLLRA; (Compound 38)
HSQGTFTSDYSKYLDSKRAHDFVEWLLRA; (Compound 39)
HSQGTFTSDYSKYLDSKAAQDFVEWLLRA; (Compound 40)
HSQGTFTSDYSKYLDSKAAHDFVQWLLRA; (Compound 41)
HSQGTFTSDYSKYLDSKAAHDFVEWLMNT; (Compound 42)
HSQGTFTSDYSKYLDSKAAHDFVKWLLRA; (Compound 43)
H-DSer-QGTFTSDYSKYLDSKAAHDFVEWLLRA; (Compound 44)
H-Alb-QGTFTSDYSKYLDSKAAHDFVEWLLRA; (Compound 45)
HSQGTFTSDYSKYLDSKAAKDFVEWLLRA; (Compound 46)
HSQGTFTSDYSKYLDKKAAHDFVEWLLRA or (Compound 47)
HSQGTFTSDYSKYLDSKAAHDFVEWLLRA (Compound 48)

In an alternative aspect, the compound may have the formula R¹-X-Z²-R²
wherein
R¹ is H, C₁₋₄ alkyl, acetyl, formyl_{;} benzoyl or trifluoroacetyl;
R² is OH or NH₂;
X is a peptide which has the Formula VI: 
His-Ser-Gln-Gly-Thr-Phe-Thr-Ser-Asp-Tyr-Ser-Lys-Tyr-Leu-Asp-Ser-Lys-Ala-Ala-His-Asp-Phe-
Val-Glu-Trp-Leu-Leu-Arg-Ala (Compound 49)
 or differs from Formula VI at up to 5 of the following positions whereby, if different from Formula VI:
   the residue at position 2 is selected from: Aib, D-Ser;
   the residue at position 16 is selected from: Arg, His, Lys, Glu;
   the residue at position 17 is: Arg, Leu, Dpu, Dpr, Orn;
   the residue at position 20 is selected from: Gln, Lys. Arg, Glu, Asp;
   the residue at position 21 is Glu;
   the residue at position 24 is selected from: Gln, Leu, Ala, Lys, Arg, Asp;
   the residue at position 27 is selected from: Met, Cys, Lys, Arg, Glu or is absent;
   the residue at position 28 is selected from: Asn, Ser, Lys, Glu, Ala, Leu, Asp or is absent; and
   the residue at position 29 is selected from: Thr, Glu, Lys or is absent;
   and Z² is absent or a peptide sequence of 1-20 amino acid units selected from the group consisting of Ala, Leu, Ser, Thr, Tyr, Cys, Glu, Lys, Arg, Dbu, Dpr and Orn;
or a pharmaceutically acceptable salt thereof.

In certain embodiments of this aspect, X may differ from Formula VI at up to 4 of the following positions whereby, if different from Formula VI:
the residue at position 2 is selected from: Aib, D-Ser;
the residue at position 16 is selected from: Arg, His, Lys, Glu, Gly;
the residue at position 17 is selected from: Arg, Leu;
the residue at position 18 is selected from: Arg, Lys, His, Ser, Tyr;
the residue at position 20 is selected from: Gln, Lys, Arg, Glu;
the residue at position 24 is selected from: Gln, Leu, Ala, Lys, Arg;
the residue at position 27 is selected from: Met, Cys, Lys, Arg, Glu;
the residue at position 28 is selected from: Asn, Ser, Lys, Glu, Ala, Leu; and
the residue at position 29 is selected from: Thr, Glu, Lys.

While maintaining consistency with the definitions in relation to Formula VI above, it may be desirable that X comprises any of the sets of residues described above in relation to the first aspect, or one or more of the following sets of residues:
20-Gln, 24-Gln, 27-Met, 28-Asn, 29-Thr; or
17-Leu, 20-Gln, 24-Gln, 28-Asn, 29-Thr.
X may have the sequence:
   HSQGTFTSDYSKYLDSKAAQDFVQWLMNT or (Compound 50)
   HSQGTFTSDYSKYLDSLAAQDFVQWLLNT (Compound 51)

The peptides defined by Formulae V and VI may carry one or more intramolecular bridge within the peptide sequence X. Each such bridge may suitably be formed between the side chains of two amino acid residues of X which are typically separated by three amino acids in the linear sequence of X (i.e. between amino acid A and amino acid A+4).

More particularly, the bridge may be formed between the side chains of residue pairs 12 and 16, 16 and 20, 17 and 21, 20 and 24, or 24 and 28. The two side chains can be linked to one another through ionic interactions or by covalent bonds. Thus these pairs of residues may comprise oppositely charged side chains in order to form a salt bridge by ionic interactions. For example, one of the residues may be Giu or Asp, while the other may be Lys or Arg. The pairings of Lys and Glu and Lys and Asp, may also be capable of reacting to form a lactam ring. Likewise, a Tyr and a Glu or a Tyr and a Asp are capable of forming a lactone ring.

In particular, residues at positions 16 and 20 may be capable of forming an intramolecular bridge. Examples of suitable pairs of residues at these positions include:
16-Asp, 20-Lys;
16-Glu, 20-Lys;
16-Asp, 20-Arg;
16-Glu, 20-Arg;
16-Lys, 20-Asp;
16-Arg, 20-Asp;
16-Lys, 20-Glu; and
16-Arg, 20-Glu.

Without wishing to be bound by any particular theory, it is believed that such intramolecular bridges stabilise the alpha helical structure of the molecule and so increase potency and/or selectivity at the GLP-1 receptor and possibly also the glucagon receptor..

Without wishing to be bound by any particular theory, the arginine residues at positions 17 and 18 of native glucagon appear to provide significant selectivity for the glucagon receptor. A hydrophobic residue (e.g. Ala) at position 18 may also increase potency at both GLP-1 and glucagon receptors. It may also increase enzymatic stability compared to native glucagon.

Without wishing to be bound by any particular theory, the residues at positions 27, 28 and 29 of native glucagon appear to provide significant selectivity for the glucagon receptor. Substitutions at one, two, or all three of these positions with respect to the native glucagon sequence may increase potency at and/or selectivity for the GLP-1 receptor, potentially without significant reduction of potency at the glucagon receptor. Particular examples include Leu or Lys at position 27, Arg or Ser at position 28 and Ala at position 29.

Substitution of the naturally-occurring Met residue at position 27 (e.g. with Leu, Lys, Arg or Glu) also reduces the potential for oxidation, so increasing the chemical stability of the compounds.

Substitution of the naturally-occurring Asn residue at position 28 (e.g. by Glu, Ser, Arg, Lys, Ala or Leu) also reduces the potential for deamidation in acidic solution, so increasing the chemical stability of the compounds.

Potency and/or selectivity at the GLP-1 receptor may also be increased by introducing residues that are likely to form an amphipathic helical structure, potentially without significant loss of potency at the glucagon receptor. This may be achieved by introduction of charged residues at one or more of positions 16, 20, 24, and 28. Thus the residues of positions 16 and 20 may all be charged, the residues at positions 16, 20, and 28 may all be charged, or the residues at positions 16, 20, 24, and 28 may all be charged. The presence of charged residues at position 16 and 20 may be particularly desirable when they are capable of forming an intramolecular bridge, e.g. when they are oppositely charged amino acids, such as Arg at position 16 and Asp or Glu at position 20 or Glu at position 16 and His or Lys at position 20.

Substitution of one or both of the naturally-occurring Gln residues at positions 20 and 24 also reduces the potential for deamidation in acidic solution, so increasing the chemical stability of the compounds. For example, the compounds may have Asp or His at position 20 and Ala in position 24, optionally also with Ser, Glu or Arg at position 28:
The compound may have the formula R¹-X-Z¹-Z²-R²
   wherein:
   R¹ is hydrogen, alkyl (e.g. methyl), acetyl, formyl, benzoyl or trifluoroacetyl;
   wherein X has the Formula VII: 
X1-X2-X3-Gly-Thr-Phe-Thr-Ser-Asp-X10-Ser-X12-Tyr-Leu-X15-X16-X17-X18-Ala-X20-X21-Phe-
X23-X24-Trp-Leu-X27-X28-X29
 wherein
      X1 is His, D-His, (Des-amino)His, hydroxyl-His, acetyl-His, homo-His, alpha,alpha-dimethyl imidiazole acetic acid (DMIA), N-methyl His, alpha-methyl His, or imidazole acetic acid;
      X2 is Ser, D-Ser, Ala, D-Ala, Val, Gly, N-methyl Ser, aminoisobutyric acid (Aib) or N-methyl Ala; X3 is Gln, Glu, Om or Nle;
      X10 is Tyr or Trp;
      X12 is Lys, Citrulline, Orn or Arg;
      X15 is Asp, Glu, cysteic acid, homoglutamic acid or homocysteic acid;
      X16 is Ser, Glu, Gln, homoglutamic acid or homocysteic acid;
      X17 is Arg, Gln, Lys, Cys, Orn, homocysteine or acetyl phenylalanine;
      X16 is Arg, Ala, Lys, Cys, Orn, homocysteine or acetyl phenylalanine;
      X20 is Gln, Lys, Arg, Om or Citrulline;
      X21 is Gln, Glu, Asp, Lys, Cys, Om, homocysteine or acetyl phenyalanine;
      X23 is Val or Ile;
      X24 is Ala, Gln, Glu, Lys, Cys, Om, homocysteine or acetyl phenyalanine;
      X27 is Met, Leu or Nle;
      X28 is Asn, Arg, Citrulline, Orn, Lys or Asp;
      X29 is Thr, Gly, Lys, Cys, Orn, homocysteine or acetyl phenyalanine;
   R² is NH₂ or OH;
   Z¹ is absent or has the sequence:
      GlyProSerSerGlyAlaProProProSer;
      GlyProSerSerGlyAlaProProProSerCys;
      LysArgAsnArgAsnAsnIleAla; or
      LysArgAsnArg;
   Z² is absent or a peptide sequence of 1-20 amino acid units selected-from the group consisting of Ala, Leu, Ser, Thr, Tyr, Asn, Gln, Asp, Glu, Lys, Arg, His, Met, Har, Dbu, Dpr and Om;
   wherein, if Z¹ is absent, the compound has a substitution or deletion relative to human glucagon at one or more of positions X1, X2, X3, X10, X12, X15, X16, X17, X18, X20, X21, X23, X24, X27, X28 and X29;
   or a pharmaceutically acceptable salt or derivative thereof;
   wherein said compound has higher GLP-1 receptor selectivity than human glucagon and/or
   wherein the compound exhibits at least 20% of the activity of native GLP-1 at the GLP-1 receptor.

In addition, in certain embodiments, X may differ from Formula VII by 1 to 3 amino acid modifications at positions selected from 1, 2, 3, 5, 7, 10, 11, 13, 14, 17, 18, 19, 21, 24, 27, 28 and 29.

Compounds having sequences according to Formula VII are described in WO2008/101017.

X may have the Formula VII.2: 
His-Ser-Gln-Gly-Thr-Phe-Thr-Ser-Asp-Tyr-Ser-Lys-Tyr-Leu- Asp-X16-X17-Arg-Ala-Gln-Asp-Phe-
Val-Gln-Trp-Leu-X27-Asn-Thr (Compound 52)
 wherein
X16 is Glu, Gln, homoglutamic acid or homocysteic acid;
X17 is Arg, Cys, Orn, homocysteine or acetyl phenylalanine;
X27 is Met, Leu or Nle

X may have the Formula VII.3: 
His-Ser-Gln-Gly-Thr-Phe-Thr-Ser-Asp-Tyr-Ser-Lys-Tyr-Leu-Asp-X16-Arg-Arg-Ala-Gln-X21-Phe-
Val-Gln-Trp-Leu-X27- Asn-Thr (Compound 53)
 wherein
X16 is Glu, Gln, homoglutamic acid or homocysteic acid;
X21 is Asp, Cys, Orn, homocysteine or acetyl phenylalanine;
X27 is Met, Leu or Nle;

X may have the Formula VII.4: 
His-Ser-Gln-Gly-Thr-Phe-Thr-Ser-Asp-Tyr-Ser-Lys-Tyr-Leu-Asp-X16-Arg-Arg-Ala-Gln-X21-Phe-
Val-Gln-Trp-Leu-X27- Asn-Thr (Compound 54)
 wherein
X16 is Glu, Gln, homoglutamic acid or homocysteic acid;
X24 is Gln, Cys, Orn, homocysteine or acetyl phenylalanine;
X27 is Met, Leu or Nle.

X may have the Formula VII.5: 
His-Ser-Gln-Gly-Thr-Phe-Thr-Ser-Asp-Tyr-Ser-Lys-Tyr-Leu-Asp-X16-Arg-Arg-Ala-Gln-X21-Phe-
Val-X24-Trp-Leu-X27- Asn-Thr (Compound 55)
 wherein
X16 is Glu, Gln, homoglutamic acid or homocysteic acid;
X21 is Asp, Cys, Orn, homocysteine or acetyl phenylalanine;
X24 is Gln, Cys, Orn, homocysteine or acetyl phenylalanine;
X27 is Met, Leu or Nle.

X may have the Formula VII.6: 
His-Ser-Gln-Gly-Thr-Phe-Thr-Ser-Asp-Tyr-Ser-Lys-Tyr-Leu-Asp-Glu-Arg-Arg-Ala-Gln-X21-Phe-
Val-Gln-Trp-Leu-X27- Asn-Thr (Compound 56)
 wherein
X21 is Asp, Cys, Om, homocysteine or acetyl phenylalanine;
X27 is Met, Leu or Nle.

X may have the Formula VII.7: 
His-Ser-Gln-Gly-Thr-Phe-Thr-Ser-Asp-Tyr-Ser-Lys-Tyr-Leu-Asp-Glu-Arg-Arg-Ala-Gln-Asp-Phe-
Val-X24-Trp-Leu-X27- Asn-Thr (Compound 57)
 wherein
X24 is Gln, Cys, Orn, homocysteine or acetyl phenylalanine;
X27 is Met, Leu or Nle.

X may have the Formula VII.8: 
His-Ser-Gln-Gly-Thr-Phe-Thr-Ser-Asp-Tyr-Ser-Lys-Tyr-Leu-Asp-X16-Arg-Arg-Ala-Gln-Asp-Phe-
Val-Gln-Trp-Leu-Met- Asn-Thr (Compound 58)
 wherein
X16 is Glu, Gln, homoglutamic acid or homocysteic acid.

X may have the Formula VII.9: 
His-Ser-Gln-Gly-Thr-Phe-Thr-Ser-Asp-Tyr-Ser-Lys-Tyr-Leu-Asp-Glu-Arg-Arg-Ala-Gln-Asp-Phe-
Val-Gln-Trp-Leu-X27- Asn-Thr (Compound 59)
 wherein
X27 is Met, Leu or Nle.

X may have the Formula VII.19: 
His-Ser-Gln-Gly-Thr-Phe-Thr-Ser-Asp-Tyr-Ser-Lys-Tyr-Leu-Asp-Glu-Arg-Arg-Ala-Gln-Asp-Phe-
Val-Glu-Trp-Leu-Met-Asn-Thr-X30 (Compound 60)
 wherein
X30 is any suitable amino acid.

X may have the Formula VII.20: 
His-Ser-Gln-Gly-Thr-Phe-Thr-Ser-Asp-Tyr-Ser-Lys-Tyr-Leu- Asp-X16-Arg-Arg- Ala-X20-Asp-Phe-
Val-X24-Trp-Leu-Met-X28-X29 (Compound 61)
 wherein
X16 is Ser, Glu, Gln, homoglutamic acid or homocysteic acid;
X20 is Gln, Lys, Arg, Orn or Citrulline;
X24 is Gln or Glu;
X28 is Asn, Asp or Lys;
X29 is Thr or Gly.

X may have the Formula VII.21: 
His-X2-Gln-Gly-Thr-Phe-Thr-Ser-Asp-Tyr-Ser-Lys-Tyr-Leu- Asp-Glu-Arg-Arg- Ala-Gln-Asp-Phe-
Val-Gln-Trp-Leu-Met-Asn-Thr (Compound 62)
 wherein
X2 is D-Ser; Ala, Gly, N-methyl Ser or aminoisobutyric acid.

X may have the Formula VII.22: 
His-X2-Gln-Gly-Thr-Phe-Thr-Ser-Asp-Tyr-Ser-Lys-Tyr-Leu- Asp-Glu-Arg-Arg- Ala-Gln-Asp-Phe-
Val-Gln-Trp-Leu-Met-Asn-Thr (Compound 63)
 wherein
X2 is aminoisobutyric acid.

X may have the Formula VII.23:
His-Ser-Gln-Gly-Thr-Phe-Thr-Ser-Asp-Tyr-Ser-Lys-Tyr-Leu-Asp-Glu-Cys-Arg-Ala-Gln-Asp-Phe-Val-Gln-Trp-Leu-X27- Asn-Thr (Compound 64)
wherein
the Cys at position 17 is PEGylated;
X27 is Met, Leu or Nle.

X may have the Formula VII.24: 
His-Ser-Gln-Gly-Thr-Phe-Thr-Ser-Asp-Tyr-Ser-Lys-Tyr-Leu- Asp-Glu-Arg-Arg-Ala-Gln-Cys-Phe-
Val-Gln-Trp-Leu-X27- Asn-Thr (Compound 65)
 wherein
the Cys at position 21 is PEGylated;
X27 is Met, Leu or Nle.

X may have the Formula VII.25: 
His-Ser-Gln-Gly-Thr-Phe-Thr-Ser-Asp-Tyr-Ser-Lys-Tyr-Leu- Asp-Glu-Arg-Arg-Ala-Gln-Asp-Phe-
Val-Cys-Trp-Leu-X27- Asn-Thr (Compound 66)
 wherein
the Cys at position 24 is PEGylated;
X27 is Met, Leu or Nle.

X may have the Formula VII.30: 
His-Ser-Gln-Gly-Thr-Phe-Thr-Ser-Asp-Tyr-Ser-Lys-Tyr-Leu- Asp-Glu-Arg-Arg-Ala-Gln-Asp-Phe-
Val-Gln-Trp-Leu-X27- Asn-Thr-Gly-Pro-Ser-Ser-Gly-Ala-Pro-Pro-Pro-Ser (Compound 67)
 wherein
X27 is Met, Leu or Nle.

X may have the Formula VII.31: 
His-Ser-Gln-Gly-Thr-Phe-Thr-Ser-Asp-Tyr-Ser-Lys-Tyr-Leu- Asp-Glu-Arg-Arg-Ala-Gln-Asp-Phe-
Val-Gln-Trp-Leu-X27- Asn-Thr-Lys-Arg-Asn-Arg-Asn-Asn-Ile-Ala (Compound 68)
 wherein
X27 is Met, Leu or Nle.

X may have the Formula VII.32: 
His-Ser-Gln-Gly-Thr-Phe-Thr-Ser-Asp-Tyr-Ser-Lys-Tyr-Leu- Asp-Glu-Arg-Arg-Ala-Gln-Asp-Phe-
Val-Gln-Trp-Leu-X27- Asn-Thr-Lys-Arg-Asn-Arg (Compound 69)
 wherein
X27 is Met, Leu or Nle.

X may have the Formula VII.33: 
His-Ser-Gln-Gly-Thr-Phe-Thr-Ser-Asp-Tyr-Ser-Lys-Tyr-Leu- X15- X16- Arg-Arg-Ala-X20-Asp-
Phe-Val-X24-Trp-Leu-Met-X28-X29 (Compound 70)
 wherein
X15 is Asp, Glu, homoglutamic acid, cysteic acid or homocysteic acid;
X16 is Ser, Glu, Gln, homoglutamic acid or homocysteic acid;
X20 is Gln or Lys;
X24 is Gln or Glu;
X28 is Asn, Lys or an acidic amino acid;
X29 is Thr, Gly or an acidic amino acid.

X may have the Formula VII.36:

His-Ser-Gln-Gly-Thr-Phe-Thr-Ser-Asp-Tyr-Ser-Lys-Tyr-Leu- Asp-Ser-Arg-Arg-Ala-Gln-Asp-Phe-
Val-Gln-Trp-Leu-Met-Asn-Thr-Gly-Pro-Ser-Ser-Gly-Ala-Pro-Pro-Pro-Ser. (Compound 71)

X may have the Formula VII..37: 
His-Ser-Gln-Gly-Thr-Phe-Thr-Ser-Asp-Tyr-Ser-Lys-Tyr-Leu- Asp-Ser-Arg-Arg-Ala-Gln-Asp-Phe-
Val-Cys-Trp-Leu-Met-Asn-Thr (Compound 72)
 wherein 24 2-butyrolactone is bound through thiol group of Cys.

X may have the Formula VII.38: 
His-Ser-Gln-Gly-Thr-Phe-Thr-Ser-Asp-Tyr-Ser-Lys-Tyr-Leu- Asp-Ser-Arg-Arg-Ala-Gln-Asp-Phe-
Val-Cys-Trp-Leu-Met-Asn-Thr (Compound 73)
 wherein a 24 carboxymethyl group is bound through thiol group of Cys.

X may have the Formula VI.39:

His-Ser-Gln-Gly-Thr-Phe-Thr-Ser-Asp-Tyr-Ser-Arg-Tyr-Leu- Asp-Ser-Arg-Arg-Ala-Gln-Asp-Phe-
Val-Gln-Trp-Leu-Met-Asn-Thr-Gly-Pro-Ser-Ser-Gly-Ala-Pro-Pro-Pro-Ser. (Compound 74)

X may have the Formula VII.40: 
His-Ser-Gln-Gly-Thr-Phe-Thr-Ser-Asp-Tyr-Ser-Lys-Tyr-Leu- X15-Glu-Arg-Arg-Ala-Gln-Asp-Phe-
Val-Gln-Trp-Leu-Met-X28-Thr (Compound 75)
 wherein
X15 is Glu or Asp;
X28 is Glu or Asp.

X may have the Formula VII.41: 
His-Ser-Gln-Gly-Thr-Phe-Thr-Ser-Asp-Tyr-Ser-Lys-Tyr-Leu- X15-Glu-Arg-Arg-Ala-Asp-Phe-Val-
Gln-Trp-Leu-Met-X28- Thr (Compound 76)
 wherein
X15 is Glu or Asp;
X28 is Glu or Asp; and
a lactam ring is present between the side chains at positions 12 and 16.

X may have the Formula VII.42: 
His-Ser-Gln-Gly-Thr-Phe-Thr-Ser-Asp-Tyr-Ser-Lys-Tyr-Leu- X15-Glu-Arg-Arg-Ala-Lys-Asp-Phe-
Val-Gln-Trp-Leu-Met-X28-Thr (Compound 77)
 wherein
X15 is Glu or Asp;
X28 is Glu or Asp; and
a lactam ring is present between the side chains at positions 16 and 20.

X may have the Formula VII.43: 
His-Ser-Gln-Gly-Thr-Phe-Thr-Ser-Asp-Tyr-Ser-Lys-Tyr-Leu- X15-Ser-Arg-Arg-Ala-Lys-Asp-Phe-
Val-Glu-Trp-Leu-Met-X28-Thr (Compound 78)
 wherein
X15 is Glu or Asp;
X28 is Glu or Asp; and
a lactam ring is present between side chains at positions 20 and 24.

X may have the Formula VI.44: 
His-Ser-Gln-Gly-Thr-Phe-Thr-Ser-Asp-Tyr-Ser-Lys-Tyr-Leu- X15-Ser-Arg-Arg-Ala-Gln-Asp-Phe-
Val-Glu-Trp-Leu-Met-Lys-X29 (Compound 79)
 wherein
X15 is Glu or Asp;
X29 is Glu or Thr.

In the above Formulae Z1 and Z2 are typically absent. The C-terminus of the compound may be amidated (R² = NH₂).

X may have the Formula VII.45: 
His-Ser-Gln-Gly-Thr-Phe-Thr-Ser-Asp-Tyr-Ser-X12-Tyr-Leu-X15-X16-Arg-Arg-Ala-X20-Asp-Phe-
Val-X24-Trp-Leu-Met-X28-X29 (Compound 80)
 wherein
X12 is Lys or Glu;
X15 is Asp, Glu, homoglutamic acid, cysteic acid or homocysteic acid;
X16 is Ser, Gln, Glu, Lys, homoglutamic acid, cysteic acid or homocysteic acid;
X20 is Gln, Glu or Lys;
X24 is Gln, Lys or Glu;
X28 is Asn, Lys or an acidic amino acid;
X29 is Thr, Gly or an acidic amino acid.

X may have the Formula VII.46: 
His-Ser-Gln-Gly-Thr-Phe-Thr-Ser-Asp-Tyr-Ser-Lys-Tyr-Leu-Asp-X16-Arg-Arg-Ala-X20-Asp-Phe-
Val-X24-Trp-Leu-Met-Asn-Thr (Compound 81)
 wherein
X16 is Ser, Glu, Gln, homoglutamic acid or homocysteic acid;
X20 is Gln or Lys;
X24 is Gln or Glu.

X may have the Formula VII.47:

His-Ser-Gln-Gly-Thr-Phe-Thr-Ser-Asp-Tyr-Ser-Lys-Tyr-Leu-Asp-Glu-Arg-Arg-Ala-Lys-Asp-Phe-
Val-Gln-Trp-Leu-Met-Asn-Thr. (Compound 82)

X may have the Formula VII.48:

His-Ser-Gln-Gly-Thr-Phe-Thr-Ser-Asp-Tyr-Ser-Lys-Tyr-Leu-Asp-Ser-Arg-Arg-Ala-Lys-Asp-Phe-
Val-Glu-Trp-Leu-Met-Asn-Thr. (Compound 83)

X may have the Formula VII.49:

His-Ser-Gln-Gly-Thr-Phe-Thr-Ser-Asp-Tyr-Ser-Lys-Tyr-Leu-Asp-Ser-Arg-Arg-Ala-Gln-Asp-Phe-
Val-Glu-Trp-Leu-Met-Asn-Thr. (Compound 84)

X may have the Formula VII.50:

His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-
Ala-Trp-Leu-Val-Lys-Gly-Arg-Gly (Compound 85)

X may have the Formula VII.51: 
His-Ser-Gln-Gly-Thr-Phe-Thr-Ser-Asp-Tyr-Ser-Lys-Tyr-Leu-X15-X16-Arg-Arg-Ala-X20-X21-Phe-
Val-X24-Trp-Leu-Met-X28-X29 (Compound 86)
 wherein
X15 is Asp, Glu, homoglutamic acid, cysteic acid or homocysteic acid;
X16 is Ser, Glu, Gln, homoglutamic acid or homocysteic acid;
X20 is Gln, Lys, Arg, Orn or Citrulline;
X21 is Asp, Glu, homoglutamic acid or homocysteic acid;
X24 is Gln or Glu;
X28 is Asn, Lys or an acidic amino acid;
X29 is Thr, Gly or an acidic amino acid.

X may have the Formula VII.52:

His-Ala-Glu-Gly-Thr-Phe-Thr-Ser-Asp-Val-Ser-Ser-Tyr-Leu-Glu-Gly-Gln-Ala-Ala-Lys-Glu-Phe-Ile-
Ala-Trp-Leu-Val-Lys-Gly-Arg. (Compound 87)

X may have the Formula VII.53: 
His-Ser-X3-Gly-Thr-Phe-Thr-Ser-Asp-Tyr-Ser-Lys-Tyr-Leu-X15-X16-Arg-Arg-Ala-X20-Asp-Phe-
Val-X24-Trp-Leu-Met-X28-X29 (Compound 88)
 wherein
X3 is Glu, Orn or Nle;
X15 is Asp, Glu, homoglutamic acid, cysteic acid or homocysteic acid;
X16 is Ser, Glu, Gln, homoglutamic acid or homocysteic acid;
X20 is Gln or Lys;
X24 is Gln or Glu;
X28 is Asn, Lys or an acidic amino acid;
X29 is Thr or an acidic amino acid.

X may have the Formula VII.54: 
His-Ser-Gln-Gly-Thr-Phe-Thr-Ser-Asp-Tyr-Ser-Lys-Tyr-Leu-Asp-Glu-X17-X18-Ala-Lys-X21-Phe-
X23-X24-Trp-Leu-Met-Asn-Thr (Compound 89)
 wherein
X17 is Arg or Gln;
X18 is Arg or Ala;
X21 is Asp or Glu;
X23 is Val or Ile;
X24 is Gln or Ala.

X may have the Formula VII.56: 
X1-X2-X3-Gly-Thr-Phe-Thr-Ser-Asp-Tyr-Ser-Lys-Tyr-Leu-X15-X16-Arg-Arg-Ala-X20-X21-Phe-
X23-X24-Trp-Leu-X27-X28-X29 (Compound 90)
 wherein
X1 is His, D-His, (Des-amino)His, hydroxyl-His, Acetyl-His, homo-His, DMIA, N-methyl His, Alpha-methyl His, or imidazole acetic acid;
X2 is Ser, D-Ser, Ala, D-Ala, Val, Gly, N-methyl Ser, Aib or N-methyl Ala;
X3 is Gln, Glu, Orn or Nle
X15 is Asp,Glu, cysteic acid, homoglutamic acid homocysteic acid;
X16 is Ser, Glu, Gln, homoglutamic acid, or homocysteic acid;
X20 is Gln, Lys, Arg, Om or Citrulline;
X21 is Gln, Glu, Asp,Cys, Orn, homocysteine or acetyl phenyalanine;
X23 is Val or lie;
X24 is Ala,Gln,Glu, Cys, Orn, homocysteine or acetyl phenyalanine;
X27 is Met, Leu or Nle;
X28 is Asn, Lys or Asp;
X29 is Thr, Gly Lys, Cys, Orn, homocysteine or acetyl phenyalanine.

X may have the Formula VII.57: 
X1-X2-X3-Gly-Thr-Phe-Thr-Ser-Asp-Tyr-Ser-Lys-Tyr-Leu-X15-Glu-Arg-Arg-Ala-X20-X21-Phe-
X23-X24-Trp-Leu-X27-X28-X29 (Compound 91)
 wherein
X1 is His, D-His, (Des-amino)His, hydroxyl-His, Acetyl-His, homo-His, DMIA, N-methyl His, Alpha-methyl His, or imidazole acetic acid;
X2 is Ser, D-Ser, Ala, D-Ala, Val, Gly, N-methyl Ser, Aib or N-methyl Ala;
X3 is Gln, Glu, Om or Nle;
X15 is Asp, Glu, Cysteic acid, nomoglutamic acid or homocysteic acid;
X20 is Gln, Lys, Arg, Orn, or Citrulline;
X21 is Gln, Glu, Asp,Cys, Orn, homocysteine or acetyl phenyalanine;
X23 is Val or Ile;
X24 is Ala, Gln Glu, Cys, Orn, homocysteine or acetyl phenyalanine;
X27 is Met, Leu or Nle;
X28 is Asn, Lys or Asp;
X29 is Thr, Gly, Cys, Orn, homocysteine or acetyl phenyalanine;
and wherein a lactam bridge is present between side chains at positions 12 and 16.

X may have the Formula VII.58: 
X1-X2-X3-Gly-Thr-Phe-Thr-Ser-Asp-Tyr-Ser-Lys-Tyr-Leu-X15-Glu-Arg-Arg-Ala-Lys-X21-Phe-
X23-X24-Trp-Leu-X27-X28-X29 (Compound 92)
 wherein
X1 is His, D-His, (Des-amino)His, hydroxyl-His, Acetyl-His, homo-His, DMIA, N-methyl His, Alpha-methyl His, or imidazole acetic acid;
X2 is Ser, D-Ser, Ala, D-Ala, Val, Gly, N-methyl Ser, Aib or N-methyl Ala;
X3 is Gln, Glu, Orn or Nle;
X15 is Asp, Glu, Cysteic acid, homoglutamic acid or homocysteic acid;
X21 is Gln Glu, Asp, Cys, Orn, homocysteine or acetyl phenyalanine;
X23 is Val or Ile;
X24 is Ala, Gln Glu, Cys, Orn, homocysteine or acetyl phenyalanine,
X27 is Met, Leu or Nle;
X28 is Asn, Lys or Asp;
X29 is Thr, Gly, Cys, Orn, homocysteine or acetyl phenyalanine;
and wherein a lactam bridge is present between side chains at positions 16 and 20.

X may have the Formula VII.59: 
X1-X2-X3-Gly-Thr-Phe-Thr-Ser-Asp-Tyr-Ser-Lys-Tyr-Leu-X15-X16-Arg-Arg-Ala-Lys-X21-Phe-
X23-Glu-Trp-Leu-X27-X28-X29 (Compound 93)
 wherein
X1 is His, D-His, (Des-amino)His, hydroxyl-His, Acetyl-His, homo-His, DMIA, N-methyl His, Alpha-methyl His, or imidazole acetic acid;
X2 is Ser, D-Ser, Ala, D-Ala, Val, Gly, N-methyl Ser, Aib or N-methyl Ala;
X3 is Gln Glu, Orn or Nle;
X15 is Asp, Glu, Cysteic acid, homoglutamic acid or homocysteic acid;
X16 is Ser, Glu, Gln homoglutamic acid or homocysteic acid;
X21 is Gln Glu, Asp, Cys, Orn, homocysteine or acetyl phenyalanine;
X23 is Val or Ile;
X27 is Met, Leu or Nle;
X28 is Asn, Lys or Asp;
X29 is Thr, Gly, Cys, Orn, homocysteine or acetyl phenyalanine;
and wherein a lactam bridge is present between side chains at positions 20 and 24.

X may have the Formula VII.60: 
X1-X2-X3-Gly-Thr-Phe-Thr-Ser-Asp-Tyr-Ser-Lys-Tyr-Leu-X15-X16-Arg-Arg-Ala-X20-X21-Phe-
X23-Glu-Trp-Leu-X27-Lys-X29 (Compound 94)
 wherein
X1 is His, D-His, (Des-amino)His, hydroxyl-His, Acetyl-His, homo-His, DMIA, N-methyl His, Alpha-methyl His, or imidazole acetic acid;
X2 is Ser, D-Ser, Ala, D-Ala, Val, Gly, N-methyl Ser, Aib or N-methyl Ala;
X3 is Gln Glu, Orn or Nle;
X15 is Asp, Glu, Cysteic acid, homoglutamic acid or homocysteic acid;
X16 is Ser, Glu, Gin, homoglutamic acid or homocysteic acid;
X20 is Gln Lys. Arg, Orn or Citrulline
X21 is Gln Glu, Asp, Cys, Orn, homocysteine or acetyl phenyalanine;
X23 is Val or Ile;
X27 is Met, Leu or Nle;
X29 is Thr, Gly, Cys, Orn, homocysteine or acetyl phenyalanine;
and wherein a lactam bridge is present between side chains at positions 24 and 28

X-Z¹ may have the Formula VII.61: 
X1-X2-Glu-Gly-Thr-Phe-Thr-Ser-Asp-Tyr-Ser-Lys-Tyr-Leu-Asp-Glu-Arg-X18-Ala-Lys-Asp-Phe-
Val-X24-Trp-Leu-Met-Asn-X29-Gly-Pro-Ser-Ser-Gly-Ala-Pro-Pro-Pro-Ser-Cys (Compound 95)
 wherein
X1 is His, D-His, (Des-amino)His, hydroxyl-His, acetyl-His, homo-His, DMIA, N-methyl His, alpha-methyl His, or imidazole acetic acid;
X2 is Ser, D-Ser, Ala, Val, Gly, N-methyl Ser, Aib, N-methyl Ala or D-Ala;
X18 is Ata or Arg;
X24 is Ala, Gln or Cys-PEG;
X29 is Thr-CONH2, Cys-PEG, or Gly;
position 40 is Cys-PEG or not present;
provided that positions 30 to 40 (Z²) are present only if position 29 is Gly.

X-Z¹ may have the Formula VII.62: 
X1-X2-Glu-Gly-Thr-Phe-Thr-Ser-Asp-Tyr-Ser-Lys-Tyr-Leu-Asp-Glu-Gln-X18-Ala-Lys-Glu-Phe-Ile-
X24-Trp-Leu-Met- Asn-X29-Gly-Pro-Ser-Ser-Gly-Ala-Pro-Pro-Pro-Ser-Cys (Compound 96)
 wherein
X1 is His, D-His, (Des-amino)His, hydroxyl-His, acetyl-His, homo-His, DMIA, N-methyl His, alpha-methyl His, or imidazole acetic acid;
X2 is Ser, D-Ser, Ala, Val, Gly, N-methyl Ser, Aib, N-methyl Ala or D-Ala;
X18 is Ala or Arg;
X24 is Ala, Gln or Cys-PEG;
X29 is Thr-CONH2, Cys-PEG, or Gly;
position 40 is Cys-PEG or not present;
provided that positions 30 to 40 (Z²) are present only if position 29 is Gly

X may have the Formula VII.63: 
His-Ser-Gln-Gly-Thr-Phe-Thr-Ser-Asp-Tyr-Ser-Lys-Tyr-Leu-Asp-X16-Arg-Arg-Ala-X20-X21-Phe-
Val-X24-Trp-Leu-X27-Asp-Thr (Compound 97)
 wherein
X16 is Ser, Glu, Gln homoglutamic acid or homocysteic acid;
X20 is Gln or Lys;
X21 is Asp, Lys. Cys, Orn, homocysteine or acetyl phenyalanine;
X24 is Gln Lys. Cys, Orn, homocysteine or acetylphenyalanine;
X27 is Met, Leu or Nle.

X-Z¹ may have the Formula VII.64: 
His-Ser-Gln-Gly-Thr-Phe-Thr-Ser-Asp-Tyr-Ser-Lys-Tyr-Leu- X15-X16-Arg-Arg-Ala-X20-Asp-Phe-
Val-X24-Trp-Leu-Met-X28-Gly-Gly-Pro-Ser-Ser-Gly-Pro-Pro-Pro-Ser (Compound 98)
 wherein
X15 is Asp, Glu, homoglutamic acid, cysteic acid or homocysteic acid;
X16 is Ser, Glu, Gln homoglutamic acid or homocysteic acid;
X20 is Gln or Lys;
X24) is Gln or Glu;
X28 is Asn, Lys or Asp.

X may have the Formula VII.66: 
His-Ser-Gln-Gly-Thr-Phe-Thr-Ser-Asp-Tyr-Ser-Lys-Tyr-Leu- Asp-Glu-Arg-Arg-Ala-Gln-Asp-Phe-
Val-Gln-Trp-Leu-Met-X28-X29 (Compound 99)
 wherein
X28 is Asp or Asn;
X29 is Thr or Gly;
and wherein a lactam ring is present between side chains at positions 12 and 16.

X may have the Formula VII.67: 
His-Ser-Gln-Gly-Thr-Phe-Thr-Ser-Asp-Tyr-Ser-Lys-Tyr-Leu- Asp-Glu-Arg-Arg-Ala-Lys-Asp-Phe-
Val-Gln-Trp-Leu-Met-X28-X29 (Compound 100)
 wherein
X28 is Asp or Asn;
X29 is Thr or Gly;
and wherein a lactam ring is present between side chains at positions 16 and 20.

X may have the Formula VII.68: 
His-Ser-Gln-Gly-Thr-Phe-Thr-Ser-Asp-Tyr-Ser-Lys-Tyr-Leu- Asp-Ser-Arg-Arg-Ala-Lys-Asp-Phe-
Val-Glu-Trp-Leu-Met-X28-X29 (Compound 101)
 wherein
X28 is Asp or Asn;
X29 is Thr or Gly;
and wherein a lactam ring is present between side chains at positions 20 and 24.

X may have the Formula VII.69: 
His-Ser-Gln-Gly-Thr-Phe-Thr-Ser-Asp-Tyr-Ser-Lys-Tyr-Leu- Asp-Ser-Arg-Arg-Ala-Gln-Asp-Phe-
Val-Glu-Trp-Leu-Met-Lys-X29 (Compound 102)
 wherein
X29 is Thr or Gly;
and wherein a lactam ring is present between side chains at positions 24 and 28.

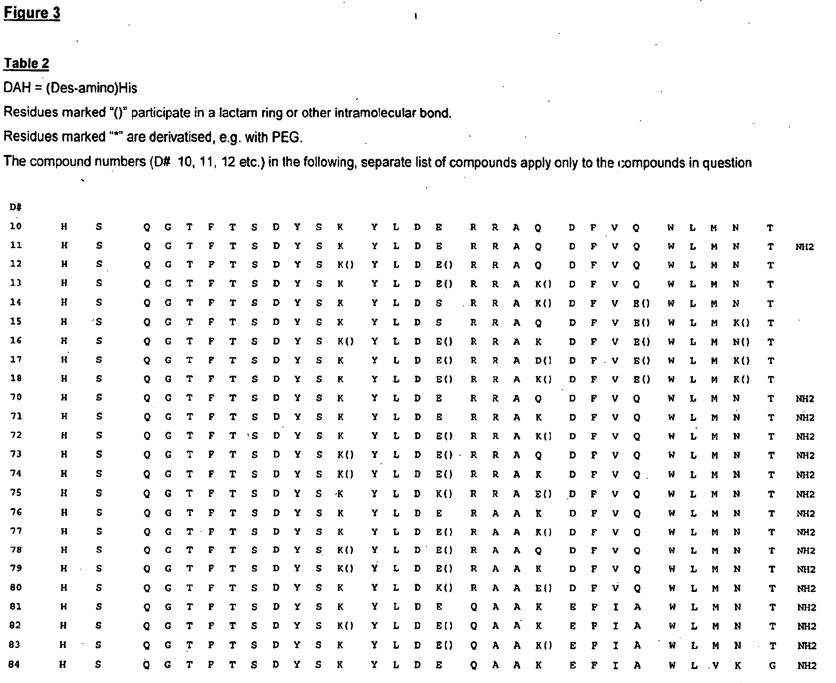

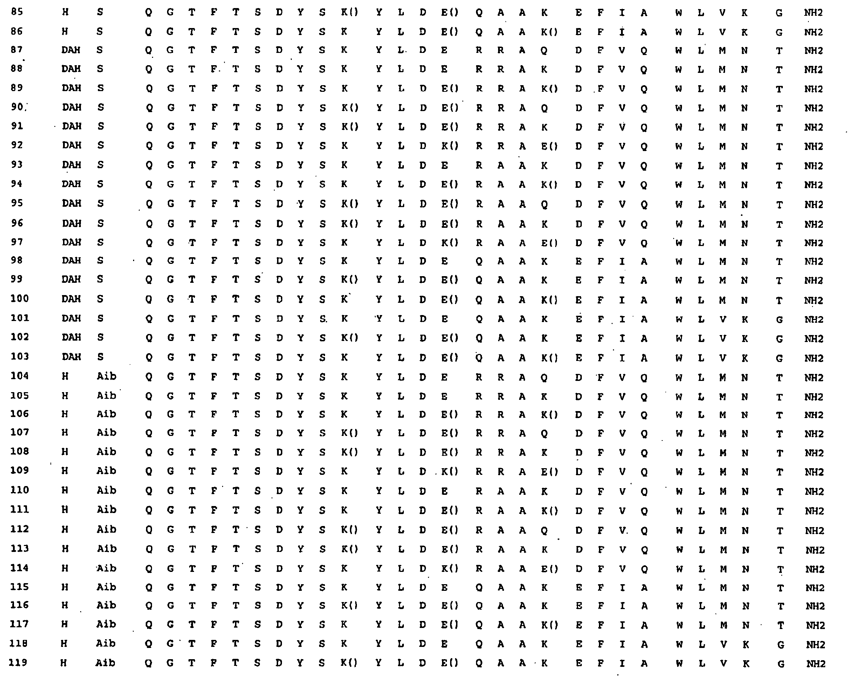

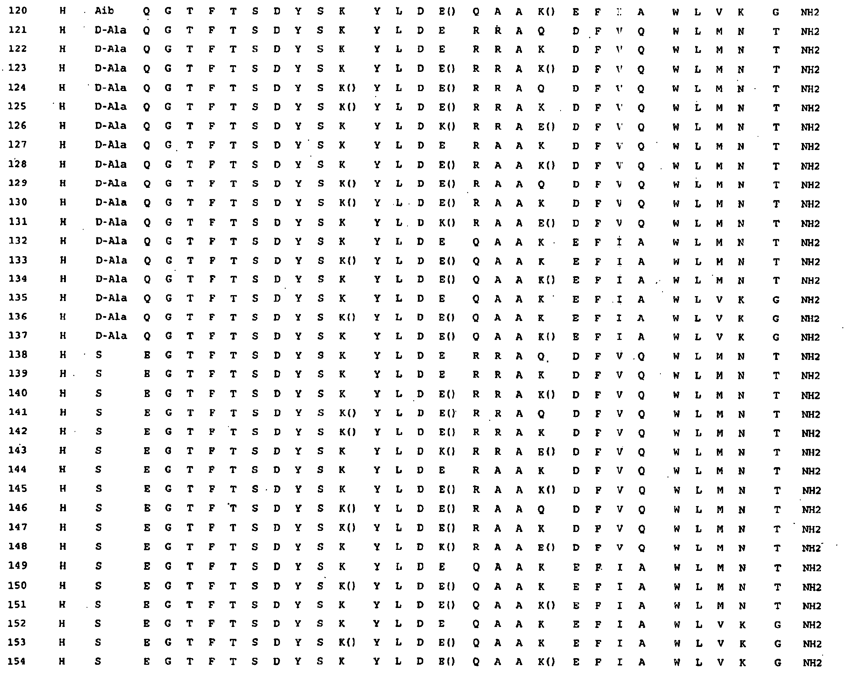

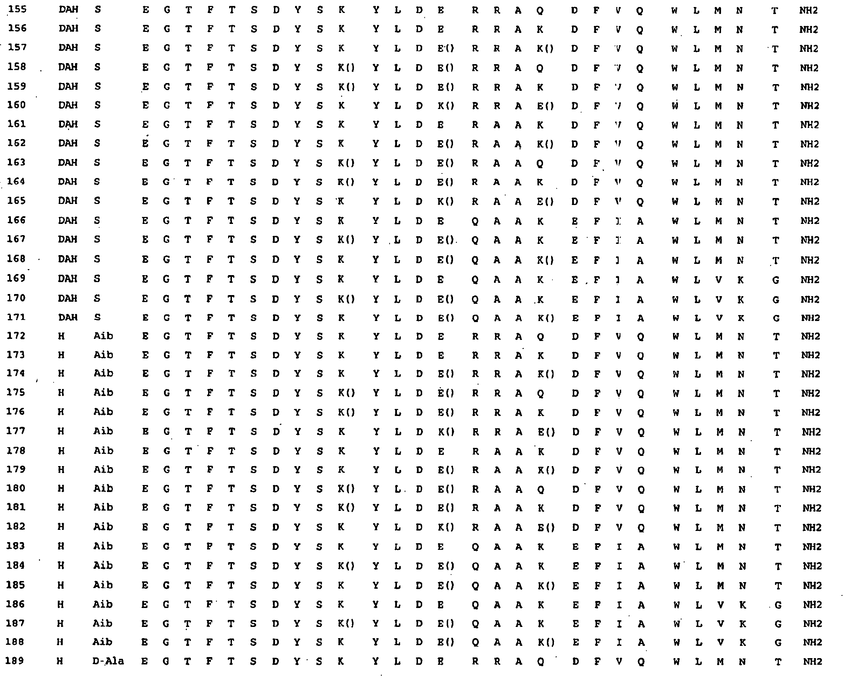

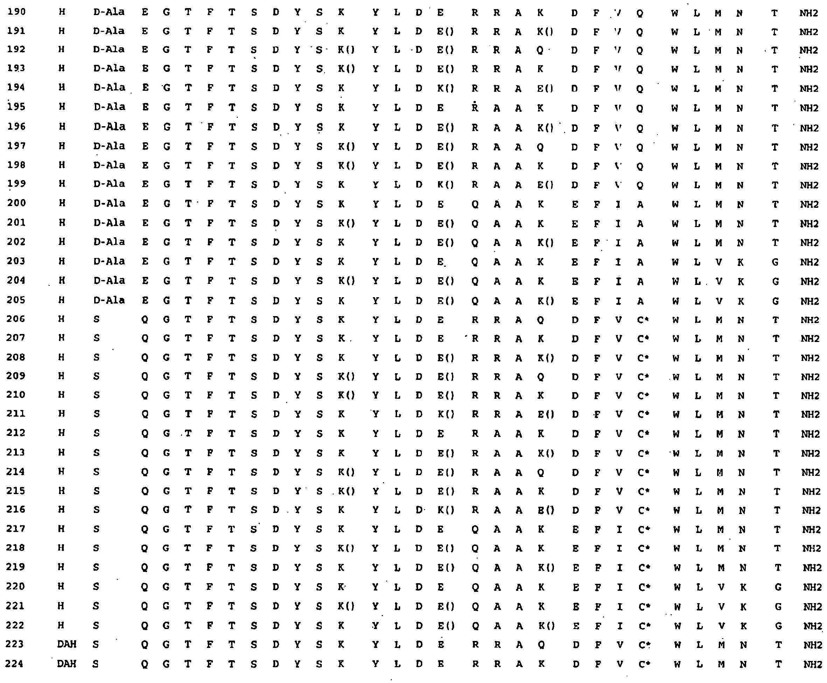

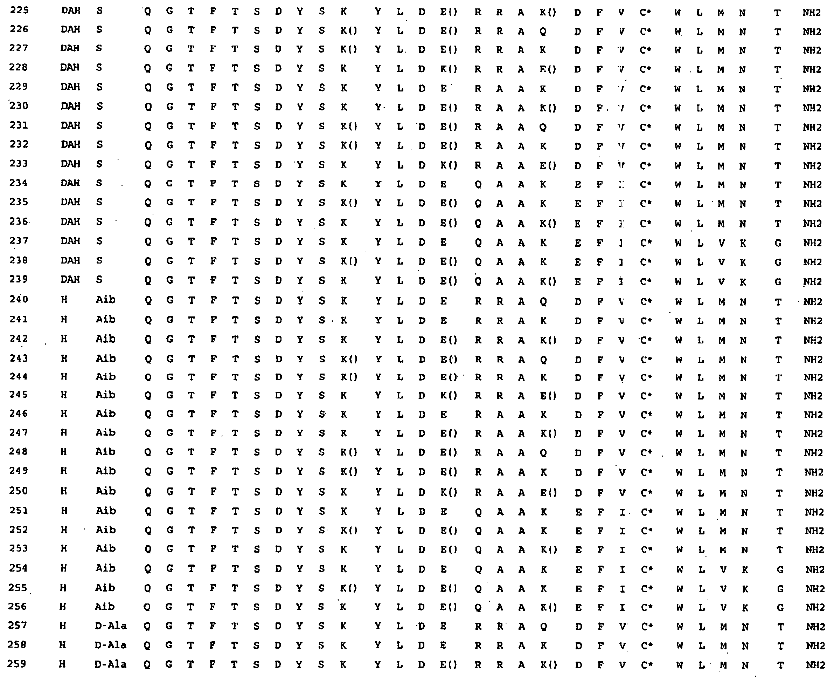

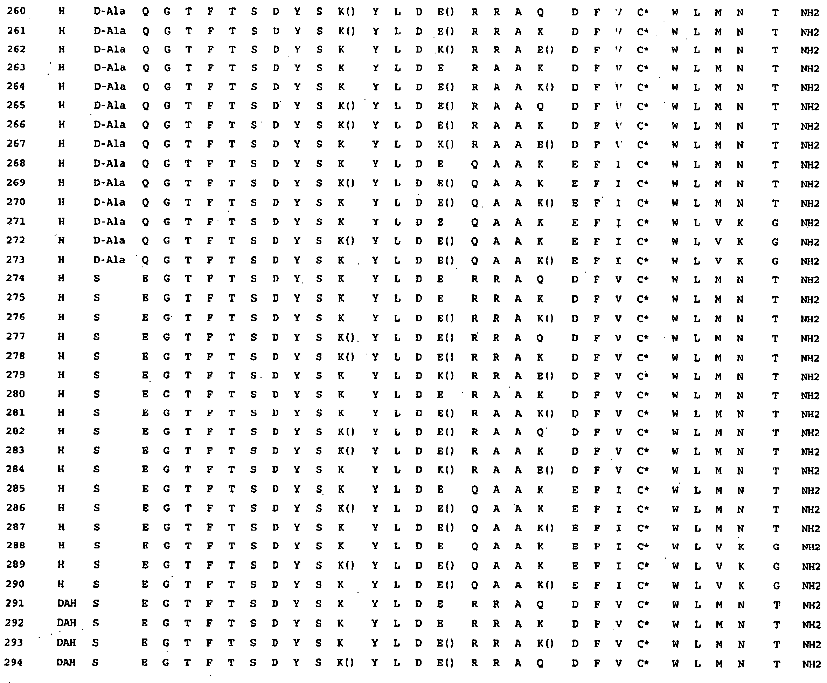

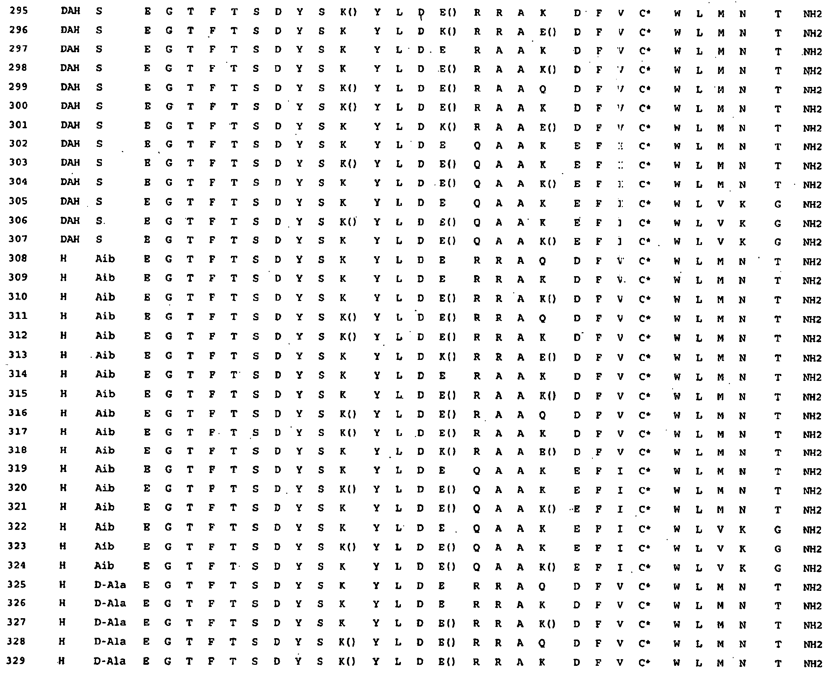

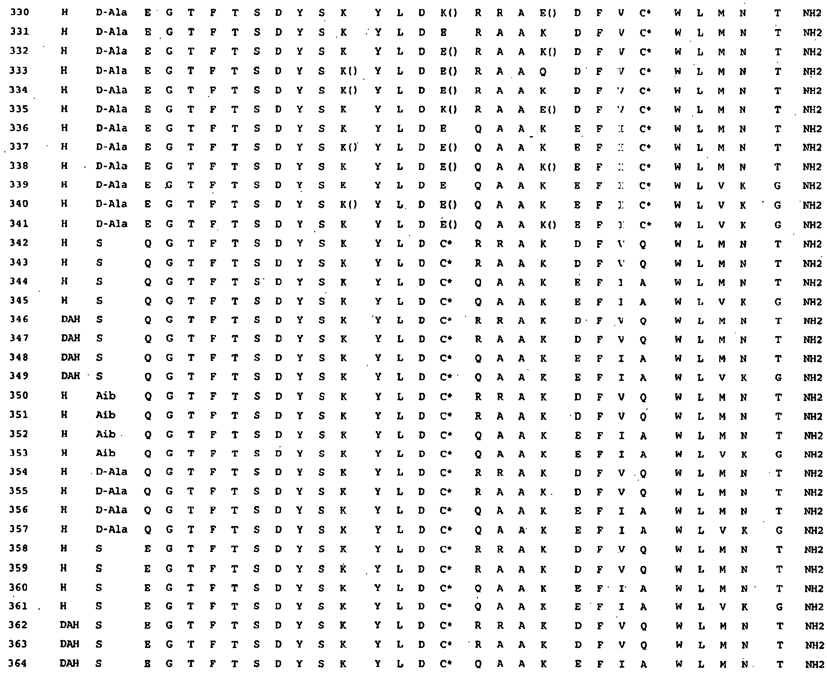

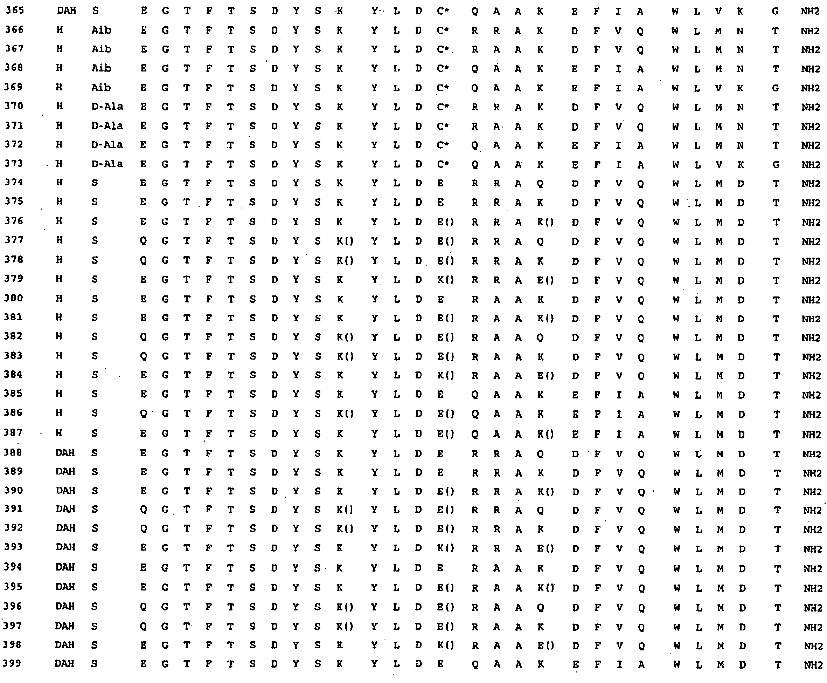

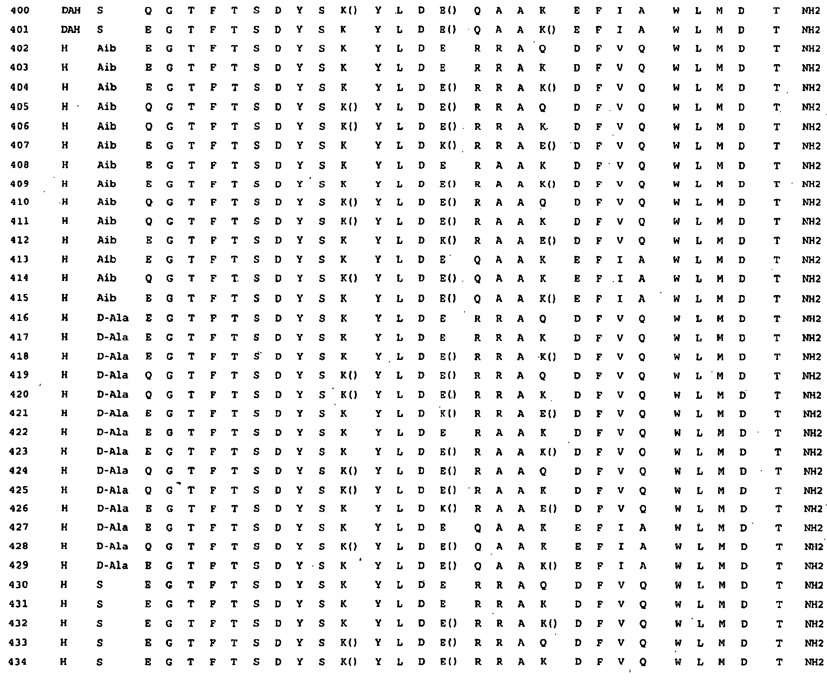

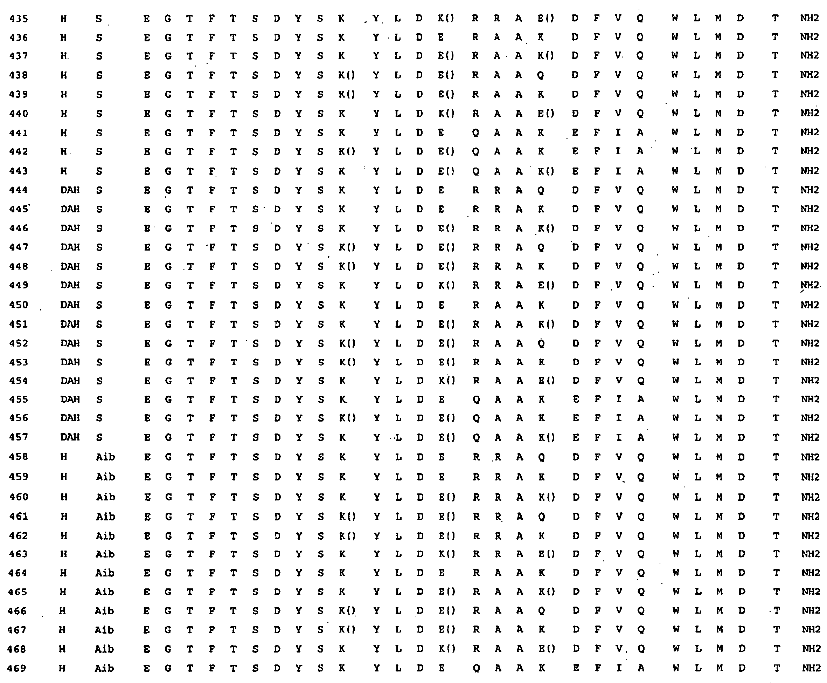

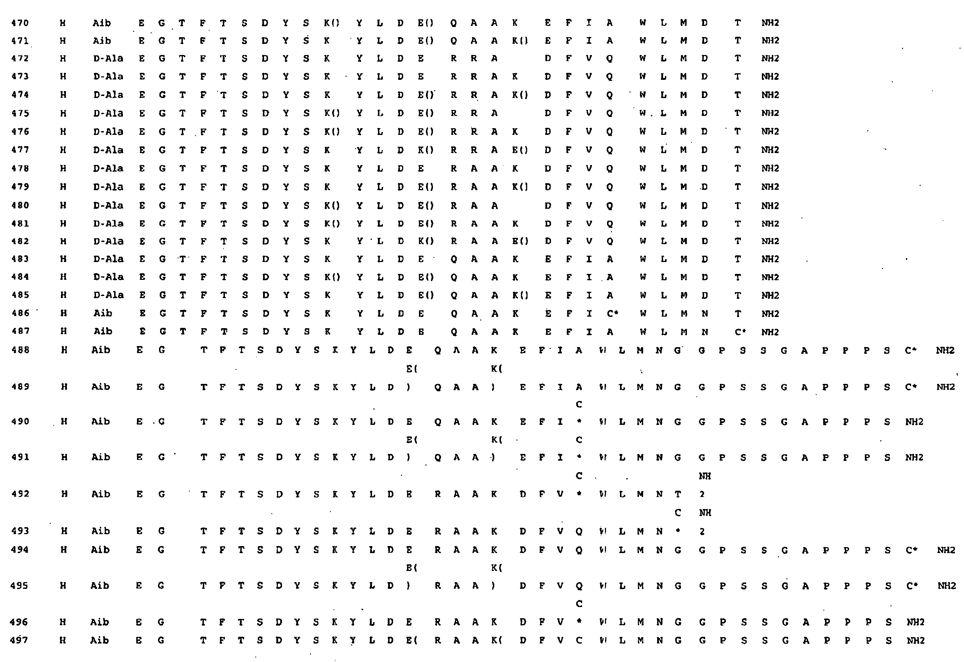

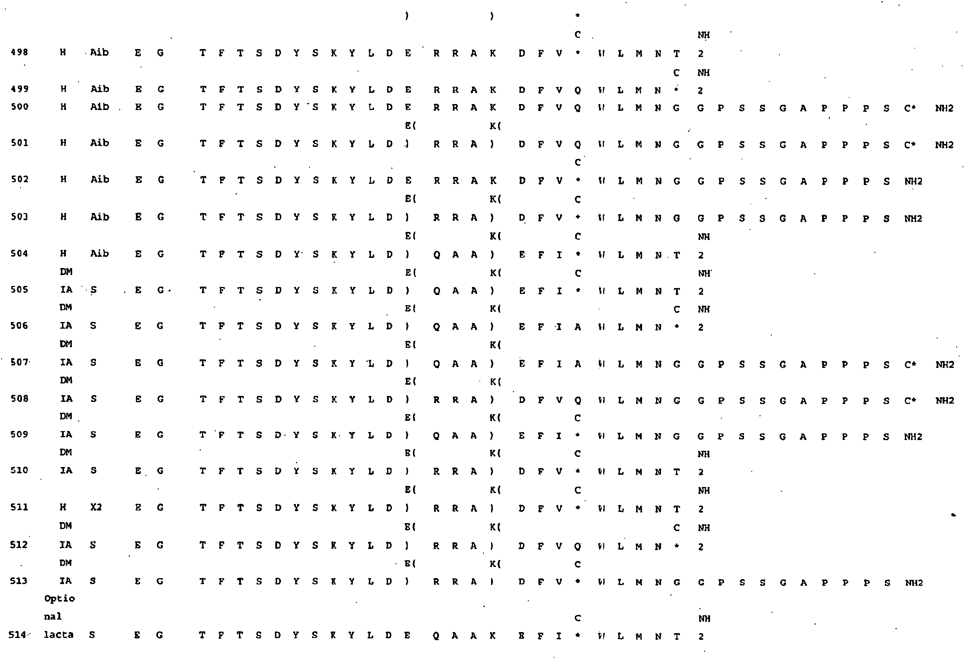

Further specific compounds which may be useful are shown in Figure 3: Table 2 and Table 3

### DESCRIPTION OF THE FIGURES

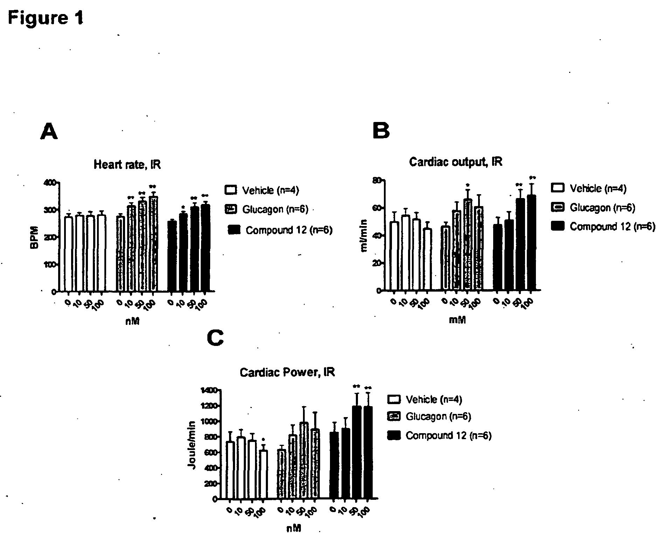

**Figure 1****:** Effects of vehicle, glucagon, and a glucagon-GLP1 dual-agonist (Compound 12) on A: Heart rate in insulin-resistant (IR) JCR: LA rat hearts; B: Cardiac output in IR hearts; C: Cardiac power in IR hearts. Values are presented as mean + SEM. * P < 0.05; ** P < 0.01 compared to baseline.

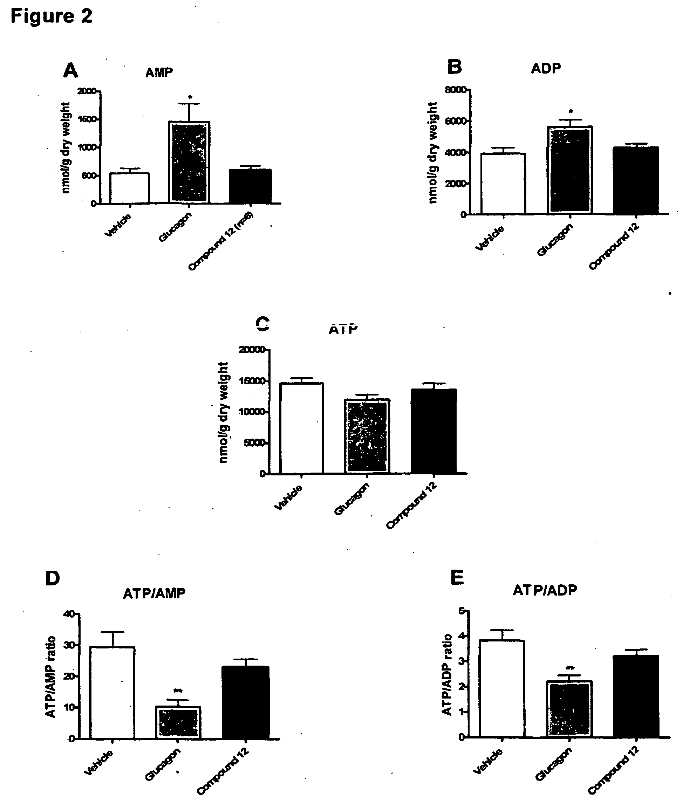

**Figure 2****:** Energy state in hearts from insulin-resistant (IR) JCR: LA rats after perfusion with increasing concentrations of vehicle (n=4), glucagon (n=6), and a glucagon-GLP1 dual-agonist (Compound 12) (n=5). A: Adenosine monophosphate (AMP) concentrations. B: Adenosine diphosphate (ADP) concentrations. C: Adenosine triphosphate (ATP) concentrations. D: ATP/AMP ratios. E: ATP/ADP ratios. Values are presented as mean + SEM. *P < 0.05; ** P < 0.01 compared to vehicle.
**Figure 3****:** Shows a table (Table 2) of compounds by sequence which may be useful in accordance with the invention.

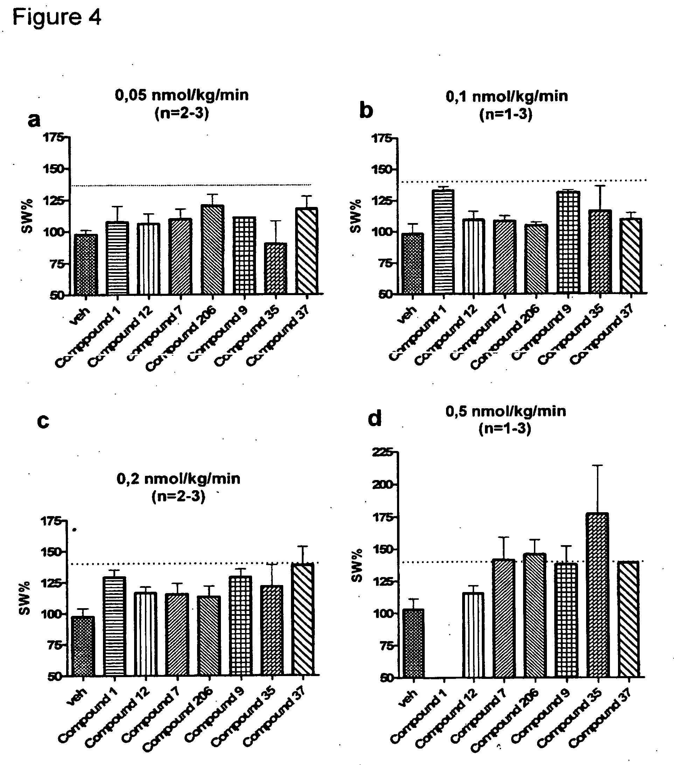

**Figure 4****:** Strokework calculated from individual data for each compound infused with compound 1 our glucagon-GLP-1 dual agonists. Dose is given in nmol/kg/min and indicated on top of each figure. A maximum of 40% increase in strokework was set as end point, after which infusion was discontinued.

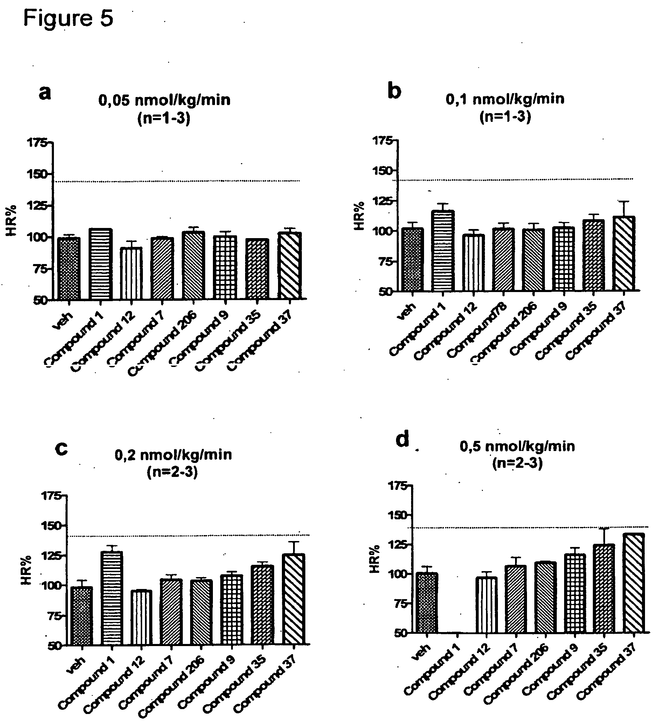

**Figure 5****:** Heart rate calculated from individual data for each compound infused with compound 1 or glucagon-GLP-1 dual agonists. Dose is given in nmol/kg/min and indicated on top of each figure. A maximum of 40% increase in strokework (figure 4) was set as end point, after which infusion was discontinued.

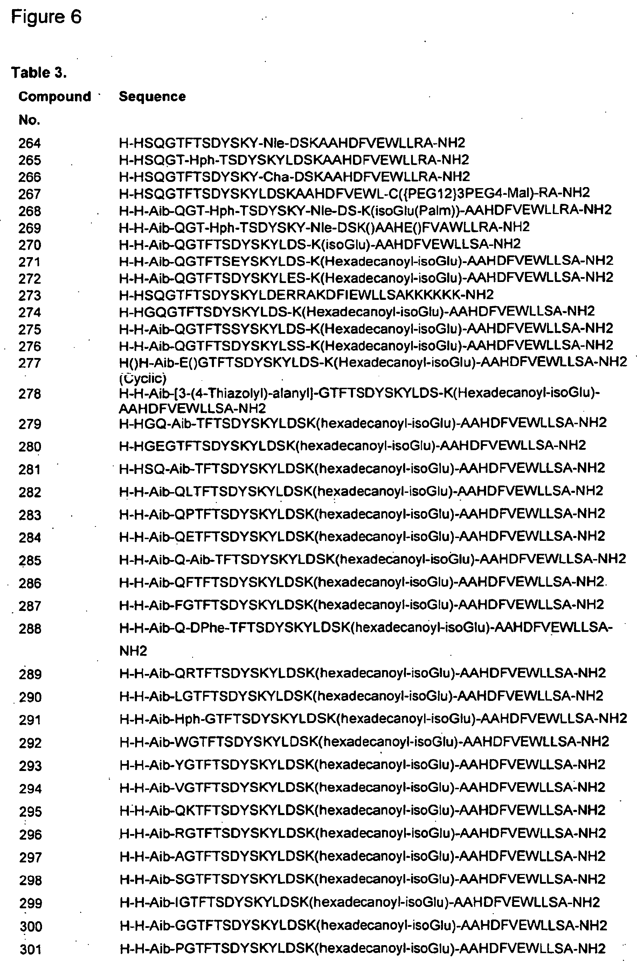

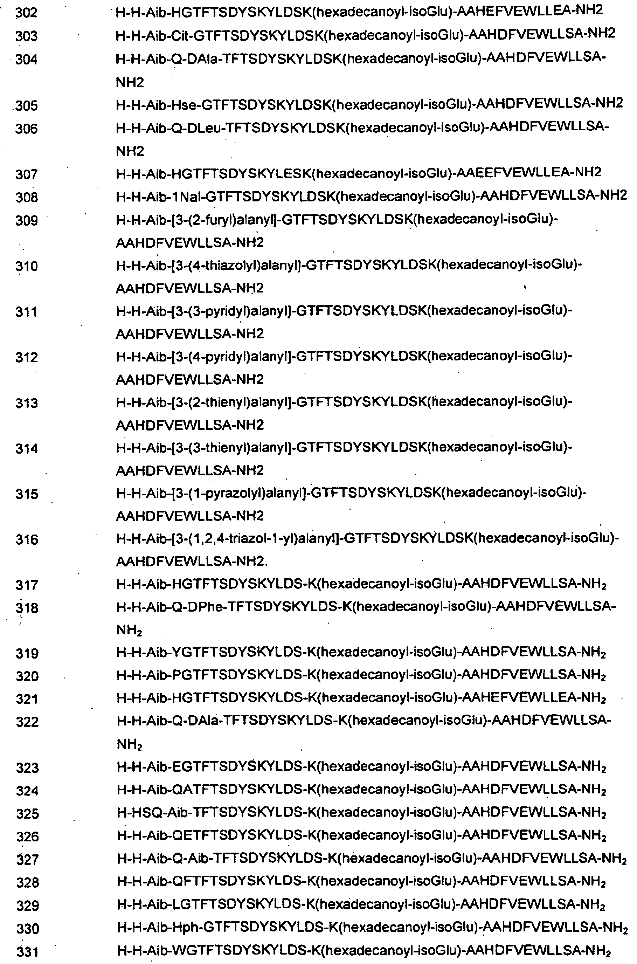

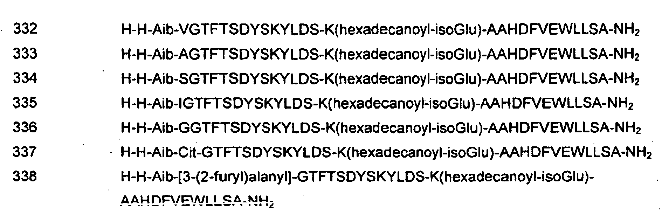

**Figure 6****:** Shows a table (Table 3) of compounds by sequence which may be useful in accordance with the invention.

### DETAILED DESCRIPTION OF THE INVENTION

Throughout this specification, the conventional one letter and three letter codes for naturally occurring amino acids are used, as well as generally accepted three letter codes for other amino acids, such as Aib (α-aminoisobutyric acid), Orn (ornithine), Dbu (2,4-diaminobutyric acid) and Dpr (2,3-diaminopropanoic acid), Cit (citrulline), 1Nal (1-naphthylalanine), Hph (homophenylalanine), Hse (homoserine) and Orn (ornithine).

In the context of the present disclosure C₁₋₆alkyl and C₁₋₄ alkyl include methyl, ethyl, 1-propyl and 2-propyl.
In the context of the present disclosure the expression "positive inotropic" refers to agents that increase the force and velocity of myocardial contractility, i.e. improves myocardial contractility.

The term "native glucagon" refers to native human glucagon having the sequence H-His-Ser-Gln-Gly-Thr-Phe-Thr-Ser-Asp-Tyr-Ser-Lys-Tyr-Leu-Asp-Ser-Arg-Arg-Ala-Gln-Asp-Phe-Val-Gln-Trp-Leu-Met-Asn-Thr-OH.

The terms "oxyntomodulin" and "OXM" refer to native human oxyntomodulin having the sequence H-His-Ser-Gln-Gly-Thr-Phe-Thr-Ser-Asp-Tyr-Ser-Lys-Tyr-Leu-Asp-Ser-Arg-Arg-Ala-Gln-Asp-Phe-Val-Gln-Trp-Leu-Met-Asn-Thr-Lys-Arg-Asn-Arg-Asn-Asn-Ile-Ala-OH.

In certain embodiments of compounds of the invention wherein the amino acid residue X3 is 3-(heterocyclyl)alanyl [i.e. an amino acid residue deriving from a 3-(heterocyclyl)-substituted alanine], then X3 may be selected from the group consisting of 3-(2-furyl)alanyl, 3-(4-thiazolyl)alanyl, 3-(3-pyridyl)alanyl, 3-(4-pyridyl)alanyl, 3-(1-pyrazolyl)alanyl, 3-(2-thienyl)alanyl, 3-(3-thienyl)alanyl and 3-(1,2,4-triazol-1-yl)alanyl.

### Peptide sequence X

For the avoidance of doubt, in the definitions above, it is generally intended that the sequence of X only differs from the formulae shown at those positions which are stated to allow variation. Amino acids within the sequences X described herein can be considered to be numbered consecutively from 1 to 29 in the conventional N-terminal to C-terminal direction. Reference to a "position" within X should be construed accordingly, as should reference to positions within native human glucagon and other molecules.

In any of the formulae provided herein, the residue at position X3 may alternatively be selected from acetamidomethyl-cysteine, acetyldiaminobutanoic acid, carbamoyldiaminopropanoic acid, methylglutamine and methionine sulfoxide.

Certain formulae presented above allow the residues at positions X27, X28 and/or X29 to be absent. Typically, if X28 is absent, then X29 is also absent. If X27 is absent, then X28 and X28 are both also absent. In other words, X28 will not be absent if X29 is present, and X27 will not be absent if either of X28 and X29 is present.

When Z¹ is absent, the peptide sequence X can be considered an analogue of glucagon. In such embodiments, the peptide sequence X differs from the sequence of native human glucagon at one or more of the 29 positions, for example at a minimum of 2 of 29 positions, e.g. at a minimum of 3, 4, 5, 6 of 29 positions.

In certain embodiments, when X differs from human glucagon at only one position, that position may be X12, X17 or X18.

The residue at X12 may be Ala or Arg.

The residue at X17 may be Glu or Lys.

The residue at X18 may be His, Ser, Ala or Tyr.

Thus the peptide X may have the sequence:
HSQGTFTSDYSAYLDSRRAQDFVQWLMNT; (Compound 103).
HSQGTFTSDYSRYLDSRRAQDFVQWLMNT; (Compound 104)
HSQGTFTSDYSKYLDSERAQDFVQWLMNT; (Compound 106)
HSQGTFTSDYSKYLDSKRAQDFVQWLMNT: (Compound 107)
HSQGTFTSDYSKYLDSRHAQDFVQWLMNT; (Compound 108)
HSQGTFTSDYSKYLDSRSAQDFVQWLMNT; (Compound 109)
HSQGTFTSDYSKYLDSRAAQDFVQWLMNT, or (Compound 110)
HSQGTFTSDYSKYLDSRYAQDFVQWLMNT. (Compound 111)

Sequences having 2 or 3 differences from human glucagon include:
HSQGTFTSDYSRYLDSRRAKDFVQWLLNT; (Compound 112)
HSQGTFTSDYSRYLDSRRAQDFVQWLLNT; (Compound 113)
HSQGTFTSDYSRYLDSRRAQDFVQWLLNK; (Compound 114)
HSQGTFTSDYSKYLDSALAQDFVQWLLNT; (Compound 115)
HSQGTFTSDYSKYLDKRRAEDFVQWLMNT; (Compound 116)
HSQGTFTSDYSKYLDK()RRAE()DFVQWLMNT: (Compound 117)
HSQGTFTSDYSRYLDERRAQDFVQWLMNT; (Compound 118)
HSQGTFTSDYSK()YLDE()RRAQDFVQWLMNT; (Compound 119)
HSQGTFTSDYSKYLDSRRAQDFIEWLMNT; and (Compound 120)
HSQGTFTSDYSKYLDSKAAQDFVQWLMNT; (Compound 121)
HSQGTFTSDYSKYLDSLAAQDFVQWLLNT. (Compound 122)

Whether Z¹ is present or absent, it may be desirable that the peptide sequence X differs from human glucagon at a maximum of 10 of 29 positions, e.g. at a maximum of 7, 8, 9 or 10 positions.

### Z¹

Z¹ may have the sequence:
GlyProSerSerGlyAlaProProProSer, representing the C-terminal 10 amino acids of native Exendin-4;
GlyProSerSerGlyAlaProProProSerCys, representing the C-terminal 10 amino acids of native Exendin-4 plus an additional C-terminal Cys residue;
LysArgAsnArgAsnAsnIleAla, representing the C-terminal 8 amino acids of native oxyntomodulin; or
LysArgAsnArg.

### Z²

The compound may comprise a C-terminal peptide sequence Z² of 1-20 amino acids, for example to stabilise the conformation and/or secondary structure of the glucagon analogue peptide, and/or to make the glucagon analogue peptide more resistant to enzymatic hydrolysis, e.g. as described in WO99/46283.

When present, Z² represents a peptide sequence of 1-20 amino acid residues, e.g. in the range of 1-15, more preferably in the range of 1-10 in particular in the range of 1-7 amino acid residues, e.g., 1, 2, 3, 4, 5, 6 or 7 amino acid residues, such as 6 amino acid residues. Each of the amino acid residues in the peptide sequence Z² may independently be selected from Ala, Leu, Ser, Thr, Tyr, Cys, Glu, Lys, Arg, Dbu (2,4 diaminobutyric acid), Dpr (2,3-diaminopropanoic acid) and Orn (ornithine). Preferably, the amino acid residues are selected from Ser, Thr, Tyr, Cys, Glu, Lys, Arg, Dbu, Dpr and Orn, more preferably may be selected exclusively from Glu, Lys, and Cys, especially Lys. The above-mentioned amino acids may have either D- or L-configuration, but preferably have an L-configuration. Particularly preferred sequences for Z² are sequences of four, five, six or seven consecutive lysine residues (i.e. Lys₃, Lys₄, Lys₅, Lys₆ or Lys₇), and particularly five or six consecutive lysine residues. Other exemplary sequences of Z are shown in WO 01/04156, the content of which is incorporated herein by reference. Alternatively the C-terminal residue of the sequence Z² may be a Cys residue. This may assist in modification of the compound, e.g. conjugation to a lipophilic substituent or polymeric moiety as described below. In such embodiments, the sequence Z² may, for example, be only one amino acid in length (i.e. Z² = Cys) or may be two, three, four, five, six or even more amino acids in length. The other amino acids therefore serve as a spacer between the peptide X and the terminal Cys residue. In such embodiments, Z¹ may be absent.

In some embodiments, the peptide sequence Z² has no more than 25% amino acid sequence identity with the corresponding sequence of the IP-1 portion of human OXM (which has the sequence Lys-Arg-Asn-Arg-Asn-Asn-Ile-Ala).

Percent (%) amino acid sequence identity" of a given peptide or polypeptide sequence with respect to another polypeptide sequence (e.g. IP-1) is calculated as the percentage of amino acid residues in the given peptide sequence that are identical with corresponding amino acid residues in the corresponding sequence of that other polypeptide when the two are aligned with one another, introducing gaps for optimal alignment if necessary. % identity values may be determined by WU-BLAST-2 (Altschul et al., Methods in Enzymology, 266:460-480 (1996)). WU-BLAST-2 uses several search parameters, most of which are set to the default values. The adjustable parameters are set with the following values: overlap span = 1, overlap fraction = 0.125, word threshold (T) = 11. A % amino acid sequence identity value is determined by the number of matching identical residues as determined by WU-BLAST-2, divided by the total number of residues of the reference sequence (gaps introduced by WU-BLAST-2 into the reference sequence to maximize the alignment score being ignored), multiplied by 100.

Thus, when Z² is aligned optimally with the 8 amino acids of IP-1, it has no more than two amino acids which are identical with the corresponding amino acids of IP-1.

### Amino acid modification

One or more of the amino acid side chains in any of the compounds suitable for use in the present invention may be conjugated to a lipophilic substituent. The lipophilic substituent may be covalently bonded to an atom in the amino acid side chain, or alternatively may be conjugated to the amino acid side chain by a spacer. The amino acid may be part of the peptide X, or part of the peptides Z¹ or Z². The spacer, when present, is used to provide a spacing between the rest of the compound and the lipophilic substituent.

Without wishing to be bound by theory, it is thought that the lipophilic substituent binds albumin in the blood stream, thus shielding the compounds of the invention from enzymatic degradation which can enhance the half-life of the compounds. Thus compound modified in this way may be particularly suitable for chronic treatment.

The lipophilic substituent may be attached to the amino acid side chain or to the spacer via an ester, a sulphonyl ester, a thioester, an amide or a sulphonamide. Accordingly it will be understood that preferably the lipophilic substituent includes an acyl group, a sulphonyl group, an N atom, an O atom or an S atom which forms part of the ester, sulphonyl ester, thioester, amide or sulphonamide. Preferably, an acyl group in the lipophilic substituent forms part of an amide or ester with the amino acid side chain or the spacer.

The lipophilic substituent may include a hydrocarbon chain having 4 to 30 C atoms. Preferably it has at least 8 or 12 C atoms, and preferably it has 24 C atoms or fewer, or 20 C atoms or fewer. The hydrocarbon chain may be linear or branched and may be saturated or unsaturated. It will be understood that the hydrocarbon chain is preferably substituted with a moiety which forms part of the attachment to the amino acid side chain or the spacer, for example an acyl group, a sulphonyl group, an N atom, an O atom or an S atom. Most preferably the hydrocarbon chain is substituted with acyl, and accordingly the hydrocarbon chain may be part of an alkanoyl group, for example palmitoyl, caproyl, lauroyl, myristoyl or stearoyl.

In certain embodiments, the lipophilic substituent may include a hydrocarbon chain having 10 to 24 C atoms, e.g. 10 to 22 C atoms, e.g. 10 to 20 C atoms. Preferably it has at least 11 C atoms, and preferably it has 18 C atoms or fewer. For example, the hydrocarbon chain may contain 12, 13, 14, 15, 16, 17 or 18 carbon atoms. The hydrocarbon chain may be linear or branched and may be saturated or unsaturated. From the discussion above it will be understood that the hydrocarbon chain is preferably substituted with a moiety which forms part of the attachment to the amino acid side chain or the spacer, for example an acyl group, a sulphonyl group, an N atom, an O atom or an S atom. Most preferably the hydrocarbon chain is substituted with acyl, and accordingly the hydrocarbon chain may be part of an alkanoyl group, for example a dodecanoyl, 2-butyloctanoyl, tetradecanoyl, hexadecanoyl, heptadecanoyl, octadecanoyl or eicosanoyl group.

Accordingly, the lipophilic substituent may have the formula shown below:

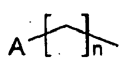

A may be, for example, an acyl group, a sulphonyl group, NH, N-alkyl , an O atom or an S atom, preferably acyl. n is an integer from 3 to 29, preferably at least 7 or at least 11, and preferably 23 or less, more preferably 19 or less.

The hydrocarbon chain may be further substituted. For example, it may be further substituted with up to three substituents selected from NH₂, OH and COOH. If the hydrocarbon chain is further substituted, preferably it is further substituted with only one substituent. Alternatively or additionally, the hydrocarbon chain may include a cycloalkane or heterocycloalkane, for example as shown below:

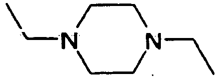

Preferably the cycloalkane or heterocycloalkane is a six-membered ring. Most preferably, it is piperidine.

Alternatively, the lipophilic substituent may be based on a cyclopentanophenanthrene skeleton, which may be partially or fully unsaturated, or saturated. The carbon atoms in the skeleton each may be substituted with Me or OH. For example, the lipophilic substituent may be cholyl, deoxycholyl or lithocholyl.

As mentioned above, the lipophilic substituent may be conjugated to the amino acid side chain by a spacer. When present, the spacer is attached to the lipophilic substituent and to the amino acid side chain. The spacer may be attached to the lipophilic substituent and to the amino acid side chain independently by an ester, a sulphonyl ester, a thioester, an amide or a sulphonamide. Accordingly, it may include two moieties independently selected from acyl, sulphonyl, an N atom, an O atom or an S atom. The spacer may have the formula: 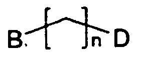 wherein B and D are each independently selected from acyl, sulphonyl, NH, N-alkyl, an O atom or an S atom, preferably from acyl and NH. Preferably, n is an integer from 1 to 10, preferably from 1 to 5. The spacer may be further substituted with one or more substituents selected from C₁₋₆ alkyl, amino-C₁₋₆ alkyl, hydroxy-C₁₋₆ alkyl and carboxy₁₋₆ alkyl.

Alternatively, the spacer may have two or more repeat units of the formula above. B, D and n are each selected independently for each repeat unit. Adjacent repeat units may be covalently attached to each other via their respective B and D moieties. For example, the B and D moieties of the adjacent repeat units may together form an ester, a sulphonyl ester, a thioester, an amide or a sulphonamide. The free B and D units at each end of the spacer are attached to the amino acid side chain and the lipophilic substituent as described above.

Preferably the spacer has five or fewer, four or fewer or three or fewer repeat units. Most preferably the spacer has two repeat units, or is a single unit.

The spacer (or one or more of the repeat units of the spacer, if it has repeat units) may be, for example, a natural or unnatural amino acid. It will be understood that for amino acids having functionalised side chains, B and/or D may be a moiety within the side chain of the amino acid. The spacer may be any naturally occurring or unnatural amino acid. For example, the spacer (or one or more of the repeat units of the spacer, if it has repeat units) may be Gly, Pro, Ala, Val, Leu, Ile. Met, Cys, Phe, Tyr, Trp, His, Lys, Arg, Gln, Asn, Glu, γ-Glu, ε-Lys, Asp, Ser, Thr, Gaba, Aib, β-Ala (i.e. 3-aminopropanoyl), 4-aminobutanoyl, 5-aminopentanoyl, 6-aminohexanoyl, 7-aminoheptanoyl, 8-aminooctanoyl, 9-aminononanoyl, 10-aminodecanoyl or 8-amino-3,6-dioxaoctanoyl. In certain embodiments, the spacer is a residue of Glu, γ-Glu, ε-Lys, β -Ala (i.e. 3-aminopropanoyl), 4-aminobutanoyl, 8-aminooctanoyl or 8-amino-3,6-dioxaoctanoyl.

For example, the spacer may be a single amino acid selected from γ-Glu, Gaba, β -Ala and -Gly.

The lipophilic substituent may be conjugated to any amino acid side chain in the compound. Preferably, the amino acid side chain includes a carboxy, hydrox, thiol, amide or amine group, for forming an ester, a sulphonyl ester, a thioester, an amide or a sulphonamide with the spacer or lipophilic substituent. For example, the lipophilic substituent may be conjugated to a side chain of a Asn, Asp, Glu, Gln, His, Lys, Arg, Ser, Thr, Tyr, Trp, Cys or Dbu, Dpr or Orn residue, e.g. a side chain of a Glu, Lys, Ser, Cys, Dbu, Dpr or Orn residue. For example it may be a side chain of a LyA, Glu or Cys residu. Where two or more side chains carry lipophilic substituent they may be independently selected from these residues. Preferably, the lipophilic substituent is conjugated to Lys. However, any amino acid shown as Lys in the formulae provided herein may be replaced by Dbu, Dpr or Orn where a lipophilic substituent is added.

An example of a lipophilic substituent and spacer is shown in the formula below:

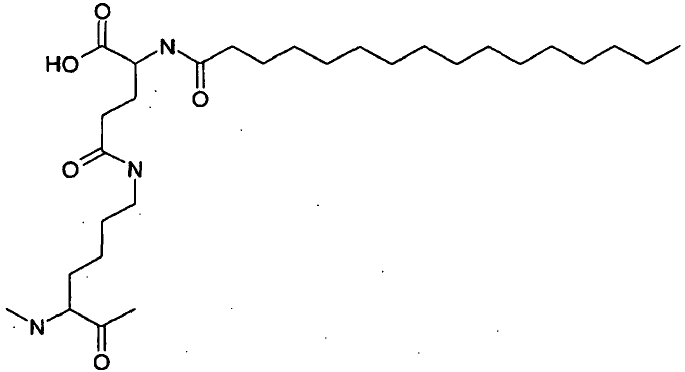

Here, the side chain of a Lys residue from the peptide X is covalently attached to a γ-Glu spacer via an amide linkage. A hexadecanoyl group is covalently attached to the γ-Glu spacer via an amide linkage. This combination of lipophilic moiety and spacer, conjugated to a Lys residue, may be referred to by the short-hand notation K(Hexadecanoyl-γ-Glu), e.g. when shown in formulae of specific compounds. γ-Glu can also be referred to as isoGlu, and a hexadecanoyl group as a palmitoyl group. Thus it will be apparent that the notation (Hexadecanoyl-γ-Glu) is equivalent to the notations (isoGlu(Palm)) or (isoGlu(Palmitoyl)) as used for example in PCT/GB2008/004121.

In certain embodiments, the side chain(s) of one or more of the residues at positions 16, 17, 18, 20, 24, 27, 28 or of Z² are conjugated to a lipophilic substituent. For example, one side chain of such a residue may be conjugated to a lipophilic substituent. Alternatively, two, or even more than two, side chains of such residues may be conjugated to a lipophilic substituent.

In some embodiments, Z¹ is absent and Z² consists of only one amino acid residuse, which can then be regarded as position 30. It may be preferable that position 30 is Cys or Lys.

For example, at least one of positions 16, 17,18, 20 and 28 may be conjugated to a lipophilic substituent. In such cases, position 30 may be absent. When position 30 is present, it is typically conjugated to a lipophilic substituent.

Thus the compound may have just one lipophilic substituent, at position 16, 17, 18, 20, 24, 27, 28 or 30, preferably at position 16, 17 or 20, particularly at position 17.

Alternatively, the compound may have precisely two lipophilic substituents, each at one of positions 16, 17, 18, 20, 24, 27, 28 or 30. Preferably one or both lipophilic substituents are present at one of positions 16, 17 or 20.

Thus, the compound may have lipophilic substituents at positions 16 and 17, 16 and 18, 16 and 20, 16 and 24, 16 and 27, 16 and 28 or 16 and 30; at 17 and 18, 17 and 20, 17 and 24, 17 and 27, 17 and 28 or 17 and 30; at 18 and 20, 18 and 24, 18 and 27, 18 and 28 or 18 and 30; at 20 and 24, 20 and 27, 20 and 28 or 20 and 30; at 24 and 27, 24 and 28 or 24 and 30; at 27 and 28 or 27 and 30; or at 28 and 30.

In yet further embodiments, the compound may have one or more further lipophilic substituents (giving three or more in total) at further positions selected from positions 16, 17, 18, 20, 24, 27, 28 or 30. However it may be desirable that a maximum of two positions are derivatised in this way.

Certain combinations of lipophilic moiety and spacer are dodecanoyl-γ-Glu, hexadecanoyl-γ-Glu, hexadecanoyl-Glu, hexadecanoyl-[3-aminopropanoyl], hexadecanoyl-[8-aminooctanoyl] hexadecanoyl-ε-Lys, 2-butyloctanoyl-γ-Glu, octadecanoyl-γ-Glu and hexadecanoyl-[4-aminobutanoyl].

In certain embodiments, the peptide X may have the sequence:
HSQGTFTSDYSKYLDKKAAHDFVEWLLRA, (Compound 123)
HSQGTFTSDYSKYLDSKAAKDFVEWLLRA; (Compound 124)
HSQGTFTSDYSKYLDSKAAHDFVEWLKRA; (Compound 125)
HSQGTFTSDYSKYLDSKAAHDFVEWLLKA; (Compound 126)
HSQGTFTSDYSRYLDSKAAHDFVEWLLRA; (Compound 127)
HSQGTFTSDYSLYLDSKAAHDFVEWLLRA; (Compound 128)
HSQGTFTSDYSKYLDSKAAHDFVEWLLRAK; (Compound 129)
HSQGTFTSDYSKYLDSKAAHDFVEWLLSAK (Compound 130)
HSQGTFTSDYSKYLDSKAAHDFVEWLKSA; (Compound 131)
HSQGTFTSDYSKYLDSKAAHDFVKWLLRA; (Compound 132)
HSQGTFTSDYSKYLDSCAAHDFVEWLLRA; (Compound 133)
HSQGTFTSDYSKYLDSCAAHDFVEWLLSA; (Compound 134)
HSQGTFTSDYSKYLDSKAACDFVEWLLRA; (Compound 135)
HSQGTFTSDYSKYLDKSAAHDFVEWLLRA; (Compound 136)
H-Aib-QGTFTSDYSKYLDSKAAHDFVEWLLSA; (Compound 137)
H-Aib-QGTFTSDYSKYLDSKAAHDFVEWLLSAK; (Compound 138)
H-Aib-QGTFTSDYSKYLDSKAARDFVAWLLRA; (Compound 139)
H-Aib-QGTFTSDYSKYLDSKAAKDFVAWLLRA; (Compound 140)
H-Aib-QGTFTSDYSKYLDSKAAHDFVEWLLKA (Compound 141)
H-Aib-QGTFTSDYSKYLDSKAAKDFVAWLLSA (Compound 142)
H-Aib-QGTFTSDYSKYLDSKAAHDFVAWLLKA; (Compound 143)
H-Aib-QGTFTSDYSKYLDKKAAHDFVAWLLRA; (Compound 144)
H-Aib-QGTFTSDYSRYLDSKAAHDFVEWLLSA; (Compound 145)
H-Aib-QGTFTSDYSKYLDSKAAHDFVKWLLSA; (Compound 146)
H-Aib-QGTFTSDYSLYLDSKAAHDFVEWLLSA; (Compound 147)
H-Aib-QGTFTSDYSKYLDSCAAHDFVEWLLSA; (Compound 148)
H-Alb-QGTFTSDYSKYLDSKAACDFVEWLLRA; (Compound 149)
H-Aib-QGTFTSDYSKYLDK()KAAE()DFVEWLLRA; (Compound 150)
H-Aib-QGTFTSDYSKYLDSKAAHDFVE()WLLK()A (Compound 151)
H-Aib-QGTFTSDYSKYLDSKAAK()DFVE()WLLRA; (Compound 152)
H-Aib-QGTFTSDYSKYLDSK()AAHE()FVEWLLKA; or (Compound 153)
H-Aib-QGTFTSDYSKYLDSK()AAKE()FVEWLLRA. (Compound 154)
HSQGTFTSDYSKYLDRARADDFVAWLKSA; (Compound 155)
HSQGTFTSDYSKYLDRARADDFVAWLKEA; (Compound 156)
HSQGTFTSDYSKYLDRARAEDFVAWLKST; (Compound 157)
HSQGTFTSDYSKYLDRARADDFVEWLKST; (Compound 158)
H-DSer-QGTFTSDYSKYLDRARADDFVAWLKST; (Compound 159)
HSQGTFTSDYSKYLDRARAHDFVAWLKST; (Compound 160)
HSQGTFTSDYSKYLDKARADDFVAWLKST; (Compound 161)
HSQGTFTSDYSKYLDRAKADDFVAWLKST; (Compound 162)
HSQGTFTSDYSKYLDRARAKDFVAWLKST, or (Compound 163)
HSQGTFTSDYSKYLDRARADDFVKWLKST (Compound 164)

In certain embodiments these peptides may carry a lipophilic substituent at the position marked "*" as follows:
WSQGTFTSDYSKYLDS-K*-AAHDFVEWLLRA; (Compound 165)
HSQGTFTSDYSKYLD-K*-KAAHDFVEWLLRA; (Compound 166)
HSQGTFTSDYSKYLDSKAA-K*-DFVEWLLRA; (Compound 167)
HSQGTFTSDYSKYLDSKAAHDFVEWL-K*-RA; (Compound 168)
HSQGTFTSDYSKYLDSKAAHDFVEWLL-K*-A; (Compound 169)
HSQGTFTSDYSRYLDS-K*-AAHDFVEWLLRA; (Compound 170)
HSQGTFTSDYSLYLDS-K*-AAHDFVEWLLRA; (Compound 171)
HSQGTFTSDYSKYLDSKAAHDFVEWLLRA-K*; (Compound 172)
HSQGTFTSDYSKYLDSKAAHDFVEWLLSA-K*; (Compound 173)
HSQGTFTSDYSKYLDSKAAHDFVEWL-K*-SA; (Compound 174)
HSQGTFTSDYSKYLDSKAAHDFV-K*-WLLRA; (Compound 175)
HSQGTFTSDYSKYLDS-C*-AAHDFVEWLLRA; (Compound 176)
HSQGTFTSDYSKYLDS-C*-AAHDFVEWLLSA; (Compound 177)
HSQGTFTSDYSKYLDSKAA-C*-DFVEWLLRA; (Compound 178)
HSQGTFTSDYSKYLD-K*-SAAHDFVEWLLRA; (Compound 179)
H-Aib-QGTFTSDYSKYLDS-K*-AAHDFVEWLLSA; (Compound 180)
H-Aib-QGTFTSDYSKYLDSKAAHDFVEWLLSA-K*. (Compound 181)
H-Aib-QGTFTSDYSKYLDS-K*-AARDFVAWLLRA; (Compound 182)
H-Aib-QGTFTSDYSKYLDSKAA-K*-DFVAWLLRA; (Compound 183)
H-Aib-QGTFTSDYSKYLDSKAAHDFVEWLL-K*-A; (Compound 184)
H-Aib-QGTFTSDYSKYLDS-K*-AAHDFVEWLLKA; (Compound 185)
H-Aib-QGTFTSDYSKYLDS-K*-AAHDFVEWLLRA; (Compound 186)
H-Aib-QGTFTSDYSKYLDSKAA-K*-DFVAWLLSA; (Compound 187)
H-Aib-QGTFTSDYSKYLDSKAAHDFVAWLL-K*-A; (Compound 188)
H-Aib-QGTFTSDYSKYLD-K*-KAAHDFVAWLLRA; (Compound 189)
H-Aib-QGTFTSDYSRYLDS-K*-AAHDFVEWLLSA; (Compound 190)
H-Aib-QGTFTSDYSKYLDSKAAHDFV-K*-WLLSA; (Compound 191)
H-Aib-QGTFTSDYSLYLDS-K*-AAHDFVEWLLSA; (Compound 192)
H-Aib-QGTFTSDYSKYLDS-C*-AAHDFVEWLLSA; (Compound 193)
H-Aib-QGTFTSDYSKYLDSKAA-C*-DFVEWLLRA; (Compound 194)
H-Aib-QGTFTSDYSKYLD-S*-KAAHDFVEWLLSA; (Compound 195)
H-Aib-QGTFTSDYSKYLDK()K*AAE()DFVEWLLRA; (Compound 196)
H-Aib-QGTFTSDYSKYLDSK*AAHDFVE()WLLK()A; (Compound 197)
H-Aib-QGTFTSDYSKYLDSK*AAK()DFVE()WLLRA; (Compound 198)
H-Aib-QGTFTSDYSKYLDSK()AAHE()FVEWLLK*A; or (Compound 199)
H-Aib-QGTFTSDYSKYLDSK()AAK*E()FVEWLLRA. (Compound 200)

Residues marked "()" participate in an intramolecular bond, such as a lactam ring, as described above.

In particular embodiments, the derivatised peptide X has the formula:
HSQGTFTSDYSKYLDS-K(Hexadecanoyl-γ-Glu)-AAHDFVEWLLRA; (Compound 201)
HSQGTFTSDYSKYLD-K(Hexadecanoyl-γ-Glu)-KAAHDFVEWLLRA; (Compound 202)
HSOGTFTSDYSKYLDSKAAHDFVEWL-K(Hexadecanoyl-γ-Glu)-RA; (Compound 203)
HSQGTFTSDYSKYLDSKAA-K(Hexadecanoy)-γ-Glu)-DFVEWLLRA: (Compound 204)
HSQGTFTSDYSKYLDSKAAHDFVEWLL-K(Hexadecanoyl-γ-Glu)-A; (Compound 205)
H-Aib-Q'GTFTSDYSKYLDS-K(Hexadecanoyl-γ-Glu)-AAHDFVEWLLRA; (Compound 206)
H-Aib-QGTFTSDYSKYLDS-K(Hexadecanoyl-γ-Glu)-AARDFVAWLLRA; (Compound 207)
H-Aib-QGTFTSDYSKYLDS-K(Hexadecanoyl-γ-Glu)-AAHDFVEWLLSA; (Compound 208)
H-Aib-QGTFTSDYSKYLDSKAAHDFVEWLL-K(Hexadecanoyl-γ-Glu)-A; (Compound 209)
H-Aib-QGTFTSDYSKYLDS-K(Hexadecanoyl-γ-Glu)-AAHDFVE()WLLK()A; (Compound 210)
H-Aib-QGTFTSDYSKYLDS-K(Hexadecanoyl-γ-Glu)-AAHDFVEWLLKA; (Compound 211)
HSQGTFTSDYSKYLDS-K(Hexadecanoyl-γ-Glu)-AAHDFVEWLLRA; (Compound212)
H-Aib-QGTFTSDYSKYLDSKAA-K(Hexadecanoyl-γ-Glu)-DFVAWLLRA; (Compound 213)
H-Aib-QGTFTSDYSKYLDS-K(Dodecanoyl-γ-Glu)-AAHDFVEWLLSA; (Compound 214)
H-Aib-QGTFTSDYSKYLDS-K(Hexadecanoyl-[3-aminopropanoyl])-AAHDFVEWLLSA; (Compound 215)
H-Aib-QGTFTSDYSKYLDS-K(Hexadecanoyl-[8-aminooctanoyl])-AAHDFVEWLLSA; (Compound 216)
H-Aib-QGTFTSDYSKYLDS-K(Hexadecanoyl-ε-Lys)-AAHDFVEWLLSA: (Compound 217)
HSQGTFTSDYSKYLDS-K(Hexadecanoyl)-AAHDFVEWLLSA; (Compound 218)
HSQGTFTSDYSKYLDS-K(Octadecanoyl- γ-Glu)-AAHDFVEWLLSA; (Compound 219)
HSQGTFTSDYSKYLDS-K([2-Butyloctanoyl]-γ-Glu)-AAHDFVEWLLSA; (Compound 220)
HSQGTFTSDYSKYLDS-K(Hexadecanoyl-[4-Aminobutanoyl])-AAHDFVEWLLSA; (Compound 221)
HSQGTFTSDYSKYLDS-K(Octadecanoyl- γ-Glu)-AAHDFVEWLLSA; (Compound 222)
HSQGTFTSDYSKYLDS-K(Hexadecanoyl-E)-AAHDFVEWLLSA; (Compound 223)
H-Aib-QGTFTSDYSKYLDS-K(Hexadecanoyl)-AAHDFVEWLLSA; (Compound 224)
H-Aib-QGTFTSDYSKYLDS-K(Octadecanoyl- γ-Glu)-AAHDFVEWLLSA; (Compound 225)
H-Aib-QGTFTSDYSKYLDS-K([2-Butyloctanoyl]-γ-Glu)-AAHDFVEWLLSA; (Compound 226)
H-Aib-QGTFTSDYSKYLDS-K(Hexadecanoyl-[4-Aminobutanoyl])-AAHDFVEWLLSA; (Compound 227)
HSQGTFTSDYSKYLDRARADDFVAWLK(Hexadecanoyl-γ-Glu)-SA; (Compound 228)
HSQGTFTSDYSKYLDRARADDFVAWLK(Hexadecanoyl-γ-Glu)-EA; (Compound 229)
HSQGTFTSDYSKYLDRARAEDFVAWLK(Hexadecanoyl-γ-Glu)-ST; (Compound 230)
HSQGTFTSDYSKYLDRARADDFVEWLK(Hexadecanoyl-γ-Glu)-ST; (Compound 231)
H-DSer-QGTFTSDYSKYLDRARADDFVAWLK(Hexadecanoyl-γ-Glu)-ST; (Compound 232)
HSQGTFTSDYSKYLDRARAHDFVAWLK(Hexadecanoy)-γ-Glu)-ST: (Compound 233)
HSQGTFTSDYSKYLDRARADDFVAWLK(Hexadecanoyl-γ-Glu)-ST; (Compound 234)
HSQGTFTSDYSKYLDK(Hexadecanoyl-γ-Glu)-ARADDFVAWLKST; (Compound 235)
HSQGTFTSDYSKYLDRAK(Hexadecanoyl-γ-Glu)-ADDFVAWLKST; (Compound 236)
HSQGTFTSDYSKYLDRARAK(Hexadecanoyl-γ-Glu)-DFVAWLKST; (Compound 237)
HSQGTFTSDYSKYLDRARADDFVK(Hexadecanoyl-γ-Glu)-WLKST: (Compound 238)
H-Aib-QGTFTSDYSKYLDS-K(Octadecanoyl- γ-Glu)-AAHDFVEWLLSA; or (Compound 239)
H-Aib-QGTFTSDYSKYLDS-K(Hexadecanoyl-E)-AAHDFVEWLLSA. (Compound 240)
Residues marked "()" participate in an intramolecular bond, such as a lactam ring.

Alternatively or additionally, one or more amino acid side chains in the compound of the invention may be conjugated to a polymeric moiety, for example, in order to increase solubility and/or half-life in vivo (e.g. in plasma) and/or bioavailability. Such modification is also known to reduce clearance (e.g. renal clearance) of therapeutic proteins and peptides.

The polymeric moiety is preferably water soluble (amphiphilic or hydrophilic), non-toxic, and pharmaceutically inert. suitable polymeric moieties include polyethylene glycol (PEG), homo- or co-polymers of PEG, a-monomethyl-substituted polymer of PEG (mPEG), or polyoxyethylene glycerol (POG). See, for example, Int. J. Hematology 68:1 (1998); Bioconjugate Chem. 6:150 (1995); and Crit. Rev. Therap. Drug Carrier Sys. 9:249 (1992).

Other suitable polymeric moieties include poly-amino acids such as poly-lysine, poly-aspartic acid and poly-glutamic acid (see for example Gombotz, et al. (1995), Bioconjugate Chem. , vol. 6 : 332-351; Hudecz, et al. (1992), Bioconjugate Chem. , vol. 3, 49-57; Tsukada, et al. (1984), J. Natl. Cancer Inst. , vol 73, : 721-729; and Pratesi, et al. (1985), Br. J. Cancer, vol. 52: 841-848).

The polymeric moiety may be straight-chain or branched. It may have a molecular weight of 500-40,000 Da, for example 500-5,000 Da, 500-10,000 Da, 1000-5000 Da, 10,000-20,000 Da, or 20,000-40,000Da.

A compound may comprise two or more such moieties, in which case the total molecular weight of all such moieties will generally fall within the ranges provided above.

The polymeric moiety may be coupled (by covalent linkage) to an amino, carboxyl or thiol group of an amino acid side chain. Preferred examples are the thiol group of Cys residues and the epsilon amino group of Lys residues, and the carboxyl groups of Asp and Glu residues may also be used.

For example, the polymeric moiety may be coupled to the side chain of the residue at one or more of positions 16, 17 18, 20, 21, 24 or 29, or to the C-terminus of the peptide. For example, it may be coupled at one or more of positions 16, 17, 21 and 24.

The skilled reader will be well aware of suitable techniques which can be used to perform the coupling reaction. For example, a PEG moiety carrying a methoxy group can be coupled to a Cys thiol group by a maleimido linkage using regents commercially available from Nektar Therapeutics AL. See also WO 2008/101017, and the references cited above for details of suitable chemistry.

### Biological activity

Binding of the relevant compounds to GLP-1 or glucagon (Glu) receptors may be used as an indication of agonist activity, but in general it is preferred to use a biological assay which measures intracellular signalling caused by binding of the compound to the relevant receptor. For example, activation of the glucagon receptor by a glucagon agonist will stimulate cellular cyclic AMP (cAMP) formation. Similarly, activation of the GLP-1 receptor by a GLP-1 agonist will stimulate cellular cAMP formation. Thus, production of cAMP in suitable cells expressing one of these two receptors can be used to monitor the relevant receptor activity. Use of a suitable pair of cell types, each expressing one receptor but not the other, can hence be used to determine agonist activity towards both types of receptor.

The skilled person will be aware of suitable assay fomats, and examples are provided below. The GLP-1 receptor and/or the glucagon receptor may have the sequence of the receptors as described in the examples. For example, the assays may make use of the human glucagon receptor (Glucagon-R) having primary accession number GI:4503947 and/or the human glucagon-like peptide 1 receptor (GLP-1 R) having primary accession number GI:166795283. (Where sequences of precursor proteins are referred to, it should of course be understood that assays may make use of the mature protein, lacking the signal sequence).

EC₅₀ values may be used as a numerical measure of agonist potency at a given receptor. An EC₅₀ value is a measure of the concentration of a compound required to achieve half of that compound's maximal activity in a particular assay. Thus, for example, a compound having EC₅₀[GLP-1] lower than the EC₅₀[GLP-1R] of glucagon in a particular assay may be considered to have higher potency at the GLP-1 R than glucagon.

The compounds described in this specification are typically Glu-GLP-1 (glucagon-GLP-1) dual agonists, i.e. they are capable of stimulating cAMP formation at both the glucagon receptor and the GLP-1 receptor. The stimulation of each receptor can be measured in independent assays and afterwards compared to each other.

By comparing the EC₅₀ value for the glucagon receptor (EC₅₀ [Glucagon-R]) with the EC₅₀ value for the GLP-1 receptor, (EC₅₀ [GLP-1 R]) for a given compound the relative glucagon selectivity (%) of that compound can be found: $Relative Glucagon - R selectivity \left[Compound\right] = \left(1/{EC}_{50} \left[Glucagon-R\right]\right) \times 100 / \left(1/{EC}_{50} \left[Glucagon-R\right]+1/{EC}_{50} \left[GLP-1R\right]\right)$

The relative GLP-1R selectivity can likewise be found: $Relative GLP - 1 R selectivity \left[Compound\right] = \left(1/{EC}_{50} \left[GLP-1R\right]\right) \times 100 / \left(1/{EC}_{50} \left[Glucagon-R\right]+1/{EC}_{50} \left[GLP-1R\right]\right)$

A compound's relative selectivity allows its effect on the GLP-1 or glucagon receptor to be compared directly to its effect on the other receptor. For example, the higher a compound's relative GLP-1 selectivity is, the more effective that compound is on the GLP-1 receptor as compared to the glucagon receptor.

Using the assays described below, we have found the relative GLP-1 selectivity for human glucagon to be approximately 5%.

Compounds suitable for use in the methods of the present disclosure typically have a higher relative GLP-1R selectivity than human glucagon. Thus, for a particular level of glucagon-R agonist activity, the compound will display a higher level of GLP-1 R agonist activity (i.e. greater potency at the GLP-1 receptor) than glucagon. It will be understood that the absolute potency of a particular compound at the glucagon and GLP-1 receptors may be higher, lower or approximately equal to that of native human glucagon, as long as the appropriate relative GLP-1 R selectivity is achieved.

Nevertheless, the compounds may have a lower EC₅₀ [GLP-1 R] than human glucagon. The compounds may have a lower EC₅₀[GLP-1-R] than glucagon while maintaining an EC₅₀ [Glucagon-R] that is less than 10-fold higher than that of human glucagon, less than 5-fold higher than that of human glucagon, or less than 2-fold higher than that of human glucagon.

The compounds may have an EC₅₀ [Glucagon-R] that is less than two-fold that of human glucagon. The compounds may have an EC₅₀ [Glucagon-R] that is less than two-fold that of human glucagon and have an EC₅₀ [GLP-1R] that is less than half that of human glucagon, less than a fifth of that of human glucagon, or less than a tenth of that of human glucagon.

The relative GLP-1 selectivity of the compounds may be between 10% and 95%. For example, the compounds may have a relative selectivity of 10-20%, 10-30%, 20-50%, 30-70%, or 50-80%; or of 30-50%, 40-60%, 50-70% or 75-95%.

### Therapeutic uses

The methods of the present disclosure are applicable for conditions in which it is desirable to improve cardiac function directly, e.g. where there is a dysfunction of the cardiac muscle (myocardium) itself. Such conditions include myocardial infarction, heart failure and cardiogenic shock. Positive inotropic agents increase the strength of myocardial contraction, and are used to improve hemodynamic parameters and thereby relieve symptoms and protect end-organs in patients with myocardial infarction, cardiogenic shock, or heart failure. Known inotropic agents such as dobutamine, norepinephrine and glucagon exert their effects (increase in cardiac work) at the expense of increased cardiac energy demand and can therefore have a severe depleting effect on the heart's energy reserves (as measured e.g. by total phosphocreatine (PCr), total ATP, or by PCr/ATP, ATP/ADP or ATP/AMP ratios). Since the failing or diseased heart is often energy-starved, the use of inotropic agents may therefore result in energy depletion and consequently in an increased incidence of arrhythmias as well as in increased short- and long-term mortality (Jessup M et al., Circulation 2009;119:1977-2016). Because of this, current guidelines for treatment of heart failure state that positive inotropic agents should only "be considered for palliation of symptoms in patients with refractory end-stage heart failure" (Dickstein K et al., Eur Heart J 2008;29:2388-2442), and that such agents should be "withdrawn as soon as adequate organ perfusion is restored and/or congestion reduced" (Jessup et al., op cit.). Typically, then, inotropic agents are adminstered only in order to stabilise a patient's condition, but withdrawn after a few hours or a few days.

Without wishing to be bound by any particular theory, it is believed that the compounds described above for use in the methods of the present disclosure act as glucagon-GLP-1 dual agonists (although they may exert their beneficial cardiac effects by a different mechanism, e.g. via a distinct receptor). They have surprisingly been found to increase cardiac inotropy while simultaneously improving myocardial metabolism, in particular preserving the energetic state of the heart, or at least depleting the reserves of high energy phosphates to a lesser extent than the other inotropic agents discussed above. They are therefore particularly useful for treating an individual suffering from myocardial infarction, heart failure, cardiogenic shock or any other condition where increased cardiac inotropy is desired without compromising the energetic state of the heart, i.e. any abnormality of cardiac function which results in the inability of the heart to pump blood at a rate commensurate with the requirements of the metabolizing tissues and/or allows it to do so only from an abnormally elevated ventricular diastolic volume. This includes, but is not restricted to; congestive heart failure, systolic dysfunction, diastolic dysfunction, myocardial infarction, ischemic heart disease, diabetic cardiomyopathy, or combinations thereof.

The myocardial energy status may be monitored by determining total phosphocreatine (PCr), total ATP, or PCr/ATP, ATP/ADP or ATP/AMP ratios. Such determinations may be made by biopsy (e.g. as described in Ally A and Park G. Rapid determination of creatine, phosphocreatine, purine bases and nucleotides (ATP, ADP, AMP, GTP, GDP) in heart biopsies by gradient ion-pair reversed-phase liquid chromatography. J Chromatogr 1992;575:19-27) or by magnetic resonance spectroscopy (Neubauer S et al., Myocardial phosphocreatine-to-ATP ratio is a predictor of mortality in patients with dilated cardiomyopathy. Circulation 1997;96:2190-2196; Yabe T et al., Quantitative measurements of cardiac phosphorus metabolites in coronary artery disease by 31P magnetic resonance spectroscopy. Circulation 1995;92:15-23).

By improving myocardial metabolism simultaneously with having positive inotropic effects, the compounds for use in accordance with this invention may be associated with fewer arrhythmias and/or lower mortality than current positive inotropic agents. Consequently, the methods of the present disclosure may be used for patients with less severe disease and/or for longer periods of time in those with severe heart failure, than is currently recommended.

For example, the subject may be treated with a suitable compound for a period greater than 12 hours, greater than 24 hours, greater than 36 hours or greater than 48 hours. For example, the subject may be treated for a period greater than 3 days, e.g. greater than 4, 5, 6, 7, 8, 9, 10, 11, 12, 13 or 14 days. The patient may be treated for a period greater than 2 weeks, greater than 3 weeks or greater than 4 weeks. The patient may be treated for a period greater than 1 month, 2 months, 3 months, 4 months,r 5 months. i.e. chronic/lifelong treatment.

The patient may be treated for a period between 1 week and 6 weeks, e.g. between 2 weeks and 6 weeks, between 3 weeks and 6 weeks, between 4 weeks and 6 weeks or between 5 weeks and 6 weeks.

The patient may be treated for a period between 1 week and 5 weeks, e.g. between 2 weeks and 5 weeks, between 3 weeks and 5 weeks, between 4 weeks and 5 weeks.

The patient may be treated for a period between 1 week and 4 weeks, e.g. between 2 weeks and 4 weeks, between 3 weeks and 4 weeks.

The patient may be treated for a period between 1 week and 3 weeks, e.g. between 2 weeks and 3 weeks.

For example, the patient may be treated for a period between 1 week and 6 months, e.g. between 1 week and 5 months, between 1 week and 4 months, between 1 week and 3 months, between 1 week and 2 months, or between 1 week and 1 month.

The patient may be treated for a period between 2 weeks and 6 months, e.g. between 2 weeks and 5 months, between 2 weeks and 4 months, between 2 weeks and 3 months, between 2 weeks and 2 months, or between 2 weeks and 1 month.

The patient may be treated for a period between 3 weeks and 6 months, e.g. between 3 weeks and 5 months, between 3 weeks and 4 months, between 3 weeks and 3 months, between 3 weeks and 2 months, or between 3 weeks and 1 month.

The patient may be treated for a period between 4 weeks and 6 months, e.g. between 4 weeks and 5 months, between 4 weeks and 4 months, between 4 weeks and 3 months, between 4 weeks and 2 months, or between 4 weeks and 1 month.

The patient may be treated for a period between 1 month and 6 months, e.g. between 2 months and 6 months, between 3 months and 6 months, between 4 months and 6 months, between 5 months and 6 months.

The patient may be treated for a period between 1 month and 5 months, e.g. between 2 months and 5 months, between 3 months and 5 months, between 4 months and 5 months.

The patient may be treated for a period between 1 month and 3 months, e.g. between 2 months and 3 months.

The patient may be treated for a period between 1 month and 2 months.

In some cases treatment may comprise a dosage regime of continuous infusion, twice daily or once daily.

Other dosage regimes are contemplated, including a dosage regime that may be once daily, twice daily, once weekly, once bi-weekly or once monthly.

### Pharmaceutical compositions

The compounds described for use in this specification, or salts thereof, may be formulated as pharmaceutical compositions prepared for storage or administration, which typically comprise a therapeutically effective amount of a compound or salt thereof in a pharmaceutically acceptable carrier.

The precise amount to be administered will depend on the route of administration, the type of mammal being treated, and the physical characteristics of the specific mammal under consideration. These factors and their relationship to determining this amount are well known to skilled practitioners in the medical arts. This amount and the method of administration can be tailored to achieve optimal efficacy, and may depend on such factors as weight, diet, concurrent medication and other factors, well known to those skilled in the medical arts. The dosage levels and dosing regimen most appropriate for human use may be established on the basis of the results obtained by the present invention, and may be confirmed in properly designed clinical trials.

An effective dosage and treatment protocol may be determined by conventional means, starting with a low dose in laboratory animals and then increasing the dosage while monitoring the effects, and systematically varying the dosage regimen as well. Numerous factors may be taken into consideration by a clinician when determining an optimal dosage for a given subject. Such considerations are known to the skilled person.

The term "pharmaceutically acceptable carrier" includes any of the standard pharmaceutical carriers. Pharmaceutically acceptable carriers for therapeutic use are well known in the pharmaceutical art, and are described, for example, in Remington's Pharmaceutical Sciences, Mack Publishing Co. (A. R. Gennaro edit. 1985). For example, sterile saline and phosphate-buffered saline at slightly acidic or physiological pH may be used. pH buffering agents may be phosphate, citrate, acetate, tris/hydroxymethyl)aminomethane (TRIS), N-Tris(hydroxymethyl)methyl -3- aminopropanesulphonic acid (TAPS), ammonium bicarbonate, diethanolamine, histidine, which is a preferred buffer, arginine, lysine, or acetate or mixtures thereof. The term further encompases any agents listed in the US Pharmacopeia for use in animals, including humans.

The term "pharmaceutically acceptable salt" refers to the salts of the dual agonist compounds. Pharmaceutically acceptable salts typically include acid addition salts and basic salts. Examples of pharmaceutically acceptable acid addition salts include hydrochloride salts, citrate salts and acetate salts. Examples of pharmaceutically acceptable basic salts include salts where the cation is selected from alkali metals, such as sodium and potassium, alkaline earth metals, such as calcium, and ammonium ions ⁺N (R³)₃(R⁴), where R³ and R⁴ independently designates optionally substituted C₁₋₆-alkyl, optionally substituted C₂₋₆-alkenyl, optionally substituted aryl, or optionally substituted heteroaryl. Other examples of pharmaceutically acceptable salts are described in "Remington's Pharmaceutical Sciences" ,17th edition. Ed. Alfonso R. Gennaro (Ed.), Mark Publishing Company, Easton, PA, U.S.A., 1985 and more recent editions, and in the Encyclopaedia of Pharmaceutical Technology.

"Treatment" is an approach for obtaining beneficial or desired clinical results. For the purposes of this disclosure, beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. "Treatment" is an intervention performed with the intention of preventing the development of, or altering the pathology of, a disorder. Accordingly, "treatment" refers to both therapeutic treatment and prophylactic or preventative measures. Those in need of treatment include those already with the disorder as well as those in which the disorder is to be prevented

The pharmaceutical compositions can be in unit dosage form. In such form, the composition is divided into unit doses containing appropriate quantities of the active component. The unit dosage form can be a packaged preparation, the package containing discrete quantities of the preparations, for example, packeted tablets, capsules, and powders in vials or ampoules. The unit dosage form can also be a capsule, cachet, or tablet itself, or it can be the appropriate number of any of these packaged forms. It may be provided in single dose injectable form, for example in the form of a pen. Compositions may be formulated for any suitable route and means of administration. Pharmaceutically acceptable carriers or diluents include those used in formulations suitable for oral, rectal, nasal, topical (including buccal and sublingual), vaginal or parenteral (including subcutaneous, intramuscular, intravenous, intradermal, and transdermal) administration. The formulations may conveniently be presented in unit dosage form and may be prepared by any of the methods well known in the art of pharmacy.

Intravenous, subcutaneous or transdermal modes of administration may be particularly suitable for the compounds described herein.

### Combination therapy

The compounds described above may be administered as part of a combination therapy with an agent for treatment of heart failure, diabetes, obesity, myocardial infarction, hypertension, or hypolipidemia.

In such cases, the two active agents may be given together or separately, and as part of the same pharmaceutical formulation or as separate formulations.

Thus the compound (or salt thereof) can be used in combination with an anti-diabetic agent including but not limited to metformin, a sulfonylurea, a glinide, a DPP-IV inhibitor, a glitazone, or insulin. In a preferred embodiment the compound or salt thereof is used in combination with insulin, DPP-IV inhibitor, sulfonylurea or metformin, particularly sulfonylurea or metformin, for achieving adequate glycemic control. In an even more preferred embodiment the compound or salt thereof is used in combination with insulin or an insulin analogue for achieving adequate glycemic control. Examples of insulin analogues include but are not limited to Lantus^{™}, Novorapid^{™}, Humalog^{™}, Novomix^{™}, and Actraphane HM^{™}.

The compound or salt thereof can further be used in combination with an anti-obesity agent including but not limited to a glucagon-like peptide receptor 1 agonist, peptide YY or analogue thereof, cannabinoid receptor 1 antagonist, lipase inhibitor, melanocortin receptor 4 agonist, or melanin concentrating hormone receptor 1 antagonist.

The analogue compound or salt thereof can be used in combination with an anti-hypertension agent including but not limited to an angiotensin-converting enzyme (ACE) inhibitor, angiotensin II receptor blocker (ARB), diuretics, beta-blocker, or calcium channel blocker.

The analogue compound or salt thereof can in particular be used in combination with an agent for treatment of myocardial infarction, heart failure or cardiogenic shock including but not limited to diuretics, angiotensin-converting enzyme (ACE) inhibitor, angiotensin II receptor blocker (ARB), aldosterone antagonists, digitalis, acute ionotropes and inotropic dilators.

The analogue compound or salt thereof can in particular be used in combination with classes of hypolipidemic drugs such as cholesterol lovering agents including but not limited to statins (HMG-CoA reductase inhibitors), fibrates and niacin.

### Recombinant expression

The compounds for use in the invention may be expressed by recombinant techniques, particularly when they consist entirely of naturally occurring amino acids. For recombinant expression, nucleic acids encoding the relevant compounds will normally be inserted in suitable vectors to form cloning or expression vectors carrying the coding sequences. The vectors can, depending on purpose and type of application, be in the form of plasmids, phages, cosmids, mini-chromosomes, or virus, but also naked DNA which is only expressed transiently in certain cells is an important vector. Cloning and expression vectors (plasmid vectors) may be capable of autonomous replication, thereby enabling high copy-numbers for the purposes of high-level expression or high-level replication for subsequent cloning.

In general outline, an expression vector comprises the following features in the 5'→3' direction and in operable linkage: a promoter for driving expression of the relevant coding nucleic acid, optionally a nucleic acid sequence encoding a ieader peptide enabling secretion (to the extracellular phase or, where applicable, into the periplasma), the nucleic acid fragment encoding the compound, and optionally a nucleic acid sequence encoding a terminator. They may comprise additional features such as selectable markers and origins of replication. When operating with expression vectors in producer strains or cell lines it may be preferred that the vector is capable of integrating into the host cell genome. The skilled person is very familiar with suitable vectors and is able to design one according to their specific requirements.

The vectors are used to transform host cells to produce the compound. Such transformed cells can be cultured cells or cell lines used for propagation of the nucleic acid fragments and vectors of the invention, or used for recombinant production of the peptides of the invention.

Preferred host cells are micro-organisms such as bacteria (such as the species Escherichia (e.g. E. coli), Bacillus (e.g. Bacillus subtilis), Salmonella, or Mycobacterium (preferably non-pathogenic, e.g. M. bovis BCG), yeasts (such as Saccharomyces cerevisiae), and protozoans. Alternatively, the host cell may be derived from a multicellular organism, i.e. it may be fungal cell, an insect cell, a plant cell, or a mammalian cell. For the purposes of cloning and/or optimised expression it is preferred that the host cell is capable of repilcating the nucleic acid fragment or vector as applicable. Cells expressing the nucleic fragment are useful embodiments of the invention; they can be used for small-scale or large-scale preparation of the compounds.

When producing the compound by means of transformed cells, it is convenient, although far from essential, that the expression product is secreted into the culture medium.

It will be understood that such nucleic acids, expression vectors and host cells may be used for treatment of any of the conditions described herein which may be treated with the compounds themselves. For example, nucleic acids encoding the compounds, particularly expression vectors containing such nucleic acids, may be suitable for direct administration to a subject so that the nucleic acid is taken up and the compound produced by the subject's own cells. The compound is preferably secreted by the cells containing the nucleic acid. Similarly, host cells capable of producing and secreting the compound may be administered to a subject so that the compound is produced in situ. The host cells may be treated (e.g. encapsulated) to inhibit or reduce their immunogenicity to the recipient subject. References to a therapeutic composition comprising a compound, administration of a compound, or any therapeutic use of such a compound, should therefore be construed to encompass the equivalent use of a nucleic acid, expression vector or host ceii as described herein except where the context demands otherwise.

### EXAMPLES

### Example 1. Assessment of inotropic effect in working heart model

The effect of the inotropic compound glucagon and a glucagon-GLP-1 dual-agonist (Compound 12 having the sequence HSQGTFTSDYSKYLDRARADDFVAWLKST) on cardiac function, metabolism, and energy state was evaluated in isolated working hearts (Lopaschuk, GD and Barr, RL. Measurements of fatty acid and carbohydrate metabolism in the isolated working rat heart. Molecular and Cellular Biochemistry. 1997; 172: 137-147) from control and insulin-resistant JCR:LA-cp rats. Isolated working hearts were subjected to aerobic perfusion with Krebs-Henseleit solution (11 mM glucose, 2000 µU/ml insulin, 1.25 mM free Ca²⁺, 0.8 mM palmitate, and 3% BSA) and during perfusion increasing concentrations (10, 50, and 100 mM) of glucagon or Compound 12 was added to the perfusion buffer. Following perfusions, high energy phosphate nucleotide concentrations were measured by high performance liquid chromatography (HPLC) (Ally, A and Park, G. Rapid determination of creatine, phosphocreatine, purine bases and nucleotides (ATP, ADP, AMP, GTP, GDP) in heart biopsies by gradient ion-pair reversed-phase liquid chromatography. Journal of Chromatography. 1992; 575: 19-27).

Glucagon and Compound 12 had similar inotropic effects on cardiac function in both normal (data not shown) and insulin-resistant JCR-LA rats (Figure 1). Despite similar effects on cardiac function and thereby cardiac energy demand, glucagon and Compound 12 had statistically significant different effects on the energetic state of insulin resistant hearts (Figure 2). Specifically, treatment with glucagon caused statistically significant increases in AMP and ADP levels and thereby decreased ATP/AMP and ATP/ADP ratios. However, following treatment with Compound 12 the energetic state of the hearts was not significantly different from vehicle perfused hearts. No effect was observed with the GLP-1 agonist exendin-4[1-39]-K₆ (H-HGEGTFTSDLSKQMEEEAVRLFIEWLKNGGPSSGAS-K₆-NH₂) (Compound 241) (data not shown).

### Example 2. Determination of efficacy at GLP-1 and glucagon receptors

HEK293 cells expressing the human glucagon receptor or human GLP-1 R (see above for details) were seeded at 40,000 cells per well in 96-well microtiter plates coated with 0.01 % poly-L-lysine and grown for 1 day in culture in 100 µl growth medium. On the day of analysis, growth medium was removed and the cells washed once with 200 µl Tyrode buffer. Cells were incubated in 100 µl Tyrode buffer containing increasing concentrations of test peptides, 100 µM IBMX, and 6 mM glucose for 15 min at 37° C. The reaction was stopped by addition of 25 µl 0.5 M HCl and incubated on ice for 60 min. The cAMP content was estimated using the FlashPlate® cAMP kit from Perkin-Elmer. EC₅₀ values were estimated by computer aided curve fitting.

**Table 1 shows results for sample compounds as EC₅₀ values.**

| **Compound No** | **Test compound** | **EC₅₀ (nM) GLP-1R** | **EC₅₀ (nM) GluR** |
|---|---|---|---|
| 1 | H-HSQGTFTSDYSKYLDSRRAQDFVWLMNT-OH (Human glucagon) | 2,0 | 0,1 |
| 12 | H-HSGTGTFTSDYSKYLDRARADDFVAWLKST-NH2 | 0.23 | 0.50 |
| 242 | H-HSQGTFTSDYSAYLDSRRAQDFVVWVLMNT-NH2 | 1,4 | 0,4 |
| 243 | -H-HSQGTFTSDYSKYLDSERAQDFVWLMNT-NH2 | 0,6 | 0,06 |
| 244 | H-HSQGTFTSDYSKYLDSRHAQDFVWLMNT-NH2 | 0,5 | 0,05 |
| 245 | -H-HSQGTFTSDYSKYLDSRSAQDFVWLMNT-NH2 | 0,1 | 0,05 |
| 246 | SQGTFTSDYSKYLDSRAAQDFVWLMNT-NH2 | 0,3 | 0,05 |
| 247 | H-HSQGTFTSDYSKYLDSRYAQDFVWLMNT-NH2 | 0,3 | 0,1 |
| 248 | H-HSQGTFTSDYSKYLDSRRAQDFVWLESA-NH2 | 0,5 | 0,1 |
| 249 | -H-HSQGTFTSDYSKYLDSRRAQDFWVLKSA-NH2 | 0,1 | 0,1 |
| 250 | H-HSQGTFTSDYSKYLDSRRAQDFVWLKRA-NH2 | 0,3 | 0,1 |
| 251 | H-HSQGTFTSDYSKYLDSRRAQDFVWLERA-NH2 | 0,2 | 0,1 |
| 252 | H-HSQGTFTSDYSRYLDSRRAKDFVWLLNT-NH2 | 0,5 | 0,3 |
| 253 | H-HSQGTFTSDYSRYLDSRRAQDFVWLLNT-NH2 | 0,2 | 0,1 |
| 254 | H-HSQGTFTSDYSRYLDSRRAQDFVWLLNK-NH2 | 0,2 | 0,2 |
| 255 | H-HSQGTFTSDYSKYLDSALAQDFVWLLNT-NH2 | 0,24 | 0,1 |
| 256 | H-HSQGTFTSDYSKYLDKRRAEDFVWLMNT-NH2 | 0,2 | 0,07 |
| 257 | H-HSQGTFTSDYSKYLDK()RRAE()DFVWLMNT-NH2 | 0,1 | 0,09 |
| 258 | H-HSQGTFTSDYSRYLDERRAQDFVWLMNT-NH2 | 0,07 | 0,06 |
| 259 | H-HSQGTFTSDYSK()YLDE()RRAQDFVWLMNT-NH2 | 0,04 | 0,03 |
| 120 | H-HSQGTFTSDYSKYLDSRRAQDFIEWLMNT-NH2 | 0,2 | 0,2 |
| 260 | H-HSQGTFTSDYSKYLDSKAAQDPVWLMNT-NH2 | 0,02 | 0,07 |
| 48 | H-HSQGTFTSDYSKYLDSKAAHDFVEWLLRA-NH2 | 0,06 | 0,06 |
| 44 | H-H-DSer-QGTFTSDYSKYLDSKAAHFDVEWLLRA-NH2 | 0,09 | 0,11 |
| 45 | H-H-Aib-QGTFTSDYSKYLDSKAAHDFVEWLLRA-NH2 | 0,08 | 0,06 |
| 261 | H-HSQGTFTSDYSKYLDSKAAHDFVE()WLLK()A-NH2 | 0,03 | 0,07 |
| 202 | H-HSQGTFTSDYSKYLD-K(Hexadecanoyl-γ-Glu)- KAAHDFVEWLLRA-NH2 | 0,20 | 0,13 |
| 262 | H-HSQGTFTSDYSKYLD-S-K(Hexadecanoyl-γ-Glu)- AAHDFVEWLLRA-NH2 | 0,11 | 0,12 |
| 204 | H-HSQGTFTSDYSKYLDSKAA-K(Hexadecanoyl-γ-Glu)- DFVEWLLRA-NH2 | 0,10 | 0,04 |
| 203 | H-HSQGTFTSDYSKYLDSKAAHDFVEWL-K(Hexadecanoyl-γ- Glu)-RA-NH2 | 0,57 | 0.22 |
| 205 | H-HSQGTFTSDYSKYLDSKAAHDFVEWLL-K(Hexadecanoyl- γ-Glu)-A-NH2 | 0,09 | 0,10 |
| 208 | H-H-Aib-QGTFTSDYSKYLDS-K(Hexadecanoyl-γ-Glu)- AAHDFVEWLLSA-NH2 | 0,11 | 0,16 |
| 263 | H-H-Aib-QGTFTSDYSKYLDE-K(Hexadecanoyl-γ-Glu)- RAKDFIEWLLSA-NH2 | 0,10 | 0,16 |
| 207 | H-H-Aib-QGTFTSDYSKYLDS-K(Hexadecanoyl-γ-Glu)- AARDFVAWLLRA-NH2 | 0,12 | 0,17 |
| 213 | H-H-Aib-QGTFTSDYSKYLDSKAA-K(Hexadecanoyl-γ-Glu)- DFVAWLLRA-NH2 | 0,15 | 0,63 |
| 206 | H-H-Aib-QGTFTSDYSKYLDS-K(Hexadecanoyl-γ-Glu)- AAHDFVEWLLRA-NH2 | 0,09 | 0,16 |
| 209 | H-H-Aib-QGTFTSDYSKYLDSKAAHDFVEWLL- K(Hexadecanoyl-γ-Glu)-A-NH2 | 0,27 | 0,27 |
| 210 | H-H-Aib-QGTFTSDYSKYLDS-K(Hexadecanoyl-γ-Glu)- AAHDFVE()WLLK()A-NH2 | 0,08 | 0,26 |
| 214 | H-H-Aib-QGTFTSDYSKYLDS-K(Dodecanoyl-γ-Glu)- AAHDFVEWLLSA-NH2 | 0,14 | 0,78 |
| 215 | H-H-Aib-QGTFTSDYSKYLDS-K(Hexadecanoyl-[3- Aminopropanoyl])-AAHDFVEWLLSA-NH2 | 0,23 | 1,87 |
| 216 | H-H-Aib-QGTFTSDYSKYLDS-K(Hexadecanoyl-[8- Aminooctanoyl])-AAHDFVEWLLSA-NH2 | 0,24 | 0,46 |
| 217 | H-H-Aib-QGTFTSDYSKYLDS-K(Hexadecanoyl-ε-Lys)- AAHDFVEWLLSA-NH2 | 0,09 | 0,39 |

| | | | |
|---|---|---|---|
| Brackets () indicate intramolecular lactam rings. | | | |

### Example 3. Assessment of inotropic effect in vivo in aneasthetized rats

The effect of the inotropic compound 1 and a glucagon-GLP-1 dual-agonist (Compound 12 on cardiac function, and heart rate was examined in anesthetized male Sprague-Dawley rats weighing approximately 300-400 g (Taconic)..

The rats were exposed to 5% isoflurane in 1:2 N₂O:O₂ until anesthesia were established. Body temperature was kept constant (37.5 ± 0.5°C) and the animals were artificially ventilated through an endotracheal cannula and anesthesia was maintained.
A catheter was inserted into the left femoral vein for drug administration and a pressure-volume catheter was inserted into the left ventricle via the right carotid artery. After instrumentation, isoflurane was delivered in pure O₂ during the experiment. After 20 min of stabilization, baseline data was recorded for 15 min while infusing vehicle (at 7µL/min). Subsequently, compounds were infused After the infusion of the 2.5 nmol/kg/min dose (or a lower dose if heart rate or stroke work was increased more than 40%), vehicle was infused for 15 minutes after which animals were euthanized.

The impact of compound 1 and various dual glucagon-GLP-1 agonists (compounds 7, 9, 12, 35, 37, 206) on cardiac hemodynamic parameters was examined in the anaesthetized rats. Cardiac stroke work is descriptive of the work that the ventricle needs to perform in order to eject a volume of blood into the aorta, and thereby a good representative of the inotropic state of the heart. The measured strokework as a function of infusion dose for each compound is shown in the figure 4a-d. The horizontal line marks 40% increase in stroke work, which was defined as the maximal increase that should be obtained during the experiment. Compound 1 increase the strokework to approximately 40 % in respectively 0,1 and 0,2 nmol/kg/min infusion rates (figure 4b and 4c) after which infusion of this compound was stopped. Except from compound 12 and compound 7, all the dual glucagon-GLP-1 agonists increased the strokework to 40 % at a given infusion rate. The acylated, and thereby more stable, compound 206 showed a prolonged increase in strokework that outlasted the compound infusion and remained high throughout the final vehicle infusion.

In the same experiments, heart rate was calculated from the hemomynamic parameters and results are given in figure 5 showing the changes in heart rate for each infusion rate. Each bar represents different compound. At 0.1 nmol/kg/min and higher doses, compound 1 showed a significant increase in heart rate compared to control (figure 5b and c). None of the other compounds showed significant dose dependent changes in heart rate, although there was a tendency for both compounds 35 and 37 to increase heart rate at 0.2 and 0.5 nmol/kg/min (figure 5c and d).

In relation to the above results, while glucacon is known to increase cardiac contractility, the concomitant increase in heart rate results in increase in myocardial oxygen demand, which can precipitate angina in patients with coronary artery disease, and thereby pose a significant risk to the heart failure patient. The present experiments show that dual glucagon-GLP-1 agonists can improve cardiac inotropic state to the same extent as glucagon. but the increase in inotropy seems not to be coupled to increase in heart rate as observed with infusions of glucagon. Taken together, the results presented above indicate that dual glucagon-GLP-1 agonists act by improving the cardiac contractility without causing the concomitant increase in heart rate observed with glucagon.

## Claims

1. A glucagon-GLP-1 dual agonist for use in a method of treatment of myocardial infarction, cardiogenic shock or heart failure, wherein said glucagon-GLP-1 dual agonist is administered for use as a positive inotropic agent, and wherein said glucagon-GLP-1 dual agonist is a glucagon or oxyntomodulin analogue.

2. A glucagon-GLP-1 dual agonist for use according to claim 1, wherein said heart failure is congestive heart failure, or is caused by systolic dysfunction, diastolic dysfunction, ischemic heart disease or diabetic cardiomyopathy

3. A glucagon-GLP-1 dual agonist for use according to claim 1 wherein the glucagon-GLP-1 dual agonist is administered in combination with an agent for treatment of heart failure, diabetes, obesity, myocardial infarction, hypolipidemia or hypertension.

4. A glucanon-GLP-1 dual agonist for use according to any one of the preceding claims wherein the glucagon-GLP-1 dual agonist is a compound having the formula: ${R}^{- 1} - X - {Z}^{1} - {Z}^{2} - {R}^{2}$ wherein:
R¹ is hydrogen, C₁₋₄ alkyl (e.g. methyl), acetyl, formyl, benzoyl or trifluoroacetyl;
X has the Formua I: 
X1-X2-X3-Gly-Thr-Phe-Thr-Ser-Asp-X10-Ser-X12-Tyr-Leu-X15-X16-X17-X18-Ala-X20-X21-
Phe-X23-X24-Trp-Leu-X27-X28-X29
 wherein
X1 is His, D-His, (Des-amino)His, hydroxyl-His, acetyl-His, homo-His, alpha,alpha-dimethyl imidazole acetic acid (DMIA), N-methyl His, alpha-methyl His or imidazole acetic acid;
X2 is Ser, Aib or D-Ser;
X3 is Gln, Glu, Orn or Nle;
X10 is Tyr or Trp;
X12 is Lys, Arg, His, Ala, Leu, Dpu, Dpr, Orn, Citrulline or Ornithine;
X15 is Asp, Glu, cysteic acid, homoglutamic acid or homocysteic acid;
X16 is Ser, Thr, Lys, Arg, His, Glu, Asp, Ala, Gly, Gln, homoglutamic acid or homocysteic acid;
X17 is Arg, Lys, His, Glu, Gln, Ala, Leu, Dpu, Dpr, Orn, Cys, homocysteine or acetyl phenylalanine;
X18 is Arg, Lys, His, Tyr, Ala, Ser, Leu, Cys, Orn, homocysteine or acetyl phenylalanine;
X20 is Gln, Lys, Arg, His, Glu, Asp, Ala, Cys, Orn or Citrulline;
X21 is Asp, Glu, Gln, Lys, Cys, Orn, homocysteine or acetyl phenyalanine;
X23 is Val, Ile or Leu;
X24 is Gln, Lys, Arg, Glu, Asp, Ser, Ala, Leu, Cys, Orn, homocysteine or acetyl phenyalanine;
X27 is Met, Lys, Arg, Glu, Leu, Nle, Cys or absent;
X28 is Asn, Lys, Arg, Glu, Asp, Ser, Ala, Leu, Cys, Citrulline, Orn, or absent;
X29 is Thr, Lys, Arg, Glu, Ser, Ala, Gly, Cys, Orn, homocysteine, acetyl phenyalanine or absent;
R² is NH₂ or OH;
Z¹ is absent or has the sequence:
Gly-Pro-Ser-Ser-Gly-Ala-Pro-Pro-Pro-Ser;
Gly Pro Ser Ser Gly Ala Pro Pro Pro Ser Cys;
Lys-Arg-Asn-Arg-Asn-Asn-Ile-Ala; or
Lys Arg Asn Arg;
Z² is absent or a peptide sequence of 1-20 amino acid units selected from the group consisting of Ala, Leu, Ser, Thr, Tyr, Cys, Glu, Lys, Arg, Dbu, Dpr and Orn;
wherein, if Z¹ is present, X27, X28 and X29 are also present; and
if Z¹ is absent, the compound has a substitution or deletion relative to human glucagon at one or more of positions X1, X2, X3, X10, X12, X15, X16, X17, X18, X20, X21, X23, X24, X27, X28 and X29:
or a pharmaceutically acceptable salt or derivative thereof;
wherein said compound has higher GLP-1 receptor selectivity than human glucagon.

5. A glucagon-GLP-1 dual agonist for use according to any one of claims 1 to 3wherein the glucagon-GLP-1 dual agonist has the formula R¹-X-Z²-R²
wherein
R¹ is H, C₁₋₄ alkyl, acetyl, formyl, benzoyl or trifluoroacetyl;
R² is OH or NH₂;
X is a peptide which has the Formula III: 
His-Ser-Gln-Gly-Thr-Phe-Thr-Ser-Asp-Tyr-Ser-Leu-Tyr-Leu-Asp-Ser-Arg-Arg-Ala-Lys-Asp-
Phe-Ile-Glu-Trp-Leu-Glu-Ser-Ala
 or differs from Formula III at up to 4 of the following positions whereby, if different from Formula III:
the residue at position 2 is selected from: Aib, D-Ser;
the residue at position 16 is selected from: Arg, His, Lys, Glu, Gly, Asp;
the residue at position 17 is selected from: Lys, Leu;
the residue at position 18 is selected from: Lys, His, Ala, Ser, Tyr;
the residue at position 20 is selected from: Gln, His, Arg, Glu, Asp;
the residue at position 21 is: Glu;
the residue at position 23 is selected from: Val, Leu;
the residue at position 24 is selected from: Gln, Leu, Ala, Lys, Arg, Asp;
the residue at position 27 is selected from: Met, Cys, Lys, Arg, Leu or is absent;
the residue at position 28 is selected from: Asn, Arg, Lys, Glu, Ala, Leu, Asp or is absent; and
the residue at position 29 is selected from: Thr, Glu, Lys or is absent;
and Z² is absent or a peptide sequence of 1-20 amino acid units selected from the group consisting of Ala, Leu, Ser, Thr, Tyr, Cys, Glu, Lys, Arg, Dbu, Dpr and Orn;
or a pharmaceutically acceptable salt thereof.

6. A glucagon-GLP-1 dual agonist for use according to any one of claims 1 to 3 wherein the glucagon-GLP-1 dual agonist has the formula R¹-X-Z²-R²
wherein
R¹ is H, C₁₋₄ alkyl, acetyl, formyl, benzoyl or trifluoroacetyl;
R² is OH or NH₂;
X is a peptide which has the Formula V:
His-Ser-Gln-Gly-Thr-Phe-Thr-Ser-Asp-Tyr-Ser-Lys-Tyr-Leu-Asp-Ser-Lys-Ala-Ala-His-Asp-Phe-Val-Glu-Trp-Leu-Leu-Arg-Ala
or differs from Formula V at up to 4 of the following positions whereby, if different from Formula V:
the residue at position 2 is selected from: Aib, D-Ser;
the residue at position 12 is selected from: Leu, Arg Dpu, Dpr, Orn;
the residue at position 16 is selected from: Arg, His, Lys, Glu, Asp;
the residue at position 17 is selected from: Arg, Leu, Dpu, Dpr, Orn;
the residue at position 18 is selected from: Arg, Lys, His, Ser, Tyr;
the residue at position 20 is selected from: Gln, Lys, Arg, Glu, Asp;
the residue at position 21 is Glu;
the residue at position 24 is selected from: Gln, Leu, Ala, Lys, Arg, Asp;
the residue at position 27 is selected from: Met, Cys, Lys, Arg, Glu or is absent;
the residue at position 28 is selected from: Asn, Ser, Lys, Glu, Ala, Leu, Asp or is absent; and the residue at position 29 is selected from: Thr, Glu, Lys or is absent;
and Z² is absent or a peptide sequence of 1-20 amino acid units selected from the group consisting of Ala, Leu, Ser, Thr, Tyr, Cys, Glu, Lys, Arg, Dbu, Dpr and Orn;
or a pharmaceutically acceptable salt thereof.

7. A glucagon-GLP-1 dual agonist for use according to any one of claims 1 to 3wherein the glucagon-GLP-1 dual agonist has the formula R¹-X-Z²-R²
wherein
R¹ is H, C₁₋₄ alkyl, acetyl, formyl, benzoyl or trifluoroacetyl;
R² is OH or NH₂;
X is a peptide which has the Formula VI: 
His-Ser-Gln-Gly-Thr-Phe-Thr-Ser-Asp-Tyr-Ser-Lys-Tyr-Leu-Asp-Ser-Lys-Ala-Ala-His-Asp-
Phe-Val-Glu-Trp-Leu-Leu-Arg-Ala
 or differs from Formula VI at up to 5 of the following positions whereby, if different from Formula VI:
the residue at position 2 is selected from: Aib, D-Ser;
the residue at position 16 is selected from: Arg, His, Lys, Glu;
the residue at position 17 is: Arg, Leu, Dpu, Dpr, Orn;
the residue at position 20 is selected from: Gln, Lys, Arg, Glu, Asp;
the residue at position 21 is Glu;
the residue at position 24 is selected from: Gln, Leu, Ala, Lys, Arg, Asp;
the residue at position 27 is selected from: Met, Cys, Lys, Arg, Glu or is absent;
the residue at position 28 is selected from: Asn, Ser, Lys, Glu, Ala, Leu, Asp or is absent; and
the residue at position 29 is selected from: Thr, Glu, Lys or is absent;
and Z² is absent or a peptide sequence of 1-20 amino acid units selected from the group consisting of Ala, Leu, Ser, Thr, Tyr, Cys, Glu, Lys, Arg, Dbu, Dpr and Orn;
or a pharmaceutically acceptable salt thereof.

8. A glucagon-GLP-1 dual agonist for use according to any one of claims 1 to 3 wherein the glucagon-GLP-1 dual agonist has the formula R¹-X-Z¹-Z²-R²
wherein:
R¹ is hydrogen, C₁₋₄ alkyl (e.g. methyl), acetyl, formyl, benzoyl or trifluoroacetyl:
wherein X has the Formula VII: 
X1-X2-X3-Gly-Thr-Phe-Thr-Ser-Asp-X10-Ser-X12-Tyr-Leu-X15-X16-X17-X18-Ala-X20-X21-
Phe-X23-X24-Trp-Leu-X27-X28-X29
 wherein
X1 is His, D-His, (Des-amino)His, hydroxyl-His, acetyl-His, homo-His, alpha,alpha-dimethyl imidazole acetic acid (DMIA), N-methyl His, alpha-methyl His, or imidazole acetic acid;
X2 is Ser, D-Ser, Ala, D-Ala, Val, Gly, N-methyl Ser, aminoisobutyric acid (Aib) or N-methyl Ala;
X3 is Gln, Glu, Orn or Nle;
X10 is Tyr or Trp;
X12 is Lys, Citrulline, Orn or Arg;
X15 is Asp, Glu, cysteic acid, homoglutamic acid or homocysteic acid;
X16 is Ser, Glu, Gln, homoglutamic acid or homocysteic acid;
X17 is Arg, Gln, Lys, Cys, Orn, homocysteine or acetyl phenylalanine;
X18 is Arg, Ala, Lys, Cys, Orn, homocysteine or acetyl phenylalanine;
X20 is Gln, Lys, Arg, Orn or Citrulline;
X21 is Gln, Glu, Asp, Lys, Cys, Orn, homocysteine or acetyl phenyalanine;
X23 is Val or Ile;
X24 is Ala, Gln, Glu, Lys, Cys, Orn, homocysteine or acetyl phenyalanine;
X27 is Met, Leu or Nle;
X28 is Asn, Arg, Citrulline, Orn, Lys or Asp;
X29 is Thr, Gly, Lys, Cys, Orn, homocysteine or acetyl phenyalanine;
R² is NH₂ or OH;
Z¹ is absent or has the sequence:
Gly-Pro-Ser-Ser-Gly-Ala-Pro-Pro-Pro-Ser;
Gly Pro Ser Ser Gly Ala Pro Pro Pro Ser Cys;
Lys-Arg-Asn-Arg-Asn-Asn-Ile-Ala; or
Lys Arg Asn Arg;
Z² is absent or a peptide sequence of 1-20 amino acid units selected from the group consisting of Ala, Leu, Ser, Thr, Tyr, Asn, Gin, Asp, Glu, Lys, Arg, His, Met, Har, Dbu, Dpr and Om;
wherein, if Z¹ is absent, the compound has a substitution or deletion relative to human glucagon at one or more of positions X1, X2, X3, X10, X12, X15, X16, X17, X18, X20, X21, X23, X24, X27, X28 and X29;
or a pharmaceutically acceptable salt or derivative thereof;
wherein said compound has higher GLP-1 receptor selectivity than human glucagon and/or wherein the compound exhibits at least 20% of the activity of native GLP-1 at the GLP-1 receptor.

9. A glucagon-GLP-1 dual agonist for use according to c)a)m R wherein X differs from Formula VII by 1 to 3 amino acid modifications at positions selected from 1, 2, 3, 5, 7, 10, 11, 13, 14, 17, 18, 19, 21, 24, 27, 28 and 29.

10. A glucagon-GLP-1 dual agonist for use according to any one of claims 4 to 9 wherein Z² is absent.

11. A glucagon-GLP-1 dual agonist for use according to any one of claims 4 to 10 wherein Z¹ is absent.

12. A glucagon-GLP-1 dual agonist for use according to any one of claims 4 to 11 wherein one or more of the amino acid side chains of the glucagon-GLP-1 dual agonist is conjugated to a lipophilic substituent.

13. A glucagon-GLP-1 dual agonist for use according to claim 12 wherein each lipophilic substituent comprises a lipophilic moiety conjugated to the amino acid side chain by a spacer.

14. A glucagon-GLP-1 dual agonist for use according to claim 13 wherein the combination of lipophilic moiety and spacer is selected from dodecanoyl-γ-Glu, hexadecanoyl-γ-Glu, hexadecanoyl-Glu, hexadecanoyl-[3-aminopropanoyl], hexadecanoyl-[8-aminooctanoyl], hexadecanoyl-ε-Lys, 2-butyloctanoyl-γ-Glu, octadecanoyl-γ-Glu and hexadecanoyl-[4-aminobutanoyl].

15. Use of a glucagon-GLP-1 dual agonist in the preparation of a medicament for the treatment of myocardial infarction, cardiogenic shock or heart failure, wherein the glucagon-GLP-1 dual agonist is to be administered for use as an inotropic agent and wherein said glucagon-GLP-1 dual agonist is a glucagon analogue or oxyntomodulin analogue.

16. Use according to claim 15 wherein the glucagon-GLP-1 dual agonist is as defined in any one of claims 4 to 14.

## Patentansprüche

1. Glucagon-GLP-1-Doppelagonist zur Verwendung in einem Verfahren zur Behandlung von Myokardinfarkt, kardiogenem Schock oder Herzinsuffizienz, worin der Glucagon-GLP-1-Doppelagonist zur Verwendung als positives inotropes Mittel verabreicht wird und worin der Glucagon-GLP-1-Doppelagonist ein Glucagon- oder Oxynto-modulin-Analogon ist.

2. Glucagon-GLP-1-Doppelagonist zur Verwendung nach Anspruch 1, worin die Herzinsuffizienz kongestive Herzinsuffizienz ist oder durch systolische Dysfunktion, diastolische Dysfunktion, ischämische Herzerkrankung oder diabetische Kardiomyopathie verursacht ist.

3. Glucagon-GLP-1-Doppelagonist zur Verwendung nach Anspruch 1, worin der Glucagon-GLP-1-Doppelagonist in Kombination mit einem Mittel zur Behandlung von Herzinsuffizienz, Diabetes, Adipositas, Myokardinfarkt, Hypolipidämie oder Hypertonie verabreicht wird.

4. Glucagon-GLP-1-Doppelagonist zur Verwendung nach einem der vorangegangenen Ansprüche, worin der Glucagon-GLP-1-Doppelagonist eine Verbindung der folgenden Formel ist: ${R}^{- 1} - X - {Z}^{- 1} - {Z}^{2} - {R}^{2} ,$ worin:
R¹ Wasserstoff, C₁₋₄-Alkyl (z.B. Methyl), Acetyl, Formyl, Benzoyl oder Trifluoracetyl ist;
X die Formel I aufweist: 
X1-X2-X3-Gly-Thr-Phe-Thr-Ser-Asp-X10-Ser-X12-Tyr-Leu-X15-X16-X17-X18-
Ala-X20-X21-Phe-X23-X24-Trp-Leu-X27-X28-X29,
 worin:
X1 His, D-His, (Desamino-)His, Hydroxyl-His, Acetyl-His, Homo-His, α,α-Dimethylimidazolessigsäure (DMIA), N-Methyl-His, α-Methyl-His oder Imidazolessigsäure ist;
X2 Ser, Aib oder D-Ser ist;
X3 Gin, Glu, Orn oder Nle ist;
X10 Tyr oder Trp ist;
X12 Lys, Arg, His, Ala, Leu, Dpu, Dpr, Orn, Citrullin oder Ornithin ist;
X15 Asp, Glu, Cysteinsäure, Homoglutaminsäure oder Homocysteinsäure ist;
X16 Ser, Thr, Lys, Arg, His, Glu, Asp, Ala, Gly, Gin, Homoglutaminsäure oder Homocysteinsäure ist;
X17 Arg, Lys, His, Glu, Gin, Ala, Leu, Dpu, Dpr, Orn, Cys, Homocystein oder Acetylphenylalanin ist;
X18 Arg, Lys, His, Tyr, Ala, Ser, Leu, Cys, Orn, Homocystein oder Acetylphenylalanin ist;
X20 Gin, Lys, Arg, His, Glu, Asp, Ala, Cys, Orn oder Citrullin ist;
X21 Asp, Glu, Gin, Lys, Cys, Orn, Homocystein oder Acetylphenylalanin ist;
X23 Val, Ile oder Leu ist;
X24 Gin, Lys, Arg, Glu, Asp, Ser, Ala, Leu, Cys, Orn, Homocystein oder Acetylphenylalanin ist;
X27 Met, Lys, Arg, Glu, Leu, Nie oder Cys ist oder fehlt;
X28 Asn, Lys, Arg, Glu, Asp, Ser, Ala, Leu, Cys, Citrullin oder Orn ist oder fehlt;
X29 Thr, Lys, Arg, Glu, Ser, Ala, Gly, Cys, Orn, Homocystein oder Acetylphenylalanin ist oder fehlt;
R² NH₂ oder OH ist;
Z¹ fehlt oder die folgende Sequenz aufweist:
Gly-Pro-Ser-Ser-Gly-Ala-Pro-Pro-Pro-Ser;
Gly Pro Ser Ser Gly Ala Pro Pro Pro Ser Cys;
Lys-Arg-Asn-Arg-Asn-Asn-Ile-Ala; oder
Lys Arg Asn Arg;
Z² fehlt oder eine Peptidsequenz von 1 bis 20 aus der aus Ala, Leu, Ser, Thr, Tyr, Cys, Glu, Lys, Arg, Dbu, Dpr und Orn bestehenden Gruppe ausgewählten Aminosäureeinheiten ist; worin, wenn Z¹ enthalten ist, X27, X28 und X29 ebenso enthalten sind; und,
wenn Z¹ fehlt, die Verbindung eine Substitution oder Deletion gegenüber menschlichem Glucagon an einer oder mehreren der Positionen X1, X2, X3, X10, X12, X15, X16, X17, X18, X20, X21, X23, X24, X27, X28 und X29 aufweist;
oder ein pharmazeutisch annehmbare Salz oder Derivat davon;
worin die Verbindung eine höhere Selektivität für den GLP-1-Rezeptor aufweist als menschliches Glucagon.

5. Glucagon-GLP-1-Doppelagonist zur Verwendung nach einem der Ansprüche 1 bis 3, worin der Glucagon-GLP-1-Doppelagonist die Formel R¹-X-Z²-R² aufweist, worin:
R¹ H, C₁₋₄-Alkyl, Acetyl, Formyl, Benzoyl oder Trifluoracetyl ist;
R² OH oder NH₂ ist;
X ein Peptid ist, das die Formel III aufweist: 
His-Ser-Gln-Gly-Thr-Phe-Thr-Ser-Asp-Tyr-Ser-Leu-Tyr-Leu-Asp-Ser-Arg-Arg-
Ala-Lys-Asp-Phe-Ile-Glu-Trp-Leu-Glu-Ser-Ala,
 oder sich von Formel III an bis zu 4 der folgenden Positionen unterscheidet, wobei, wenn es sich von Formel III unterscheidet,
der Rest an Position 2 aus Aib, D-Ser ausgewählt ist;
der Rest an Position 16 aus Arg, His, Lys, Glu, Gly, Asp ausgewählt ist;
der Rest an Position 17 aus Lys, Leu ausgewählt ist;
der Rest an Position 18 aus Lys, His, Ala, Ser, Tyr ausgewählt ist;
der Rest an Position 20 aus Gin, His, Arg, Glu, Asp ausgewählt ist;
der Rest an Position 21 Glu ist;
der Rest an Position 23 aus Val, Leu ausgewählt ist;
der Rest an Position 24 aus Gin, Leu, Ala, Lys, Arg, Asp ausgewählt ist;
der Rest an Position 27 aus Met, Cys, Lys, Arg, Leu ausgewählt ist oder fehlt;
der Rest an Position 28 aus Asn, Arg, Lys, Glu, Ala, Leu, Asp ausgewählt ist oder fehlt; und
der Rest an Position 29 aus Thr, Glu, Lys ausgewählt ist oder fehlt;
und Z² fehlt oder eine Peptidsequenz von 1 bis 20 aus der aus Ala, Leu, Ser, Thr, Tyr, Cys, Glu, Lys, Arg, Dbu, Dpr und Orn bestehenden Gruppe ausgewählten Aminosäureeinheiten ist;
oder ein pharmazeutisch annehmbares Salz davon.

6. Glucagon-GLP-1-Doppelagonist zur Verwendung nach einem der Ansprüche 1 bis 3, worin der Glucagon-GLP-1-Doppelagonist die Formel R¹-X-Z²-R² aufweist, worin:
R¹ H, C₁₋₄-Alkyl, Acetyl, Formyl, Benzoyl oder Trifluoracetyl ist;
R² OH oder NH₂ ist;
X ein Peptid ist, das die Formel V aufweist: 
His-Ser-Gln-Gly-Thr-Phe-Thr-Ser-Asp-Tyr-Ser-Lys-Tyr-Leu-Asp-Ser-Lys-Ala-
Ala-His-Asp-Phe-Val-Glu-Trp-Leu-Leu-Arg-Ala,
 oder sich von Formel V an bis zu 4 der folgenden Positionen unterscheidet, wobei, wenn es sich von Formel V unterscheidet,
der Rest an Position 2 aus Aib, D-Ser ausgewählt ist;
der Rest an Position 12 aus Leu, Arg, Dpu, Dpr, Orn ausgewählt ist;
der Rest an Position 16 aus Arg, His, Lys, Glu, Gly, Asp ausgewählt ist;
der Rest an Position 17 aus Arg, Leu, Dpu, Dpr, Orn ausgewählt ist;
der Rest an Position 18 aus Arg, Lys, His, Ser, Tyr ausgewählt ist;
der Rest an Position 20 aus Gin, His, Arg, Glu, Asp ausgewählt ist;
der Rest an Position 21 Glu ist;
der Rest an Position 24 aus Gin, Leu, Ala, Lys, Arg, Asp ausgewählt ist;
der Rest an Position 27 aus Met, Cys, Lys, Arg, Leu ausgewählt ist oder fehlt;
der Rest an Position 28 aus Asn, Ser, Lys, Glu, Ala, Leu, Asp ausgewählt ist oder fehlt; und
der Rest an Position 29 aus Thr, Glu, Lys ausgewählt ist oder fehlt;
und Z² fehlt oder eine Peptidsequenz von 1 bis 20 aus der aus Ala, Leu, Ser, Thr, Tyr, Cys, Glu, Lys, Arg, Dbu, Dpr und Orn bestehenden Gruppe ausgewählten Aminosäureeinheiten ist;
oder ein pharmazeutisch annehmbares Salz davon.

7. Glucagon-GLP-1-Doppelagonist zur Verwendung nach einem der Ansprüche 1 bis 3, worin der Glucagon-GLP-1-Doppelagonist die Formel R¹-X-Z²-R² aufweist, worin:
R¹ H, C₁₋₄-Alkyl, Acetyl, Formyl, Benzoyl oder Trifluoracetyl ist;
R² OH oder NH₂ ist;
X ein Peptid ist, das die Formel VI aufweist: 
His-Ser-Gln-Gly-Thr-Phe-Thr-Ser-Asp-Tyr-Ser-Lys-Tyr-Leu-Asp-Ser-Lys-Ala-
Ala-His-Asp-Phe-Val-Glu-Trp-Leu-Leu-Arg-Ala,
 oder sich von Formel VI an bis zu 5 der folgenden Positionen unterscheidet, wobei, wenn es sich von Formel VI unterscheidet,
der Rest an Position 2 aus Aib, D-Ser ausgewählt ist;
der Rest an Position 16 aus Arg, His, Lys, Glu, ausgewählt ist;
der Rest an Position 17 aus Arg, Leu, Dpu, Dpr, Orn ausgewählt ist;
der Rest an Position 20 aus Gln, Lys, Arg, Glu, Asp ausgewählt ist;
der Rest an Position 21 Glu ist;
der Rest an Position 24 aus Gln, Leu, Ala, Lys, Arg, Asp ausgewählt ist;
der Rest an Position 27 aus Met, Cys, Lys, Arg, Glu ausgewählt ist oder fehlt;
der Rest an Position 28 aus Asn, Ser, Lys, Glu, Ala, Leu, Asp ausgewählt ist oder fehlt; und
der Rest an Position 29 aus Thr, Glu, Lys ausgewählt ist oder fehlt;
und Z² fehlt oder eine Peptidsequenz von 1 bis 20 aus der aus Ala, Leu, Ser, Thr, Tyr, Cys, Glu, Lys, Arg, Dbu, Dpr und Orn bestehenden Gruppe ausgewählten Aminosäureeinheiten ist;
oder ein pharmazeutisch annehmbares Salz davon.

8. Glucagon-GLP-1-Doppelagonist zur Verwendung nach einem der Ansprüche 1 bis 3, worin der Glucagon-GLP-1-Doppelagonist die Formel R¹-X-Z²-R² aufweist, worin:
R¹ Wasserstoff, C₁₋₄-Alkyl (z.B. Methyl), Acetyl, Formyl, Benzoyl oder Trifluoracetyl ist;
worin X die Formel VII aufweist: 
X1-X2-X3-Gly-Thr-Phe-Thr-Ser-Asp-X10-Ser-X12-Tyr-Leu-X15-X16-X17-X18-
Ala-X20-X21-Phe-X23-X24-Trp-Leu-X27-X28-X29,
 worin:
X1 His, D-His, (Desamino-)His, Hydroxyl-His, Acetyl-His, Homo-His, α,α-Di-methylimidazolessigsäure (DMIA), N-Methyl-His, α-Methyl-His oder Imidazolessigsäure ist;
X2 Ser, D-Ser, Ala, D-Ala, Val, Gly, N-Methyl-Ser, Aminoisobuttersäure (Aib) oder N-Methyl-Ala ist;
X3 Gln, Glu, Orn oder Nle ist;
X10 Tyr oder Trp ist;
X12 Lys, Citrullin, Orn oder Arg ist;
X15 Asp, Glu, Cysteinsäure, Homoglutaminsäure oder Homocysteinsäure ist;
X16 Ser, Glu, Gln, Homoglutaminsäure oder Homocysteinsäure ist;
X17 Arg, Gln, Lys, Cys, Orn, Homocystein oder Acetylphenylalanin ist;
X18 Arg, Ala, Lys, Cys, Orn, Homocystein oder Acetylphenylalanin ist;
X20 Gln, Lys, Arg, Orn oder Citrullin ist;
X21 Gln, Glu, Asp, Lys, Cys, Orn, Homocystein oder Acetylphenylalanin ist;
X23 Val oder Ile ist;
X24 Ala, Gln, Glu, Lys, Cys, Orn, Homocystein oder Acetylphenylalanin ist;
X27 Met, Leu oder Nle ist;
X28 Asn, Arg, Citrullin, Orn, Lys oder Asp ist;
X29 Thr, Gly, Lys, Cys, Orn, Homocystein oder Acetylphenylalanin ist;
R² NH₂ oder OH ist;
Z¹ fehlt oder die folgende Sequenz aufweist:
Gly-Pro-Ser-Ser-Gly-Ala-Pro-Pro-Pro-Ser;
Gly Pro Ser Ser Gly Ala Pro Pro Pro Ser Cys;
Lys-Arg-Asn-Arg-Asn-Asn-Ile-Ala; oder
Lys Arg Asn Arg;
Z² fehlt oder eine Peptidsequenz von 1 bis 20 aus der aus Ala, Leu, Ser, Thr, Tyr, Asn, Gln, Asp, Glu, Lys, Arg, His, Met, Har, Dbu, Dpr und Orn bestehenden Gruppe ausgewählten Aminosäureeinheiten ist;
worin, wenn Z¹ fehlt, die Verbindung eine Substitution oder Deletion gegenüber menschlichem Glucagon an einer oder mehreren der Positionen X1, X2, X3, X10, X12, X15, X16, X17, X18, X20, X21, X23, X24, X27, X28 und X29 aufweist;
oder ein pharmazeutisch annehmbare Salz oder Derivat davon;
worin die Verbindung eine höhere Selektivität für den GLP-1-Rezeptor aufweist als menschliches Glucagon und/oder worin die Verbindung zumindest 20 % der Aktivität von nativem GLP-1 am GLP-1-Rezeptor aufweist.

9. Glucagon-GLP-1-Doppelagonist zur Verwendung nach Anspruch 8, worin X sich von der Formel VII durch 1 bis 3 Aminosäuremodifikationen an aus 1, 2, 3, 5, 7, 10, 11, 13, 14, 17, 18, 19, 21, 24, 27, 28 und 29 ausgewählten Positionen unterscheidet.

10. Glucagon-GLP-1-Doppelagonist zur Verwendung nach einem der Ansprüche 4 bis 9, worin Z² fehlt.

11. Glucagon-GLP-1-Doppelagonist zur Verwendung nach einem der Ansprüche 4 bis 10, worin Z¹ fehlt.

12. Glucagon-GLP-1-Doppelagonist zur Verwendung nach einem der Ansprüche 4 bis 11, worin eine oder mehrere der Aminosäurenseitenketten des Glucagon-GLP-1-Doppelagonisten an einen lipohilen Substituenten konjugiert ist.

13. Glucagon-GLP-1-Doppelagonist zur Verwendung nach Anspruch 12, worin jeder lipophile Substituent eine über einen Spacer an die Aminosäurenseitenkette konjugierte lipophile Gruppierung umfasst.

14. Glucagon-GLP-1-Doppelagonist zur Verwendung nach Anspruch 13, worin die Kombination aus liphophiler Gruppierung und Spacer aus Dodecanoyl-y-Glu, Hexadecanoyl-γ-Glu, Hexadecanoyl-Glu, Hexadecanoyl-[3-aminopropanoyl], Hexadecanoyl-[8-aminooctanoyl], Hexadecanoyl-ε-Lys, 2-Butyloctanoyl-γ-Glu, Octadecanoyl-γ-Glu und Hexadecanoyl-[4-aminobutanoyl] ausgewählt ist.

15. Verwendung eines Glucagon-GLP-1-Doppelagonisten zur Herstellung eines Medikaments zur Behandlung von Myokardinfarkt, kardiogenem Schock oder Herz-insuffizienz, worin der Glucagon-GLP-1-Doppelagonist zur Verwendung als inotropes Mittel zu verabreichen ist und worin der Glucagon-GLP-1-Doppelagonist ein Glucagon-Analogon oder ein Oxyntomodulin-Analogon ist.

16. Verwendung nach Anspruch 15, worin der Glucagon-GLP-1-Doppelagonist wie in einem der Ansprüche 4 bis 14 definiert ist.

## Revendications

1. Double agoniste du glucagon-GLP-1 destiné à être utilisé dans un procédé de traitement de l'infarctus du myocarde, d'un choc cardiogénique ou de l'insuffisance cardiaque, où ledit double agoniste du glucagon-GLP-1 est administré pour être utilisé en tant qu'agent inotrope positif, et où ledit double agoniste du glucagon-GLP-1 est un analogue du glucagon ou de l'oxyntomoduline.

2. Double agoniste du glucagon-GLP-1 destiné à être utilisé selon la revendication 1, où ladite insuffisance cardiaque est l'insuffisance cardiaque congestive, ou est provoquée par une dysfonction systolique, une dysfonction diastolique, une cardiopathie ischémique ou une cardiomyopathie diabétique.

3. Double agoniste du glucagon-GLP-1 destiné à être utilisé selon la revendication 1, où le double agoniste du glucagon-GLP-1 est administré en combinaison avec un agent pour le traitement de l'insuffisance cardiaque, du diabète, de l'obésité, de l'infarctus du myocarde, de l'hypolipidémie ou de l'hypertension.

4. Double agoniste du glucagon-GLP-1 destiné à être utilisé selon l'une quelconque des revendications précédentes, où le double agoniste du glucagon-GLP-1 est un composé possédant la formule :
R¹-X-Z¹-Z²-R²
dans laquelle :
R¹ est l'hydrogène, un groupe alkyle en C₁₋₄ (par exemple, méthyle), acétyle, formyle, benzoyle ou trifluoroacétyle ;
X possède la Formule I : 
X1-X2-X3-Gly-Thr-Phe-Thr-Ser-Asp-X10-Ser-X12-Tyr-Leu-X15-
X16-X17-X18-Ala-X20-X21-Phe-X23-X24-Trp-Leu-X27-X28-X29
 dans laquelle
X1 est His, D-His (désamino)His, hydroxyl-His, acétyl-His, homo-His, l'acide alpha,alpha-diméthyl-imidazole-acétique (DMIA), N-méthyl-His, alpha-méthyl-His ou l'acide imidazole-acétique ;
X2 est Ser, Aib ou D-Ser ;
X3 est Gln, Glu, Orn ou Nle ;
X10 est Tyr ou Trp ;
X12 est Lys, Arg, His, Ala, Leu, Dpu, Dpr, Orn, la citrulline ou l'ornithine ;
X15 est Asp, Glu, l'acide cystéique, l'acide homoglutamique ou l'acide homocystéique ;
X16 est Ser, Thr, Lys, Arg, His, Glu, Asp, Ala, Gly, Gln, l'acide homoglutamique ou l'acide homocystéique ;
X17 est Arg, Lys, His, Glu, Gln, Ala, Leu, Dpu, Dpr, Orn, Cys, l'homocystéine ou l'acétylphénylalanine ;
X18 est Arg, Lys, His, Tyr, Ala, Ser, Leu, Cys, Orn, l'homocystéine ou l'acétylphénylalanine ;
X20 est Gln, Lys, Arg, His, Glu, Asp, Ala, Cys, Orn ou la citrulline ;
X21 est Asp, Glu, Gln, Lys, Cys, Orn, l'homocystéine ou l'acétylphénylalanine ;
X23 est Val, Ile ou Leu ;
X24 est Gln, Lys, Arg, Glu, Asp, Ser, Ala, Leu, Cys, Orn, l'homocystéine ou l'acétylphénylalanine ;
X27 est Met, Lys, Arg, Glu, Leu, Nle, Cys ou absent ;
X28 est Asn, Lys, Arg, Glu, Asp, Ser, Ala, Leu, Cys, la citrulline, Orn, ou absent ;
X29 est Thr, Lys, Arg, Glu, Ser, Ala, Gly, Cys, Orn, l'homocystéine, l'acétylphénylalanine ou absent ;
R² est NH₂ ou OH ;
Z¹ est absent ou possède la séquence :
Gly-Pro-Ser-Ser-Gly-Ala-Pro-Pro-Pro-Ser ;
Gly Pro Ser Ser Gly Ala Pro Pro Pro Ser Cys ;
Lys-Arg-Asn-Arg-Asn-Asn-Ile-Ala ; ou
Lys Arg Asn Arg ;
Z² est absent ou une séquence peptidique de 1-20 unités d'acide aminé sélectionnées dans le groupe consistant en Ala, Leu, Ser, Thr, Tyr, Cys, Glu, Lys, Arg, Dbu, Dpr et Orn ;
où, si Z¹ est présent, X27, X28 et X29 sont également présents ; et
si Z¹ est absent, le composé comporte une substitution ou une délétion par rapport au glucagon humain au niveau d'une ou de plusieurs des positions X1, X2, X3, X10, X12, X15, X16, X17, X18, X20, X21, X23, X24, X27, X28 et X29 ;
ou un sel ou dérivé de celui-ci pharmaceutiquement acceptable ;
où ledit composé possède une sélectivité pour le récepteur du GLP-1 plus élevée que celle du glucagon humain.

5. Double agoniste du glucagon-GLP-1 destiné à être utilisé selon l'une quelconque des revendications 1 à 3, où le double agoniste du glucagon-GLP-1 possède la formule R¹-X-Z²-R²
dans laquelle
R¹ est H, un groupe alkyle en C₁₋₄, acétyle, formyle, benzoyle ou trifluoroacétyle ;
R² est OH ou NH₂ ;
X est un peptide qui possède la Formule III : 
His-Ser-Gln-Gly-Thr-Phe-Thr-Ser-Asp-Tyr-Ser-Leu-Tyr-Leu-
Asp-Ser-Arg-Arg-Ala-Lys-Asp-Phe-Ile-Glu-Trp-Leu-Glu-Ser-Ala
 ou diffère de la Formule III jusqu'à 4 des positions suivantes moyennant quoi, si différent de la Formule III : le résidu à la position 2 est sélectionné parmi : Aib, D-Ser ;
le résidu à la position 16 est sélectionné parmi : Arg, His, Lys, Glu, Gly, Asp ;
le résidu à la position 17 est sélectionné parmi : Lys, Leu ;
le résidu à la position 18 est sélectionné parmi : Lys, His, Ala, Ser, Tyr ;
le résidu à la position 20 est sélectionné parmi : Gln, His, Arg, Glu, Asp ;
le résidu à la position 21 est : Glu ;
le résidu à la position 23 est sélectionné parmi : Val, Leu ;
le résidu à la position 24 est sélectionné parmi : Gln, Leu, Ala, Lys, Arg, Asp ;
le résidu à la position 27 est sélectionné parmi : Met, Cys, Lys, Arg, Leu ou est absent ;
le résidu à la position 28 est sélectionné parmi : Asn, Arg, Lys, Glu, Ala, Leu, Asp ou est absent ; et
le résidu à la position 29 est sélectionné parmi : Thr, Glu, Lys ou est absent ;
et Z² est absent ou une séquence peptidique de 1-20 unités d'acide aminé sélectionnées dans le groupe consistant en Ala, Leu, Ser, Thr, Tyr, Cys, Glu, Lys, Arg, Dbu, Dpr et Orn ;
ou un sel de celui-ci pharmaceutiquement acceptable.

6. Double agoniste du glucagon-GLP-1 destiné à être utilisé selon l'une quelconque des revendications 1 à 3, où le double agoniste du glucagon-GLP-1 possède la formule R¹-X-Z²-R²
dans laquelle
R¹ est H, un groupe alkyle en C₁₋₄, acétyle, formyle, benzoyle ou trifluoroacétyle ;
R² est OH ou NH₂ ;
X est un peptide qui possède la Formule V : 
His-Ser-Gln-Gly-Thr-Phe-Thr-Ser-Asp-Tyr-Ser-Lys-Tyr-Leu-
Asp-Ser-Lys-Ala-Ala-His-Asp-Phe-Val-Glu-Trp-Leu-Leu-Arg-Ala
 ou diffère de la Formule V jusqu'à 4 des positions suivantes moyennant quoi, si différent de la Formule V :
le résidu à la position 2 est sélectionné parmi : Aib, D-Ser ;
le résidu à la position 12 est sélectionné parmi : Leu, Arg, Dpu, Dpr, Orn ;
le résidu à la position 16 est sélectionné parmi : Arg, His, Lys, Glu, Asp ;
le résidu à la position 17 est sélectionné parmi : Arg, Leu, Dpu, Dpr, Orn ;
le résidu à la position 18 est sélectionné parmi : Arg, Lys, His, Ser, Tyr ;
le résidu à la position 20 est sélectionné parmi : Gln, Lys, Arg, Glu, Asp ;
le résidu à la position 21 est Glu ;
le résidu à la position 24 est sélectionné parmi : Gln, Leu, Ala, Lys, Arg, Asp ;
le résidu à la position 27 est sélectionné parmi : Met, Cys, Lys, Arg, Glu ou est absent ;
le résidu à la position 28 est sélectionné parmi : Asn, Ser, Lys, Glu, Ala, Leu, Asp ou est absent ; et
le résidu à la position 29 est sélectionné parmi : Thr, Glu, Lys ou est absent ;
et Z² est absent ou une séquence peptidique de 1-20 unités d'acide aminé sélectionnées dans le groupe consistant en Ala, Leu, Ser, Thr, Tyr, Cys, Glu, Lys, Arg, Dbu, Dpr et Orn ;
ou un sel de celui-ci pharmaceutiquement acceptable.

7. Double agoniste du glucagon-GLP-1 destiné à être utilisé selon l'une quelconque des revendications 1 à 3, où le double agoniste du glucagon-GLP-1 possède la formule R¹-X-Z²-R² dans laquelle
R¹ est H, un groupe alkyle en C₁₋₄, acétyle, formyle, benzoyle ou trifluoroacétyle ;
R² est OH ou NH₂ ;
X est un peptide qui possède la Formule VI : 
His-Ser-Gln-Gly-Thr-Phe-Thr-Ser-Asp-Tyr-Ser-Lys-Tyr-Leu-
Asp-Ser-Lys-Ala-Ala-His-Asp-Phe-Val-Glu-Trp-Leu-Leu-Arg-Ala
 ou diffère de la Formule VI jusqu'à 5 des positions suivantes moyennant quoi, si différent de la Formule VI :
le résidu à la position 2 est sélectionné parmi : Aib, D-Ser ;
le résidu à la position 16 est sélectionné parmi : Arg, His, Lys, Glu ;
le résidu à la position 17 est : Arg, Leu, Dpu, Dpr, Orn ;
le résidu à la position 20 est sélectionné parmi : Gln, Lys, Arg, Glu, Asp ;
le résidu à la position 21 est Glu ;
le résidu à la position 24 est sélectionné parmi : Gln, Leu, Ala, Lys, Arg, Asp ;
le résidu à la position 27 est sélectionné parmi : Met, Cys, Lys, Arg, Glu ou est absent ;
le résidu à la position 28 est sélectionné parmi : Asn, Ser, Lys, Glu, Ala, Leu, Asp ou est absent ; et
le résidu à la position 29 est sélectionné parmi : Thr, Glu, Lys ou est absent ;
et Z² est absent ou une séquence peptidique de 1-20 unités d'acide aminé sélectionnées dans le groupe consistant en Ala, Leu, Ser, Thr, Tyr, Cys, Glu, Lys, Arg, Dbu, Dpr et Orn ;
ou un sel de celui-ci pharmaceutiquement acceptable.

8. Double agoniste du glucagon-GLP-1 destiné à être utilisé selon l'une quelconque des revendications 1 à 3, où le double agoniste du glucagon-GLP-1 possède la formule R¹-X-Z¹-Z²-R² dans laquelle :
R¹ est l'hydrogène, un groupe alkyle en C₁₋₄ (par exemple, méthyle), acétyle, formyle, benzoyle ou trifluoroacétyle ;
dans laquelle X possède la Formule VII : 
X1-X2-X3-Gly-Thr-Phe-Thr-Ser-Asp-X10-Ser-X12-Tyr-Leu-X15-
X16-X17-X18-Ala-X20-X21-Phe-X23-X24-Trp-Leu-X27-X28-X29
 dans laquelle
X1 est His, D-His (désamino)His, hydroxyl-His, acétyl-His, homo-His, l'acide alpha,alpha-diméthyl-imidazole-acétique (DMIA), N-méthyl-His, alpha-méthyl-His ou l'acide imidazole-acétique ;
X2 est Ser, D-Ser, Ala, D-Ala, Val, Gly, N-méthyl-Ser, l'acide aminoisobutyrique (Aib) ou N-méthyl-Ala ;
X3 est Gln, Glu, Orn ou Nle ;
X10 est Tyr ou Trp ;
X12 est Lys, la citrulline, Orn ou Arg ;
X15 est Asp, Glu, l'acide cystéique, l'acide homoglutamique ou l'acide homocystéique ;
X16 est Ser, Glu, Gln, l'acide homoglutamique ou l'acide homocystéique ;
X17 est Arg, Gln, Lys, Cys, Orn, l'homocystéine ou l'acétylphénylalanine ;
X18 est Arg, Ala, Lys, Cys, Orn, l'homocystéine ou l'acétylphénylalanine ;
X20 est Gln, Lys, Arg, Orn ou la citrulline ;
X21 est Gln, Glu, Asp, Lys, Cys, Orn, l'homocystéine ou l'acétylphénylalanine ;
X23 est Val ou Ile ;
X24 est Ala, Gln, Glu, Lys, Cys, Orn, l'homocystéine ou l'acétylphénylalanine ;
X27 est Met, Leu ou Nle ;
X28 est Asn, Arg, la citrulline, Orn, Lys ou Asp ;
X29 est Thr, Gly, Lys, Cys, Orn, l'homocystéine ou l'acétylphénylalanine ;
R² est NH₂ ou OH ;
Z¹ est absent ou possède la séquence :
Gly-Pro-Ser-Ser-Gly-Ala-Pro-Pro-Pro-Ser ;
Gly Pro Ser Ser Gly Ala Pro Pro Pro Ser Cys ;
Lys-Arg-Asn-Arg-Asn-Asn-Ile-Ala ; ou
Lys Arg Asn Arg ;
Z² est absent ou une séquence peptidique de 1-20 unités d'acide aminé sélectionnées dans le groupe consistant en Ala, Leu, Ser, Thr, Tyr, Asn, Gln, Asp, Glu, Lys, Arg, His, Met, Har, Dbu, Dpr et Orn ;
où, si Z¹ est absent, le composé comporte une substitution ou une délétion par rapport au glucagon humain au niveau d'une ou de plusieurs des positions X1, X2, X3, X10, X12, X15, X16, X17, X18, X20, X21, X23, X24, X27, X28 et X29 ;
ou un sel ou dérivé de celui-ci pharmaceutiquement acceptable ;
où ledit composé possède une sélectivité pour le récepteur du GLP-1 plus élevée que celle du glucagon humain et/ou dans lequel le composé exhibe au moins 20 % de l'activité du GLP-1 natif au niveau du récepteur du GLP-1.

9. Double agoniste du glucagon-GLP-1 destiné à être utilisé selon la revendication 8, dans lequel X diffère de la Formule VII par 1 à 3 modifications d'acide aminé aux positions sélectionnées parmi 1, 2, 3, 5, 7, 10, 11, 13, 14, 17, 18, 19, 21, 24, 27, 28 et 29.

10. Double agoniste du glucagon-GLP-1 destiné à être utilisé selon l'une quelconque des revendications 4 à 9, dans lequel Z² est absent.

11. Double agoniste du glucagon-GLP-1 destiné à être utilisé selon l'une quelconque des revendications 4 à 10, dans lequel Z¹ est absent.

12. Double agoniste du glucagon-GLP-1 destiné à être utilisé selon l'une quelconque des revendications 4 à 11, dans lequel une ou plusieurs des chaînes latérales d'acide aminé de l'agoniste du glucagon-GLP-1 sont conjuguées à un substituant lipophile.

13. Double agoniste du glucagon-GLP-1 destiné à être utilisé selon la revendication 12, dans lequel chaque substituant lipophile comprend un fragment lipophile conjugué à la chaîne latérale d'acide aminé par un espaceur.

14. Double agoniste du glucagon-GLP-1 destiné à être utilisé selon la revendication 13, dans lequel la combinaison du fragment lipophile et de l'espaceur est sélectionnée parmi dodécanoyl-γ-Glu, hexadécanoyl-γ-Glu, hexadécanoyl-Glu, hexadécanoyl-[3-aminopropanoyle], hexadecanoyl-[8-aminooctanoyle], hexadécanoyl-ε-Lys, 2-butyloctanoyl-γ-Glu, octadécanoyl-γ-Glu et hexadécanoyl-[4-aminobutanoyle].

15. Utilisation d'un double agoniste du glucagon-GLP-1 dans la préparation d'un médicament destiné au traitement de l'infarctus du myocarde, d'un choc cardiogénique ou de l'insuffisance cardiaque, où le double agoniste du glucagon-GLP-1 doit être administré pour être utilisé en tant qu'agent inotrope et où ledit double agoniste du glucagon-GLP-1 est un analogue du glucagon ou un analogue de l'oxyntomoduline.

16. Utilisation selon la revendication 15, dans laquelle le double agoniste du glucagon-GLP-1 est tel que défini dans l'une quelconque des revendications 4 à 14.
